(19) Europäisches Patentamt / European Patent Office / Office européen des brevets

(11) **EP 3 892 734 B1**

(12) **EUROPEAN PATENT SPECIFICATION**

(45) Date of publication and mention of the grant of the patent:
**28.08.2024 Bulletin 2024/35**

(21) Application number: **21151950.9**

(22) Date of filing: **15.01.2021**

(51) International Patent Classification (IPC):
***C12Q 1/18*** *(2006.01)* ***C12Q 1/6897*** *(2018.01)*
***A61K 31/00*** *(2006.01)*

(52) Cooperative Patent Classification (CPC):
(C-Sets available)
**C12Q 1/18; A61K 31/4152; A61K 45/06;**
**C12Q 1/6897;** C12Q 2600/136; G01N 2500/10

(Cont.)

(54) **SCREENING METHODS AND THERAPEUTIC APPLICATIONS BASED ON BACTERIAL MOTILITY INHIBITION**

SCREENING-VERFAHREN UND THERAPEUTISCHE ANWENDUNGEN AUF DER BASIS VON BAKTERIELLER MOTILITÄTSHEMMUNG

PROCÉDÉS DE CRIBLAGE ET APPLICATIONS THÉRAPEUTIQUES BASÉS SUR L'INHIBITION DE LA MOTILITÉ BACTÉRIENNE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.01.2020 EP 20152555**

(43) Date of publication of application:
**13.10.2021 Bulletin 2021/41**

(73) Proprietors:
- **Ludwig-Maximilians-Universität München**
  **80539 München (DE)**
- **Helmholtz-Zentrum für Infektionsforschung GmbH**
  **38124 Braunschweig (DE)**
- **Medizinische Hochschule Hannover (MHH)**
  **30625 Hannover (DE)**

(72) Inventors:
- **JOSENHANS, Christine**
  **80335 München (DE)**
- **SUERBAUM, Sebastian**
  **30559 Hannover (DE)**
- **BRÖNSTRUP, Mark**
  **38106 Braunschweig (DE)**
- **BILITEWSKI, Ursula**
  **38304 Wolfenbüttel (DE)**
- **COOMBS, Nina**
  **30171 Hannover (DE)**

(74) Representative: **Vossius & Partner**
**Patentanwälte Rechtsanwälte mbB**
**Siebertstrasse 3**
**81675 München (DE)**

(56) References cited:
- **ERHARDT MARC ED - RAFI AHMED ET AL: "Strategies to Block Bacterial Pathogenesis by Interference with Motility and Chemotaxis", CURRENT TOPICS IN MICROBIOLOGY AND IMMUNOLOGY, SPRINGER BERLIN HEIDELBERG, BERLIN, HEIDELBERG, vol. 398, 1 January 2016 (2016-01-01), pages 185 - 205, XP009520220, ISSN: 0070-217X, ISBN: 978-3-540-92165-3, DOI: 10.1007/82_2016_493**
- **LYNN RASMUSSEN ET AL: "A High-Throughput Screening Assay for Inhibitors of Bacterial Motility Identifies a Novel Inhibitor of the Na+-Driven Flagellar Motor and Virulence Gene Expression in Vibrio cholerae", ANTIMICROBIAL AGENTS AND CHEMOTHERAPY, vol. 55, no. 9, 1 September 2011 (2011-09-01), US, pages 4134 - 4143, XP055689775, ISSN: 0066-4804, DOI: 10.1128/AAC.00482-11**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- ISOO ITO ET AL: "Synthesis of Pyrazolone Derivatives. XII. Synthesis of 3-Mercaptomethyl-2-methyl-4-substituted-1-phenyl-3-pyrazolin-5-one", CHEMICAL AND PHARMACEUTICAL BULLETIN, vol. 14, no. 11, 1 January 1966 (1966-01-01), JP, pages 1237 - 1241, XP055689721, ISSN: 0009-2363, DOI: 10.1248/cpb.14.1237
- ANONYMOUS: "CAS REGISTRY NUMBER 499197-44-3", REGISTRY, CHEMICAL ABSTRACTS SERVICES, 17 March 2003 (2003-03-17), XP055701292
- DATABASE CAPLIUS [online] CHEMICAL ABSTRACTS SERVICE, Ohio, Columbus, US; 1 January 1959 (1959-01-01), KOST AU ET AL: "Reactions of hydrazine derivatives. XXIII. 1-Acylpyrazolines and their action on pathogenic microorganisms", XP055818367, retrieved from caplus accession no. 122045 Database accession no. 122045
- COOMBS N ET AL: "Poster P02.35.- Identification and characterization of novel antivirulence approaches using small compound libraries for motility and metabolic inhibition of Helicobacter pylori", HELICOBACTER, vol. 22, no. s1, 1 September 2017 (2017-09-01), XP055818342



(52) Cooperative Patent Classification (CPC): (Cont.)

C-Sets
**A61K 31/4152, A61K 2300/00**

**Description**

The present invention is defined by the claims as appended. Generally described herein, but not covered by the claims, is a method of identifying a compound having bacterial anti-motility activity, the method comprising: (a) culturing flagellated bacteria in the presence of a test compound under growth conditions; (b) determining the expression level of a bacterial flagellar gene or a bacterial chemotaxis gene, wherein said expression level is determined by detecting (i) the mRNA and/or protein level of said bacterial flagellar gene or said bacterial chemotaxis gene; and/or (ii) a signal provided by a reporter gene construct, wherein the reporter gene construct comprises a nucleotide sequence encoding a reporter gene product operatively linked to a promoter of a bacterial flagellar gene or of a bacterial chemotaxis gene; and wherein a reduction in the expression level as compared to the expression level in bacteria of the same species not cultured in the presence of said test compound is indicative of said test compound having bacterial anti-motility activity and/or bacteriostatic activity and/or bactericidal activity; (c) culturing under growth conditions bacteria of the same species as in (a) in the presence of the test compound identified in (b) having bacterial anti-motility activity and/or bacteriostatic activity and/or bactericidal activity; and (d) determining the presence or absence of growth of said bacteria, wherein the absence of growth-inhibition is indicative of said test compound having a bacterial anti-motility activity and having substantially no bacteriostatic and no bactericidal activity; and optionally (e) determining the identity and/or structure of (i) the test compound having a bacterial anti-motility activity; and/or (ii) a target molecule of said test compound, preferably the binding epitope of said test compound on said target molecule.

In this specification, a number of documents including patent applications and manufacturer's manuals are cited.

*Helicobacter pylori* (*H. pylon*), previously known as *Campylobacter pylori* (*C. pylon*), is a Gram-negative, microaerophilic flagellated bacterium of the class *Epsilonproteobacteria* and prevalent human gastric pathogen that colonizes the gastroduodenal mucosa and produces inflammation, causing millions of severe gastrointestinal disease conditions, including an estimated 900,000 new gastric cancer cases each year worldwide (Herrera & Parsonnet, 2009; Suerbaum & Michetti, 2002; Lertsethtakarn et al., 2011; Chevance FF and Hughes KT, Methods Mol Biol. 2017;1593:47-71). *H. pylori* eradication is a viable strategy for preventing gastric cancer (IARC monograph 2014; Lee et al., 2008; Zhang et al., 2015).

Current established treatments of *H. pylori* infections are based on combination therapies, either as triple therapy, utilizing two antibiotics (usually amoxicillin and clarithromycin) and a proton-pump inhibitor (PPI), or as quadruple therapy, wherein bismuth or additional antibiotics is/are additionally applied (Ko, Kim, Chung, & Lee, 2019; Lobo et al., 1994; Luther et al., 2010; C. O'Morain et al., 2003; N. R. O'Morain, Dore, O'Connor, Gisbert, & O'Morain, 2018). The current standard of using multiple antibiotics to treat *H. pylori* infections originates from the fact that single therapies did not successfully remove the bacteria in humans upon first trials. Treatment failure with single antibiotics may be due, as proposed, to the inapparent persistence of the bacteria during and after treatment, which then leads to recrudescence/relapse of the infection at later time points after the treatment is stopped (Xia, Talley, Keane, & O'Morain, 1997). Such bacterial recrudescence indicates that suppressed bacterial colonization can retreat into so called "sanctuary sites", such as the transition zone between stomach corpus and antrum (van Zanten, Kolesnikow, Leung, O'Rourke, & Lee, 2003). Those persistence zones might favor survival of the bacteria and be inaccessible to full antimicrobial action, in particular, if only a single antibacterial compound is used.

The presently applied combination therapies suffer from poor efficacy and severe side effects. One growing concern is the destructive, irreversible effect of common antibiotic-based therapies against *H. pylori* on the beneficial resident human microbiota, in particular of the digestive tract (Blaser, 2016; Dethlefsen, Huse, Sogin, & Relman, 2008; Hsu et al., 2018). This is because most currently applied antibiotics act against conserved essential targets common to different bacterial species. Another major hurdle to the treatment success of currently used antibiotics is the increased development of antibiotic resistance in *H. pylori,* but also other bacterial pathogens simultaneously present in the patient's body (Agudo et al., 2009; Duck et al., 2004; Pilotto et al., 2000). This has led to the inclusion of resistant *H. pylori* strains (in particular clarithromycin-resistant strains) into the WHO top 10 priority list of antibiotic resistant infections in urgent need of novel therapeutics. A further disadvantage of current combination therapies arises from proton-pump inhibitors which are suspected to contribute to cancerogenesis in consequence of their disturbing impact on the gastric microbiome. Vaccines against *H. pylori* are currently not clinically available and intense efforts into this area in human studies have not been effective to date (Aebischer et al., 2008; Maffertheiner et al., 2018; Zeng et al., 2015).

In recent years, various strategies have been suggested and are currently pursued to overcome these multiple disadvantages. One strategy has been to develop new generations of antibiotics with increased target species selectivity. For example, *Gavrish* and colleagues, by applying an antibacterial screening approach, identified HPi1 (3-hydrazinoquinoxaline-2-thiol), a novel compound which, in an *in vitro* assay and preliminary assessment in a mouse model, showed classical antibiotic activity, apparently, selectively against *H. pylori* (Gavrish et al., 2014). Another study by Kennedy and colleagues reported the development of amixicile, a novel compound with classical antibacterial activity which inhibits the function of pyruvate-ferredoxin oxidoreductase (PFOR), an essential metabolic enzyme in *H. pylori* involved in energy generation through oxidative decarboxylation of pyruvate, yet clinical validation in human patients is also still missing

(Kennedy et al., 2016). However, although an increase in selectivity toward *H. pylori* may help to spare the resident microbiota, such agents with classical antibiotic activity, *i.e.,* targeting bacterial growth and/or survival, are likely to promote pathogen evasion mechanisms that may culminate in the development of antibiotic resistance and/or recurring persistence.

Alternative strategies are directed to the inhibition of bacterial virulence, which, in contrast to compounds with classical antibiotic activity, are aimed at disarming pathogens rather than killing them or inhibiting their growth. In general, virulence-targeted drugs (so-called "antivirulence drugs" or "patho-blockers") are thought to apply less selective pressure for the development of antibiotic resistance than traditional approaches. In addition, anti-virulence drugs can be expected to be more organism specific, and therefore are less likely to impact host commensal flora. Various targets for anti-virulence strategies have been suggested including the inhibition of biofilm formation, adherence, and bacterial toxins (Allen, Popat, Diggle, & Brown, 2014; Rasko & Sperandio, 2010; Totsika, 2017). However, except for some recently approved antibodies against bacterial toxins, advances in this direction have been limited (Theurezbacher & Piddock, 2019).

Examples of antivirulence therapeutic strategies recently pursued against *H. pylori* are inhibitors (e.g., of the aryl-amino hydroxamate class) of the bacterial enzyme urease, which has been shown to be required for *H. pylori* colonization, but not its survival (Ferrero, Thiberge, Huerre, & Labigne, 1994; Kavermann et al., 2003; Salama, Shepherd, & Falkow, 2004); and compounds (e.g. of the selenazole class) targeting the metabolic enzyme carbonic anhydrase, which, similar to urease, is not essential for *H. pylori* survival (Angeli et al., 2019).

Another recently endorsed anti-virulence strategy is based on targeting bacterial motility (*i.e.*, the ability of directional movement) and/or chemotaxis (*i.e.*, the ability of movement in response to a chemical stimulus) which, in *H. pylori* and other flagellated bacteria, have been found to be critical factors for host colonization and pathogenicity (Erhardt, 2016). However, despite multiple efforts devoted to research and development, to date no effective anti-virulence drug targeting bacterial motility and no suitable screening method are available.

Accordingly, there is still an urgent need in the art to provide means and methods to successfully treat infections of pathogenic bacteria, and in particular pathogenic bacteria such as *H. pylori.* The present invention addresses this need and provides such a method and compounds useful for such treatments.

The present invention is defined by the claims. Hence, the present invention relates to a compound of formula (I),

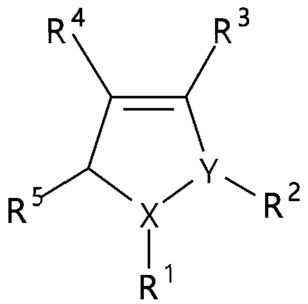

(I)

for use in

(A) treating and/or preventing a bacterial infection, wherein the bacterial infection is caused by flagellated bacteria selected from *Helicobacter spp.* and *Campylobacter spp.*;
(B) treating a condition associated with the bacterial infection as defined in (A), wherein the condition is selected from peptic ulcer, gastritis, duodenitis, gastric mucosa-associated lymphoid tissue (MALT) lymphoma, enteritis, and enterocolitis; or
(C) preventing a condition associated with the bacterial infection as defined in (A), wherein the condition is selected from peptic ulcer, gastritis, duodenitis, gastric (stomach) cancer, gastric MALT lymphoma, enteritis, and enterocolitis; and

wherein in (A) to (C) a therapeutically effective amount of the compound is to be administered to a subject in need thereof, wherein the subject is a mammal, preferably a human, or other animal; and
wherein:

(a) X and Y of formula (I) are independently selected from N, O and S;
(b) $R^1$ is:

(b-1) phenyl, optionally substituted by one or more:

(i) $C_{1-3}$ alkyl or isopropyl; and/or
(ii) X or $CX_3$ (X is selected from Cl, Br, I and F); or

(b-2) biphenyl;

(c) $R^2$ is $CX_3$ or $CH_2CX_3$, where X is selected from H, F, Cl, Br and I;
(d) $R^3$ is $CH_2SX$, where X is H or $C(NH)NH_2$;
(e) $R^4$ is selected from F, Cl, Br and I; and
(f) $R^5$ is O; and

wherein the compound has an anti-motility activity on the bacteria.

In a preferred embodiment of the compound for use according to the invention, the compound is of formula (II):

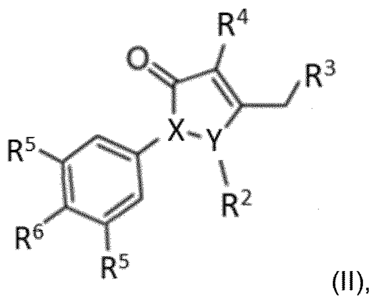

(II),

wherein

(a) X and Y of formula (II) are each independently selected from N, O and S;
(b) $R^2$ is $CH_3$ or $CH_2CH_3$;
(c) $R^3$ is SX, where X is H or

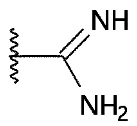

;

and
(d) $R^4$ is selected from F, Cl, Br and I; and
(e) $R^5$ and $R^6$ are each independently selected from X or $CX_3$ (X is selected from H, F, Cl, Br and I), ethyl, isopropyl or phenyl.

In a preferred embodiment of the latter embodiment,

(a) X and Y of formula (II) are each independently selected from N, O and S;
(b) $R^2$ is $CH_3$ or $CH_2CH_3$;
(c) $R^3$ is SX, where X is H or

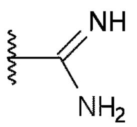

;

and
(d) $R^4$ is selected from F, Cl, Br and I;
(e) $R^5$ is H or $CH_3$; and
(f) $R^6$ is X or $CX_3$ (X is selected from H, F, Cl, Br and I), ethyl, isopropyl or phenyl.

In a further preferred embodiment of the compound for use according to the invention, the compound is of formula (III):

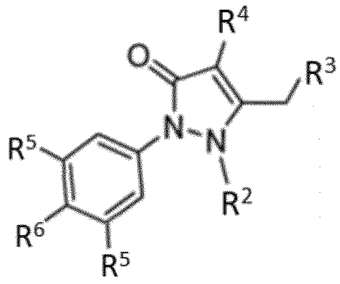

(III),

(a) $R^2$ is $CH_3$ or $CH_2CH_3$;
(b) $R^3$ is SX, where X is H or

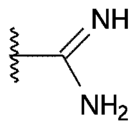

;

and
(c) $R^4$ is selected from F, Cl, Br and I;
(d) $R^5$ is H or $CH_3$; and
(e) $R^6$ is H, X or $CX_3$ (where X is H, F, Cl, Br or I), ethyl, isopropyl or phenyl.

In a preferred embodiment of the latter embodiment,

(a) $R^2$ is $CH_2CH_3$ and $R^3$ is SH; or
(b) $R^2$ is $CH_3$ and $R^6$ is H, X (where X is F, Cl, Br or I), $CH_3$, ethyl, isopropyl or phenyl.

In a preferred embodiment of the latter two embodiments,

(a) $R^2$ is $CH_2CH_3$, $R^3$ is SH, and $R^5$ and $R^6$ are each H; or
(b) $R^2$ is $CH_3$, $R^3$ is SH, and $R^5$ and $R^6$ are each H.

In a further preferred embodiment of the compound for use according to the invention, the compound is selected from:

(a)

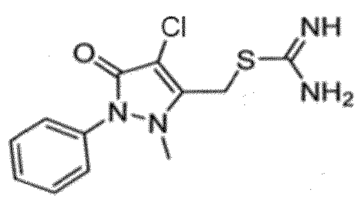

(b)

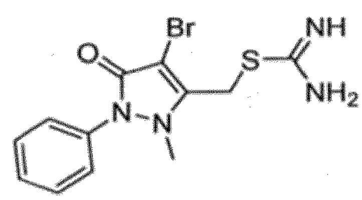

(c)

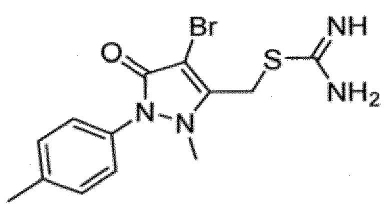

(d)

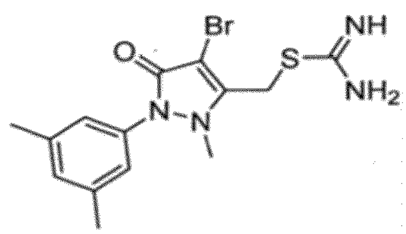

(e)

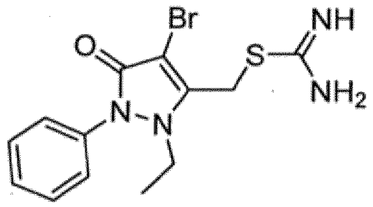

(f)

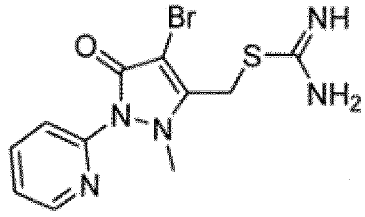

In a further preferred embodiment of the compound for use according to the invention, the compound is selected from:

(a)

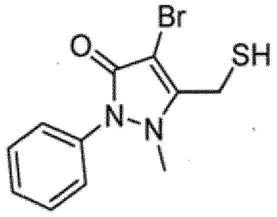

(b)

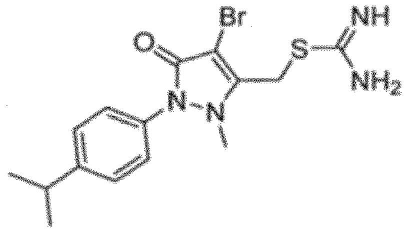

(c)

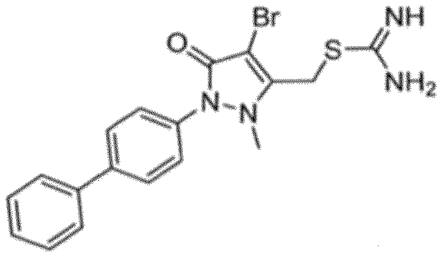

(d)

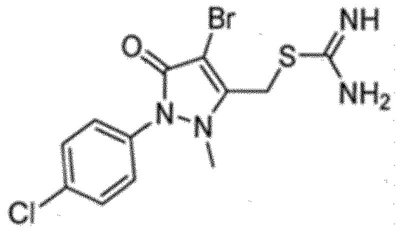

(e)

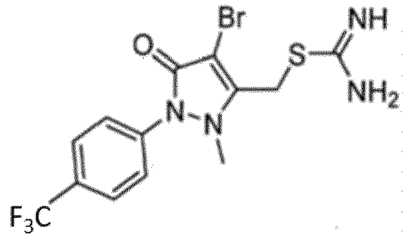

(f)

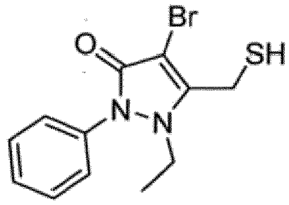

In a further preferred embodiment of the compound for use according to the invention, the *Helicobacter spp.* and/or the *Campylobacter spp.* are

(a) gastro-intestinal pathogenic bacteria;
(b) gastro-duodenal pathogenic bacteria;
(c) urogenital pathogenic bacteria;
(d) not susceptible to antibiotic treatment;
(e) *Helicobacter pylori*; and/or
(f) *Campylobacter jejuni* or *Campylobacter coli.*

In a further preferred embodiment of the compound for use according to the invention, the compound is to be administered in combination, or sequentially, with one or more further agents selected from:

(a) antibiotic, preferably selected from amoxicillin, penicillin, clarithromycin, metronidazole, tetracycline, tinidazole, rifampin, rifabutin, ciprofloxacin, gemifloxacin, levofloxacin, moxifloxacin, norfloxacin and ofloxacin or other fluoroquinolone or quinolone; and/or
(b) bismuth salt, preferably bismuth subcitrate potassium; and/or

(c) proton pump inhibitor (PPI), preferably selected from pantoprazole, omeprazole, lansoprazole, esomeprazole, rabeprazole, dexlansoprazole, dexrabeprazole and vonoprazan; and/or
(d) urease inhibitor; and/or
(e) carbonic anhydrase inhibitor, preferably acetazolamide.

Also described herein, but not claimed is a method of identifying a compound having bacterial antimotility activity, the method comprising: (a) culturing flagellated bacteria in the presence of a test compound under growth conditions; (b) determining the expression level of a bacterial flagellar gene or a bacterial chemotaxis gene, wherein said expression level is determined by detecting (i) the mRNA and/or protein level of said bacterial flagellar gene or said bacterial chemotaxis gene; and/or (ii) a signal provided by a reporter gene construct, wherein the reporter gene construct comprises a nucleotide sequence encoding a reporter gene product operatively linked to a promoter of a bacterial flagellar gene or of a bacterial chemotaxis gene; and wherein a reduction in the expression level as compared to the expression level in bacteria of the same species not cultured in the presence of said test compound is indicative of said test compound having bacterial anti-motility activity and/or bacteriostatic activity and/or bactericidal activity; (c) culturing under growth conditions bacteria of the same species as in (a) in the presence of the test compound identified in (b) having bacterial antimotility activity and/or bacteriostatic activity and/or bactericidal activity; and (d) determining the presence or absence of growth of said bacteria, wherein the absence of growth-inhibition is indicative of said test compound having a bacterial anti-motility activity and having substantially no bacteriostatic and no bactericidal activity; and optionally (e) determining the identity and/or structure of (i) the test compound having a bacterial anti-motility activity; and/or (ii) a target molecule of said test compound, preferably the binding epitope of said test compound on said target molecule.

The term "flagellated bacteria", as used herein, refers to any bacterial species that express at least one flagellum. A flagellum (*plural*: flagella) is the bacterial organelle conferring motility.

The term "motility", or "flagellar motility" as interchangeably used herein, refers to the ability of a flagellated bacterium for directional movement. A functional bacterial motility system requires the functional expression of one or more flagella as the organelle for locomotion and the chemotaxis system (or chemosensory system) that controls flagellar rotation based on environmental conditions (reviewed in Lertsethtakarn et al., 2011; Chevance FF and Hughes KT, Methods Mol Biol. 2017;1593:47-71). The general components of these systems and their complex interplay are/is further described below.

The term "chemotaxis", as used herein, refers to the orientation or movement of a flagellated bacterium in response to the presence of a chemical or chemical gradient, whereby said orientation or movement is influenced in a positive or negative manner by the substance exhibiting chemical properties. Chemoattractants and chemorepellents function to induce either positive or negative chemotaxis respectively.

The term "anti-motility activity", as used herein, refers to any activity or effect caused by a compound that leads to a dysfunctional or entire inhibition of motility.

An anti-motility activity may be due to an activity of the compound which leads to an impaired or defective biosynthesis and/or an impaired function of a bacterial flagellum. An anti-motility activity may also be due to an activity of a compound which leads to an impaired or defective biosynthesis and/or an impaired function of any component of the bacterial chemotaxis system. It is, hence, understood that an anti-motility activity may result from an activity of the compound on any structural or regulatory component of the bacterial flagellum and/or chemotaxis system.

The term "anti-flagellar activity", as used herein, refers to a particular form of "anti-motility activity" where the compound affects the biosynthesis and/or function of a bacterial flagellum.

A "bacterial flagellum" is composed of about 25 to 30 relatively conserved proteins with copy numbers ranging from a few to a few thousands and is made by self-assembly of those proteins. Generally, a flagellum consists of a membrane embedded molecular motor that rotates a long helical filament that works as a propeller driving the bacterium through the liquid environment.

A bacterial flagellum can be divided into two main substructures: the hook-basal body (HBB) and the extracellular filament (reviewed in Lertsethtakarn et al., 2011; Chevance FF and Hughes KT, Methods Mol Biol. 2017;1593:47-71).

The HBB can further be divided into three structures: (a) the base (or basal body), located in the cytoplasm; (b) the rod and associated ring structures, located in the periplasm; and (c) the surfacelocalized hook. The base consists of the MS ring, the C ring (or switch complex), the flagellar type III secretion system (T3SS), and the motor. The MS ring, located at the inner membrane, is a homomultimer of FliF with a functional flagellar T3SS (composed of FlhA, FlhB, FliO, FliP, FliQ, and FliR) within the central membrane patch of the ring. The flagellar T3SS exports many proteins that are part of the flagellum. The C ring (composed of FliG, FliM, FliN or FliY; *Epsilonproteobacteria* contain both FliN and FliY), located at the cytoplasmic face of the MS ring, functions as a flagellar switch (regulating the motor rotation) and also aids in secretion. FliG is largely responsible for the rotary component of the switch, turning the flagellum either clockwise or counterclockwise depending on the input from the chemotaxis system. Motor components include MotA and MotB, which form an ion channel and the stator of the flagellar motor complex, and act together to translate proton flow into a turning force (torque) for flagellar rotation. Most flagellar proteins located outside of the inner membrane are

dependent on the T3SS and the MS ring and switch complex for export.

The biosynthesis of the flagellum occurs from the inside out, beginning with the MS ring and the switch complex, continuing with the remaining HBB complex and terminating with the extracellular filament. Ordered secretion of proteins to promote proper flagellar biosynthesis is an inherent property of the flagellar T3SS. FliE, FlgB and FlgC are the first rod proteins secreted and polymerize on the periplasmic surface of the MS ring to form a hollow tube. Additional nascently secreted flagellar proteins (such as the rod proteins FlgF and FlgG) polymerize on the tip of the sprouting flagellar structure. This pattern of secretion and polymerization on the tip extends throughout the biosynthesis of the hook and filament. FlgI and FlgH form the P ring in the peptidoglycan layer and the L ring in the outer membrane, respectively. These rings form around the rod, likely providing support and passage through these cell envelope components. After the rod is completed, FlgE and minor hook proteins are secreted. FliK determines the correct number of FlgE proteins needed to form a proper hook length in *Campylobacter* and *Helicobacter species.* As shown in *Salmonella* species, FliK likely functions as a molecular ruler to measure the hook length as it is synthesized. Simultaneous contact of the N and C termini of FliK with FlgD, which is present at the tip of the growing hook, and with FlhB of the T3SS at the flagellar base, signifies that a hook of an ideal length has formed. An autoproteolytic cleavage event then occurs in FlhB that alters the substrate specificity of secretion by the T3SS so that proteins for filament biosynthesis, such as the flagellins, are then exported. Flagellar biosynthesis terminates with the biosynthesis of the filament. In *H. pylori* and C. *jejuni,* the filament is composed primarily of the major flagellin (flagellin A, FlaA), and the less abundantly present minor flagellin (flagellin B, FlaB). Whereas FlaA has been shown to be essential for flagellation and motility, the requirement of FlaB varies between species. In some bacteria (e.g. *H. pylon*), the filament is additionally covered by a membrane-like sheath.

Expression of the flagellar genes is controlled by a complex transcriptional regulatory cascade in a hierarchical manner that allows flagellar proteins to be produced in stages, facilitating their ordered secretion and proper interactions to ensure that flagellar biosynthesis occurs correctly. In motile bacteria, flagellar genes can be organized into classes (class 1, 2 and 3; or early, middle and late genes, respectively) on the basis of their temporal order of expression (Lertsethtakam et al., 2011; Chevance FF and Hughes KT, Methods Mol Biol. 2017;1593:47-71).

Class 1 genes are transcribed first and usually encode master transcriptional regulators that control the expression of the class 2 genes, which include those encoding the HBB proteins. However, unlike most other motile bacteria, in *H. pylori* and C. *jejuni* canonical flagellar master regulators appear to be absent. Instead, the class 1 genes of these bacteria include those encoding the flagellar T3SS, the FlgS (histidine kinase) and FlgR (response regulator) two-component system (TCS), the FlhF, GTPase, and sigma factor 54 ($\sigma^{54}$) (a transcriptional regulator which mediates binding of RNA polymerase to gene promoters) which are constitutively expressed by a housekeeping promoter.

Class 3 genes include the major flagellin, *flaA* and other filament genes. The expression of these late flagellar genes is controlled by a sigma factor 28 ($\sigma^{28}$)-dependent promoter (in *H. pylori* and *C. jejuni* encoded by *fliA*) and is negatively regulated by the anti-sigma factor FlgM. It is of note that the minor flagellin, *flaB* (as several other flagellar genes discussed above), unlike *flaA*, corresponds to a class 2 gene which is regulated by a $\sigma^{54}$-dependent promoter (Josenhans et al., 2002). As in other bacterial systems (e.g. *Salmonella*), the expression of *H. pylori* and *C. jejuni* $\sigma^{28}$-dependent class 3 genes is repressed by FlgM (through binding of $\sigma^{28}$) until the HBB is completed. Once the HBB is completed, FlgM is secreted or removed by the T3SS, which relieves $\sigma^{28}$ from repression and allows it to interact with RNA polymerase to express class 3 genes. FlaA and other filament proteins are then produced and secreted through the HBB complex for filament biosynthesis.

In addition to the above-described transcriptional regulatory cascade, flagellar biosynthesis is also controlled by posttranscriptional and posttranslational regulatory mechanisms. Posttranscriptional regulatory mechanisms of flagellar biosynthesis in *H. pylori* include the action of the cytoplasmic protein HP0985 which binds to both mRNA and protein substrates to influence transcription or translation of various flagellar components (Lertsethtakam et al., 2011). For example, HP0985 interacts with *flaA* mRNA, thereby promoting the translation of the major flagellin. Posttranslational regulatory mechanisms of flagellar biosynthesis include the glycosylation of the flagellin proteins. Thus, *Campylobacter* and *Helicobacter* flagellins need to be glycosylated for flagellar filament biosynthesis. FlaA of both of these species, and FlaB of *H. pylori,* are modified by O-linked protein glycosylation systems which add a glycan to specific serines or threonines of the flagellins. This modification is thought to provide additional subunit interactions for stacking the flagellins during filament biosynthesis.

Flagellated bacteria can be differentiated based on the number and arrangement of their one or more flagella: "peritrichously"-flagellated bacteria have multiple flagella all over the cell body projecting in all directions (e.g., *Escherichia coli, Bacillus subtilis and Salmonella enterica*), whereas in "polar"-flagellated bacteria, flagellar biosynthesis is spatially and numerically restricted, including "monotrichously"-flagellated bacteria, which have a single flagellum at one cell pole; "amphitrichously"-flagellated bacteria, which have a single flagellum at each of the two cell poles (e.g. *spirochetes* or *Spirillum sp*.); and lophotrichously-flagellated bacteria have multiple flagella located at one pole of the bacterial cell which act in concert to drive the bacteria in a single direction (e.g., *Helicobacter pylon*).

The term "bacterial flagellar gene", as used herein, refers to any gene whose expression is involved in the biosynthesis and/or function of the one or more flagella of the flagellated bacteria. Such genes may encode a protein

which itself forms a structural component of said flagella (denoted hereafter as "structural flagellar gene" and "structural flagellar protein", respectively), and/or which acts as an accessory protein (e.g. a chaperone) for such a structural flagellar protein during translation, secretion and/or assembly (designated hereafter as "non-structural flagellar gene" and "non-structural flagellar protein", respectively). "Non-structural flagellar genes" also include genes encoding a transcriptional or translational regulator of a structural or non-structural flagellar protein, wherein said transcriptional or translational regulator may also be an RNA (e.g. a non-coding RNA) or a protein (e.g. a transcription factor). "Non-structural flagellar genes" also refer to genes encoding enzymes involved in posttranslational modification of structural flagellar proteins, for example, the biosynthetic enzymes of pseudaminic acid (Pse) which sugar is found as O-linked glycosylation on flagellar proteins of *H. pylori* and *Campylobacter jejuni* (as described above).

The bacterial flagellar gene may be a structural flagellar gene.

The bacterial flagellar gene may be a middle (class 2) or late (class 3) bacterial flagellar gene; and wherein said bacterial flagellar gene may be a structural flagellar gene. Various exemplary class 2 and class 3 bacterial flagellar genes (or their corresponding proteins), as known in the art, are described above, and more are known in the art. Any of these, including any known homologs (*i.e.*, orthologs or paralogs) thereof (some of which may be known by different names dependent on the bacterial species), may be employed in the herein described method.

The bacterial flagellar gene may be one exclusively found in the particular targeted bacteria or group of bacteria (e.g., certain pathogenic bacteria) while being absent in non-targeted bacteria (e.g., certain (beneficial) bacteria of the resident microbiota). The term "absent in non-targeted bacteria" as used herein, means that the bacterial flagellar gene whose expression level is assessed in the described method differs with respect to its mRNA sequence and/or encoded protein sequence from any mRNA sequence and/or encoded protein sequence in the non-targeted bacteria (e.g. certain beneficial bacteria of the resident microbiota) by at least 20%, 30%, 40% or 50%. In case the expression level of the bacterial flagellar gene is assessed indirectly based on its promoter activity (according to step (b) (ii)), it is preferred that the promoter of said bacterial flagellar gene differs with respect to its nucleotide sequence from any nucleotide sequence in the non-targeted bacteria (e.g. certain (beneficial) bacteria of the resident microbiota) by at least 20%, 30%, 40% or 50%.

The bacterial flagellar gene may be one abundantly expressed in the flagellated bacteria. As used herein, the term "abundantly expressed" refers to an mRNA and/or protein, which in the respective bacteria is typically expressed at several thousand copies per cell.

The bacterial chemotaxis system (Lertsethtakam et al., 2011; Chevance FF and Hughes KT, Methods Mol Biol. 2017;1593:47-71) is less conserved and can involve a few to dozens of chemoreceptors and signaling proteins. The core signal transduction molecules include (i) chemoreceptors, (ii) the CheA kinase, (iii) the CheY response regulator, and (iv) the CheW coupling protein, which physically couples receptors to CheA.

The direction of flagellar rotation is controlled by the phosphorylation status of CheY. The amount of CheY-P is regulated, *inter alia,* by ligand binding to the chemoreceptors, such that an attractant ligand squelches CheA kinase activity and nonphosphorylated CheY predominates. In non phosphorylated form, CheY cannot interact with the flagellar switch, the flagella rotate counterclockwise, thus making the bacteria move forward. In the absence of an attractant ligand, CheA phosphorylates CheY. CheY-P binds the flagellar switch to turn the flagellum clockwise, which promotes bacterial tumbling and direction changes. Several chemotaxis proteins outside of the core chemotaxis set are commonly found in many but not all motile bacteria. The first set are phosphatases, such as CheZ and FliY, that accelerate the intrinsic dephosphorylation activity of CheY. The second set are the CheR and CheB enzymes that methylate and demethylate, respectively, chemoreceptor glutamyl residues to control adaptation responses to sustained ligand levels. The third set are the alternative coupling proteins such as CheV, which are homologs of CheW proteins with an additional receiver (REC) domain. *H. pylori* and *C. jejuni* collectively contain all of the above chemotaxis proteins and respond chemotactically to several different compounds.

Chemotactic signals are sensed by chemoreceptors, which are soluble or membrane bound multidomain proteins bearing a sensory domain and a signaling domain. *H. pylori* has four known chemoreceptors (also known as "Transducer-like proteins" (Tips) or "Methyl Accepting Chemotaxis Proteins" (MCPs)), TlpA, TlpB, TlpC and TlpD, which are involved in sensing and mediating responses to different environmental signals (e.g. arginine, low pH or energy levels). C. *jejuni* has ten known chemoreceptors. Among which are CetA and CetB, which function together and sense pyruvate and fumarate, the chemoreceptor Tlp7, which senses formate, and Tlp1, which senses aspartate as a chemoattractant. Mutagenesis studies in *H. pylori* and C. *jejuni* have shown or suggested that the functional expression of these chemoreceptors has a critical impact on host colonization.

Chemoreceptors communicate their ligand binding information to the flagellar motor via the core signal transduction proteins CheA, CheW and CheY (as described above). CheA has been shown to be highly critical for chemotaxis due to its role in promoting CheY phosphorylation (which in turn controls the flagellar rotation), and studies with *cheA* mutants have been shown to be non-chemotactic *in vitro* and to have defects in the colonization of animals. CheW is a coupling protein which links chemoreceptors with CheA to modulate the kinase activity of the latter, and studies have shown that CheW is essential for chemotaxis in *H. pylori* and *C. jejuni.* CheY, which is highly homologous in *H. pylori* and C. *jejuni,* are known to interact with FliM (part of the flagellar C ring or switch complex; described above) in a

phosphorylation dependent manner.

The activity of the above-described core chemotaxis proteins is affected by a multitude of accessory chemotaxis proteins, not found in all chemotactic bacteria, including CheY-P phosphatases, adaptation-related CheR methyltransferases and CheB methylesterases, and CheV CheW-REC hybrid coupling proteins: CheY-P phosphatases, by regulating the dephosphorylation of CheY, play a critical role in controlling flagellar rotation. These enzymes include CheZ (e.g., in *H. pylori, C. jejuni* and *E. coli*) and FliY (part of the flagellar C ring in *H. pylori,* and *C. jejuni*). In *H. pylori, fliY* mutants have been shown to be nonmotile. CheR methyltransferases and CheB methylesterases are functionally antagonistic, acting in demethylating and methylating chemoreceptors for adapting their activity. Whereas most *Epsilonproteobacteria* encode CheB, CheR, and CheV, *H. pylori* lacks CheB and CheR and instead has three CheV proteins (CheV1, CheV2 and CheV3).

The signal from the chemoreceptor complex is relayed to the flagellar motor via the switch complex that consists of FliG, FliM, and FliN (and additionally FliY in all *Epsilonproteobacteria,* including *H. pylori* and *C. jejuni*). Mutation of any one of these genes in *H. pylori* has been shown to result in nonmotile phenotypes. Mechanistically (as has been shown in *E. coli*), CheY-P binds to FliM and FliN to control the rotational direction of the flagellar rotor FliG.

The term "bacterial chemotaxis gene", as used herein, refers to any gene whose expression is involved in the biosynthesis and/or function of the chemotaxis system in the flagellated bacteria. Such genes may encode a protein which itself forms a structural component of said chemotaxis system (denoted hereafter as "structural chemotaxis gene" and "structural chemotaxis protein", respectively), and/or which acts as an accessory protein (e.g. a chaperone) for such a structural chemotaxis protein during translation, secretion and/or assembly (designated hereafter as "non-structural chemotaxis gene" and "non-structural chemotaxis protein", respectively). "Non-structural chemotaxis genes" also include genes encoding a transcriptional or translational regulator of a structural or non-structural chemotaxis protein, wherein said transcriptional or translational regulator may also be an RNA (e.g. a non-coding RNA) or a protein (e.g. a transcription factor). "Non-structural chemotaxis genes" also refer to genes encoding enzymes involved in post-translational modification of structural chemotaxis proteins, for example, enzymes involved in the phosphorylation/de-phosphorylation, methylation/de-methylation or glycosylation.

The bacterial chemotaxis gene may be a structural chemotaxis gene. Various (structural) chemotaxis genes (or corresponding proteins) which can be employed in the herein described, but unclaimed method are known in the art, and exemplary representatives are described herein above.

The structural chemotaxis gene may encode a chemoreceptor, preferably selected from the group of Tlps/MCPs (as described above); more preferably, the chemoreceptor is selected from TlpA, TlpB, TlpC and TlpD (e.g., from *H. pylori*).

The structural chemotaxis gene may encode a core signal transduction protein, preferably selected from CheA, CheW and CheY.

The term "culturing", as used herein, refers to maintaining a population of bacterial cells under conditions suitable for growth (e.g., at "growth conditions", defined below) using a suitable culture medium (e.g., liquid, solid, or semi-solid media) which contains all elements required by the bacteria for growth, such as a carbon source, water, various salts and a source of amino acids and nitrogen.

The term "growth conditions", or "non-stress conditions" as interchangeably used herein, refers to such culture conditions that facilitate growth (cell proliferation or cell division) of the bacterial cells, and wherein environmental stressors (e.g., extreme temperature or pH, osmotic stress, physical/mechanical stress (e.g., shearing forces), extreme pressure, presence of biocides, chemical stress, nutritional limitation, etc.) that may cause a cell to enter a resting state (cell arrest) or even cell death, are minimized or entirely eliminated. "Growth conditions" are generally defined by the culture medium and the surrounding (e.g., material, shape and size of the culturing vessel (e.g. flask or microwell plate, etc.) and the culturing device (e.g. shaker (shaking speed/amplitude) or static incubator), temperature, atmosphere gas composition (aerobic or anaerobic conditions, dependent of $N_2$, $CO_2$ and/or $O_2$ levels), atmospheric pressure, etc.) at which the bacteria are cultured. It is, however, understood that, when selecting appropriate "growth conditions", any effect(s) from one or more of the test compounds (e.g., bacteriostatic and/or bactericidal effect) also present in the culture is/are not be taken into account.

For most bacteria, suitable growth conditions are known in the art and may, if required, be optimized by adjusting individual components and/or conditions by routine measures. The skilled person is aware that the particular "growth conditions" may differ depending on conditions at the natural habitat of the particular kind of bacteria. Exemplary growth conditions suitable for *H. pylori* are specified in the appended examples (see Example 1).

The term "expression level", as used herein in the context of determining the expression level of a bacterial flagellar gene or of a bacterial chemotaxis gene, refers to the level (or amount) of expression of said bacterial flagellar gene or said bacterial chemotaxis gene. The act of actually determining (*i.e.*, detecting and/or quantifying) the expression level refers to the act of actively determining whether said gene is expressed in a sample or not. This act can include determining whether the gene expression is up-regulated, down-regulated or substantially unchanged as compared to a control level expressed in a sample.

The expression level of a bacterial flagellar gene or bacterial chemotaxis gene can be determined by detecting the mRNA and/or protein level of said bacterial flagellar gene or bacterial chemotaxis gene.

The term "nucleic acid", as used herein interchangeably with the term "nucleotide sequence", refers to polynucleotides including deoxyribonucleic acid (DNA), such as cDNA or genomic DNA, and, where appropriate, ribonucleic acid (RNA). It is understood that the term "RNA" as used herein comprises all forms of RNA including mRNA.

The term "protein" describes a group of molecules which comprises the group of peptides, consisting of up to 30 amino acids, as well as the group of polypeptides, consisting of more than 30 amino acids. Proteins may further form dimers, trimers and higher oligomers, *i.e.*, consisting of more than one protein molecule. Protein molecules forming such dimers, trimers etc. may be identical or nonidentical. The corresponding higher order structures are, consequently, termed homo- or heterodimers, homo- or heterotrimers etc. Homo- or heterodimers etc. also fall under the definition of the term "protein". The term "protein" also refers to chemically or posttranslationally modified peptides and polypeptides.

Commonly used methods known in the art for the quantification of mRNA expression in a sample include, without limitation, RNA-sequencing (RNA-seq) (e.g., Stark, Grzelak & Hadfield, Nature Reviews Genetics (2019) 20:631-656), northern blotting and *in situ* hybridization (e.g., Parker and Barnes, Meth. Mol. Biol., 106:247-283, 1999); RNAse protection assays (e.g., Hod, Biotechniques, 13:852-854, 1992); and PCR-based methods, such as reverse transcription polymerase chain reaction (RT-PCR) (Weis et al., Trends in Genetics, 8:263-264, 1992) and real time quantitative PCR, also referred to as qRT-PCR). Alternatively, antibodies may be employed that can recognize specific mRNA. Representative methods for sequencing-based gene expression analysis include Serial Analysis of Gene Expression (SAGE), and gene expression analysis by massively parallel signature sequencing (MPSS).

General methods for mRNA and/or RNA (e.g., total RNA) isolation are well known in the art and are disclosed in standard textbooks of molecular biology, including Ausubel et al., Current Protocols of Molecular Biology, John Wiley and Sons (1997). In particular examples, RNA isolation can be performed using a purification kit, buffer set and protease obtained from commercial manufacturers, such as Qiagen, according to the manufacturer's instructions. Other commercially available RNA isolation kits include MASTERPURE™ Complete DNA and RNA Purification Kit (EPICENTRE™ Biotechnologies) and Paraffin Block RNA Isolation Kit (Ambion, Inc.). In the MassARRAY™ gene expression profiling method (Sequenom, Inc.), cDNA obtained from reverse transcription of total RNA is spiked with a synthetic DNA molecule (competitor), which matches the targeted cDNA region in all positions, except a single base, and serves as an internal standard. The cDNA/competitor mixture is amplified by standard PCR and is subjected to a post-PCR shrimp alkaline phosphatase (SAP) enzyme treatment, which results in the dephosphorylation of the remaining nucleotides. After inactivation of the alkaline phosphatase, the PCR products from the competitor and cDNA are subjected to primer extension, which generates distinct mass signals for the competitor- and cDNA-derived PCR products. After purification, these products are dispensed on a chip array, which is preloaded with components needed for analysis with matrix-assisted laser desorption ionization time-of-flight (MALDI-TOF) mass spectrometry analysis. The cDNA present in the reaction is then quantified by analyzing the ratios of the peak areas in the mass spectrum generated. For further details see, e.g., Ding and Cantor, Proc. Natl. Acad. ScL USA, 100:3059-3064, 2003. Other methods for determining mRNA expression that involve PCR include, for example, differential display (Liang and Pardee, Science, 257:967-971, 1992); amplified fragment length polymorphism (Kawamoto et al., Genome Res., 12:1305-1312, 1999); BEAD ARRAY™ technology (Illumina, San Diego, CA, USA; Oliphant et al., Discovery of Markers for Disease (Supplement to Biotechniques), June 2002; Ferguson et al., Anal. Chem., 72:5618, 2000; and Examples herein); XMAP™ technology (Luminex Corp., Austin, TX, USA); BADGE assay (Yang et al, Genome Res., 11 :1888-1898, 2001); and high-coverage expression profiling (HiCEP) analysis (Fukumura et al., Nucl. Acids. Res., 31(16):e94, 2003).

Differential gene expression can also be determined using microarray techniques. In these methods, specific binding partners, such as probes (including cDNAs or oligonucleotides) specific for RNAs of interest or antibodies specific for proteins of interest are plated, or arrayed, on a microchip substrate. The microarray is contacted with a sample containing one or more targets (e.g., mRNA or protein) for one or more of the specific binding partners on the microarray. The arrayed specific binding partners form specific detectable interactions (e.g., hybridized or specifically bind to) with their cognate targets in the sample of interest.

Serial analysis of gene expression (SAGE) is a method that allows the simultaneous and quantitative analysis of a large number of gene transcripts, without the need of providing an individual hybridization probe for each transcript. In the SAGE method, a short sequence tag (about 10-14 bp) is generated that contains sufficient information to uniquely identify a transcript, provided that the tag is obtained from a unique position within each transcript. Then, many transcripts are linked together to form long serial molecules, that can be sequenced, revealing the identity of the multiple tags simultaneously. The expression pattern of any population of transcripts can be quantified by determining the abundance of individual tags, and identifying the gene corresponding to each tag (see, e.g., Velculescu et al., Science, 270:484-487, 1995, and Velculescu et al., Cell, 88:243-51, 1997).

Gene expression analysis by massively parallel signature sequencing (MPSS) was first described by Brenner et al. (Nature Biotechnology, 18:630-634, 2000). It is a sequencing approach that combines non-gel-based signature sequencing with *in vitro* cloning of millions of templates on separate 5 μm diameter microbeads. A microbead library of

DNA templates is constructed by *in vitro* cloning. This is followed by the assembly of a planar array of the template-containing microbeads in a flow cell at a high density. The free ends of the cloned templates on each microbead are analyzed simultaneously using a fluorescence-based signature sequencing method that does not require DNA fragment separation.

Differential gene expression can also be determined using *in situ* hybridization techniques, such as fluorescence *in situ* hybridization (FISH) or chromogen *in situ* hybridization (CISH). In these methods, specific binding partners, such as probes labeled with a flouorphore or chromogen specific for a target cDNA or mRNA (e.g., a GASI, TKI, or WNT5A cDNA or mRNA molecule) is contacted with a sample, such as a bacterial cell (or lysate thereof) sample mounted on a substrate (e.g., glass slide). The specific binding partners form specific detectable interactions (e.g., hybridized to) their cognate targets in the sample. For example, hybridization between the probes and the target nucleic acid can be detected, for example, by detecting a label associated with the probe. In some examples, microscopy, such as fluorescence microscopy, is used.

Methods to measure protein (*i.e.*, peptide/polypeptide) expression levels include, but are not limited to: Western blot, immunoblot, enzyme-linked immunosorbant assay (ELISA), radioimmunoassay (RIA), immune-precipitation, surface plasmon resonance, chemiluminescence, fluorescent polarization, phosphorescence, immunohistochemical analysis, liquid chromatography mass spectrometry (LC-MS), matrix-assisted laser desorption/ionization time-of-flight (MALDI-TOF), mass spectrometry, microcytometry, microarray, microscopy, fluorescence activated cell sorting (FACS), flow cytometry, and assays based on a property of the protein including but not limited to DNA binding, ligand binding, or interaction with other protein partners.

The expression level of a bacterial flagellar gene or bacterial chemotaxis gene may also be determined by detecting a signal provided by a reporter gene construct, wherein the reporter gene construct comprises a nucleotide sequence encoding a reporter gene product operatively linked to a promoter of a bacterial flagellar gene or of a bacterial chemotaxis gene.

The term "reporter gene product", as used herein, refers to any transcriptional or translational product (including mRNA and proteins/polypeptides) that (i) is not endogenously present in the tested host cells and (ii) provides a detectable and/or quantifiable signal. The signal may be detected/quantified directly, by the presence or absence of the reporter gene product, and/or indirectly, by the activity (e.g. enzymatic activity) of the reporter gene product.

The term "operably linked" refers to the association of nucleic acid sequences on a single nucleic acid fragment so that the function of one is affected by the other. For example, a promoter is operably linked with a coding sequence (e.g., a reporter gene) when it is capable of affecting the expression of that coding sequence (*i.e.*, that the coding sequence is under the transcriptional control of the promoter).

Suitable reporter genes and products thereof (also herein commonly referred to as "reporters") which may be used in the described method are known in the art. For example, a variety of enzymes may be used as reporters including, but not limited to, chloramphenicol acetyltransferase (CAT) (Gorman, et al. (1982) Molecular Cell Biology 2:1044; Prost, et al. (1986) Gene 45:107-111), β-galactosidase (Nolan, et al. (1988) Proc. Natl. Acad. Sci. USA 85:2603-2607), β-lactamase, β-glucuronidase and alkaline phosphatase (Berger, et al. (1988) Gene 66:1-10; Cullen, et al. (1992) Methods Enzymol. 216:362-368). Transcription of the reporter gene leads to production of the enzyme in test cells. The amount of enzyme present may then be measured via its enzymatic action on a substrate resulting in the formation of a detectable reaction product. Methods for determining the amount of reaction product are known in the art. Such methods either detect the amount of reaction product generated or the remaining amount of substrate which is related to the amount of enzyme activity. For some enzymes, such as β-galactosidase, β-glucuronidase and β-lactamase, well-known fluorogenic substrates are available that allow the enzyme to convert such substrates into detectable fluorescent products.

A variety of bioluminescent, chemiluminescent and fluorescent proteins may be used as light-emitting reporters.

Exemplary light-emitting reporters, which are enzymes and require cofactor(s)/substrate(s) to emit light, include, without limitation, the bacterial luciferase (luxAB gene product) of *Vibrio harveyi* (Karp (1989) Biochim. Biophys. Acta 1007:84-90; Stewart, et al. (1992) J. Gen. Microbiol. 138:1289-1300), the luciferase from firefly, *Photinus pyralis* (De Wet, et al. (1987) Mol. Cell. Biol. 7:725-737), *Gaussia* luciferase (GLuc), *Renilla* luciferase (RLuc), *Oplophorus* luciferase (OLuc) and engineered luciferases, such as NanoLuc® (Nluc) luciferase (Promega). Other types of light-emitting reporters, which do not require substrates or cofactors include, without limitation, the wild-type green fluorescent protein (GFP) of *Aequorea victoria* (Chalfie, et al. (1994) Science 263:802-805), modified GFPs (Heim, et al. (1995) Nature 373:663-4; Dammeyer T et al., Comput Struct Biotechnol J. 2012 22;3), and the gene products encoded by the *Photorhabdus luminescens* lux operon (luxABCDE) (Francis, et al. (2000) Infect. Immun. 68(6):3594-600). Transcription and translation of these types of reporter genes leads to the accumulation of the fluorescent or bioluminescent proteins in test cells, which may be measured by a device, such as a fluorimeter, flow cytometer, or luminometer. Methods for performing assays on fluorescent materials are well-known in the art (e.g., Lackowicz, 1983, Principles of Fluorescence Spectroscopy, New York, Plenum Press).

In the herein disclosed examples, a bacterial reporter strain was employed having a chromosomally integrated luciferase gene (encoding LuxAB luciferase from *V. harveyi*) operably linked to the promoter region of the major flagellin

A. Accordingly, based on this particular setup, upon transcription, the luciferase is intracellularly expressed. As this enzyme, for producing a luminescence signal, requires substrate conversion, the bacterial cells have been lysed in the presence of the added substrate decanal (Sigma) prior to luminescence measurement (see Example 1). Accordingly, it is understood that in certain cases, e.g. where a reporter gene product is intracellularly expressed in the test cells and/or where the reporter gene product requires a co-factor/substrate for producing a signal through enzymatic conversion of (or interaction with) that co-factor/substrate, the bacterial cells may be lysed/disrupted before the detection of the signal provided by the reporter gene product. Suitable methods for bacterial cell disruption are known by the skilled person, including, without limitation, mechanical/physical, chemical and enzymatic techniques, including, without limitation, high pressure homogenizer (e.g. French press), bead mill, liquid homogenization, high frequency sound waves (sonication), osmotic shock, freeze/thaw cycles, and manual grinding (Islam, MS, et al. Micromachines (2017); 8(3): 83. A Review on Macroscale and Microscale Cell Lysis Methods).

However, in cases where the reporter gene product is extracellularly expressed, *i.e.,* solubly expressed and secreted into the surrounding culture medium (e.g., due to the presence of signal sequence) and/or expressed and secreted as part of a soluble or membrane-bound fusion protein (e.g., a luminescence protein fused to an extracellular structural flagellar protein), and/or does not require a substrate/co-factor for producing a detectable signal (e.g., GFP), no lysis step may be required for detection of the reporter signal.

Compounds, or interchangeably referred to herein as "test compounds", can include proteins (including post-translationally modified proteins), peptides (including chemically or enzymatically modified peptides), lipids, carbohydrates, DNA, RNA, aptamers or small molecules (including antibodies, hormones, monosaccharides, oligosaccharides, glycans, lipids, glycolipids, steroids, anions or cations, drugs, small organic molecules, oligonucleotides, and genes encoding proteins of the agents or antisense molecules), including libraries of compounds. The test compounds can be naturally occurring (e.g., found in nature or isolated from nature) or can be non-naturally occurring (e.g., synthetic, chemically synthesized or man-made). If desired, test compounds can be obtained using any of the numerous combinatorial library methods known in the art, including but not limited to, biological libraries, spatially addressable parallel solid phase or solution phase libraries, synthetic library methods requiring deconvolution, the "one-bead one-compound" library method, and synthetic library methods using affinity chromatography selection. The biological library approach is limited to polypeptide libraries, while the other four approaches are applicable to polypeptide, non-peptide oligomer, or small molecule libraries of compounds. See Lam, Anticancer Drug Des. 12, 145, 1997. Methods for the synthesis of molecular libraries are well known in the art (see, for example, DeWitt et al., Proc. Natl. Acad. Sci. U.S.A. 90, 6909, 1993; Erb et al. Proc. Natl. Acad. Sci. U.S.A. 91, 11422, 1994; Zuckermann et al., J Med Chem. 37,2678, 1994; Cho et al., Science 261, 1303, 1993; Carell et al., Angew. Chem. Int. Ed Engl. 33,2059,1994; Carell et Angew. Chem. Int. Ed. Engl. 33, 2061; Gallop et J. Med Chem. 37, 1233, 1994). Libraries of compounds can be presented in solution (see, e.g., Houghten, BioTechniques 13, 412-421, 1992), or on beads (Lam KS, Nature 354, 82-84, 1991), chips (Fodor, Nature 364, 555-556, 1993), bacteria or spores, plasmids (Cull et Proc. Natl. Acad Sci. U.S.A. 89, 1865-1869, 1992), or phage (Scott & Smith, Science 249, 386-390, 1990; Devlin, Science 249, 404-406, 1990); Cwirla et al., Proc. Natl. Acad. Sci. 97, 6378-6382, 1990; Felici, J. Mol. Biol. 222, 301-310, 1991).

The term "bacteriostatic", as used herein, refers to the capacity of inhibiting the growth (cell division) of the bacteria, whereas the term "bactericidal" refers to the capacity of killing (or essentially killing) the bacteria.

Presence or absence of bacterial "growth" (also interchangeably referred to herein as "growth activity") can be determined and monitored by various methods well known in the art. For example, bacterial growth can be determined by assessing the amount of bacterial cells after different time intervals of culturing. The amount of bacterial cells can be assessed, for example, by turbidity or optical density (OD) measurements of bacteria in suspension and uses a photometer (also referred to as spectrophotometer). The OD may be measured at 600 nm (also designated herein as "O.D. 600," "o.d. 600," "OD600" etc.). In addition, physical measurements (e.g., dry weight of cells following centrifugation) or chemical measurements (e.g., rate of oxygen consumption, nitrogen, protein or DNA content) can be used to determine and/or monitor bacterial growth in a culture. Also, plating to determine colony forming units (CFUs) or to observe an increase in colony diameter over time is another standard method for determining and/or monitoring bacterial growth in a culture.

In accordance with the herein described, yet unclaimed method, an absence of growth-inhibition may be determined in step (d), if the bacteria cultured in the presence of the test compound possess, with increasing preference, at least 70%, at least 75%, at least 80%, at least 85%, at least 90%, at least 95%, at least 98%, at least 99%, and most preferably at least 100% of growth-activity as compared to a control, wherein said control comprises bacteria of the same species cultured in the absence of the test compound. Methods for assessing the presence or absence of bacterial growth are described above.

In accordance with step (d), test compounds having "substantially no bacteriostatic activity", may preferably refer to such compounds having no detectable growth-inhibitory effect on the bacteria, but may also refer to such compounds which have only a minor growth-inhibitory effect on said bacteria, meaning that the bacteria, in the presence of the test compound, possess, with increasing preference, at least 70%, at least 75%, at least 80%, at least 85%, at least 90%, at least 95%, at least 98%, at least 99%, and most preferably at least 100% of growth-activity as compared

to in the absence of the test compound.

In order to identify those test compounds having (exclusively) bacterial anti-motility activity and to sort out those test compounds having anti-viability effects (e.g. growth-inhibitory activity (bactericidal and/or bacteriostatic activity)), instead of assessing the presence or absence of growth-inhibition (in steps (d) and (e)), alternative secondary assays (or counter-screens) can be employed, including, without limitation, metabolic tests (respiratory inhibition assays), ATP (BacTiter Glo) content assay (e.g. BacTiter-Glo™ Microbial Cell Viability Assay (Promega; Madison WI, USA)), or MIC assay, as also referred to herein (e.g. Example 2).

The method may comprise the additional step (e) of determining the identity and/or structure of (i) the test compound having a bacterial anti-motility activity; and/or (ii) a target molecule of said test compound, preferably the binding epitope of said test compound on said target molecule. Methods for determining the identity and/or structure of a test compound are known in the art, including, without limitation, mass spectroscopy, NMR spectroscopy, vibrational spectroscopy, or X-ray crystallography.

The term "target molecule", as referred to herein, refers to an endogenous molecule of the bacteria with which a test compound having a bacterial anti-motility activity interacts, which interaction is causative for, or at least contributing to, the anti-motility effect of the test compound.

The term "binding epitope", as interchangeably used herein with the terms "binding site" or "interaction site", refers to a region on the target molecule (such a region may be, e.g., constituted of certain amino acid residues and/or posttranslational modifications or portions thereof on a protein, or certain nucleotides or portions thereof on a nucleic acid) that binds to or interacts with the test compound.

Methods for determining the identity of a target molecule of a test compound are also well-known in the art. For example, bacteria that have been cultured in the presence of a test compound having a bacterial anti-motility activity can be subjected to transcriptional and/or translational expression analysis, wherein mRNA or protein expression level(s), respectively, of one or more selected gene(s) (e.g., certain flagellar or chemotaxis genes), which are suspected as potential target molecule(s), is/are determined. Alternatively, the bacteria may be subjected to transcriptomic and/or proteomic profiling. A detected mRNA and/or protein level which may be up- or down-regulated as compared to bacteria cultured in the absence of the test compound may then be indicative of a potential target molecule. Once a potential target molecule has been identified, further known methods (e.g., biochemical, biophysical and/or structural techniques) can be used to verify/confirm an interaction between the test compound and said target molecule. The letter methods may also be used for identifying a binding epitope of the test compound on said target molecule.

Others have recently, in parallel with the present inventors, endorsed the concept of anti-motility therapies against various human pathogens (reviewed, e.g., in Erhardt 2016). For example, *Menard* and colleagues used an *in vitro* approach for screening and identifying compounds targeting the biosynthetic pathway of pseudaminic acid (Pse), a sugar, which in *H. pylori* and *C. jejuni* is *O*-glycosidically attached to the major flagellin proteins and thought to be required for flagellin assembly and consequent motility. In this assay, five isolated Pse biosynthetic enzymes were combined in a single cell-free *in vitro* reaction and their activity or inhibition was assessed in the presence of test compounds through detection of phosphate which is usually released in the final enzymatic step upon substrate conversion (Menard et al., 2014). The authors reported their finding of few compounds which inhibited flagellin production in subsequent cellular assays, and without inhibiting bacterial growth (yet, corresponding growth data has not been shown). However, this assay design suffers from several limitations and disadvantages. For example, its sensitivity for identifying agents having antiflagellar/anti-motility activity is restricted to compounds interfering with these five particular enzymes (on protein level), thereby excluding any potential direct or indirect effects that, in the more complex cellular context, may arise from an inhibition of other target molecules further upstream in the flagellar expression cascade (including also regulators), also putting in general question whether assaying these enzymes in cell-free can be reflective of their cellular activity. In addition, complex recombinant expression and purification of these enzymes has to be conducted, which may not be adaptable to other bacterial systems and renders this assay costly and unsuitable, particularly for applications wherein several different bacterial species should be analyzed.

In a different study by *Johnson* and colleagues, a high-throughput screening (HTS) assay was reported based on an engineered *C. jejuni* strain having a reporter gene (encoding chloramphenicol acetyltransferase (CAT)) under control of the *flgDE2* promoter (the *flgDE2* operon in *C. jejuni* comprises the genes encoding the putative hook proteins FlgD and FlgE2). By this setup, bacterial growth/survival in chloramphenicol-comprising selection medium was rendered dependent on the activity of the flgDE2 promoter, which activity was assessed in the presence of a test compound (Johnson, Yuhas, McQuade, Larsen, & DiRita, 2015). However, despite of the screening of a large number of test compounds, only such compounds having primarily classical antibacterial (growth-inhibitory) activity were identified.

Here, the present inventors set out to develop a screening method for identifying novel compounds exclusively inhibiting motility-associated functions in flagellated bacteria without exerting anti-viability effects for being used as a new therapeutic approach for treating and/or preventing corresponding bacterial infections and associated conditions, in particular for those involving *H. pylori.* A powerful reporter-based screening system for detecting diverse antiflagellar/antimotility effects was developed, suitable for high-throughput screening (HTS) applications.

By using the herein described method for screening approximately 4,000 small compounds from various compound libraries, various compounds could be identified which had strong anti-bacterial and/or anti-flagellar effects on *H. pylori.* Several primary actives had low (very effective) antibacterial "minimum inhibitory concentration" (MIC) values and acted on *H. pylori* at different levels, probably via metabolic inhibition. However, most strikingly, several compounds were identified that exclusively inhibited *H. pylori* motility without inhibiting viability (i.e., bacterial growth and/or survival). A subsequent proof-of-principle treatment study in an animal model of *H. pylori* chronic infection confirmed that the compounds identified exhibit significant *in vivo* activity and could reduce bacterial counts in a persistent *H. pylori* infection without harming the microbiota richness or diversity *in vivo,* thereby demonstrating that this screening approach is capable of identifying potent and selective antimotility agents for treating *H. pylori* infection.

In order to be indicative of a test compound to having bacterial anti-motility activity and/or bacteriostatic activity and/or bactericidal activity, the reduction in the expression level determined in step (b) in the presence of a test compound may be, with increasing preference, at least 10%, at least 20%, at least 30%, at least 40%, at least 50%, at least 60%, at least 70%, at least 75%, at least 80%, at least 85% and most preferably at least 90% compared to the expression level determined in bacteria of the same species not cultured in the presence of said test compound. As shown in the appended Examples, a cut-off of at least 30% reduction of detected fluorescence reporter signal (inhibition >30%) was effective to select compounds having an anti-flagellar activity.

The time period of the culturing of the flagellated bacteria in step (a) before determining the expression level of the bacterial flagellar gene in step (b) may be chosen to be significantly shorter than the time period required by bacteria of the same species cultured at identical culture conditions in the absence of the test compound, to undergo a detectable growth (e.g., increase in cell number/density) by more than, with increasing preference, 50%, 40%, 30%, 20%, 10%, 5%, 4%, 3%, 2%, 1% (from the starting point of the culturing). As shown in appended Examples 1 and 2, the culturing time of the *H. pylori* reporter strain before the quantification of the reporter signal was 4h at 37 °C, which is significantly shorter than the expected duplication time of *H. pylori* at the applied culture conditions. This setup was chosen by the present inventors in order to limit the contribution arising from growth effects (an increase in the number of cells) to the detected reporter signal, which rendered this primary assay more sensitive toward detecting anti-flagellar effects.

The bacterial anti-motility activity may be an inhibition of (a) motility; (b) chemotaxis; (c) flagellar biosynthesis, secretion and/or assembly; and/or (d) flagellar gene regulation; by at least 30% as compared to in absence of the test compound.

The bacterial flagellar gene may be a late-stage bacterial flagellar gene; and preferably the late-stage bacterial flagellar gene is a structural flagellar gene.

The term "late" or "late-stage" bacterial flagellar gene as interchangeably used herein, refers to such bacterial flagellar genes which are expressed late in the regulatory cascade of flagellar biosynthesis (as described above). In general, late genes are down-regulated in bacteria defective in early or middle genes, and neither middle nor late genes are expressed in bacteria defective in early class genes. Accordingly, due to the position of the bacterial flagellar gene as "late-stage" gene in the hierarchical cascade of flagellar gene regulation, assessing the expression of such a late-stage bacterial flagellar gene is capable of detecting potential effects of a test compound on multiple different pathways affecting flagellar gene regulation, assembly and substrate secretion that converge at the inhibition of flagellar motility.

The "late-stage bacterial flagellar gene" may be a gene whose expression is controlled by a sigma factor 28 ($\sigma^{28}$)-dependent promoter and/or is a "class 3 gene" (as described above).

The "late-stage bacterial flagellar gene" may be a gene encoding a filament protein.

The "late-stage bacterial flagellar gene" may be a gene encoding a flagellin, preferably a major structural flagellin, and even more preferably a flagellin A (FlaA), flagellin B (FlaB), or any other orthologous flagellin subunit gene.

Flagellin is the building block of the flagellar filament. In some bacteria, the filaments consist of a single kind of flagellin; in others they consist of several kinds of flagellin which are closely related isomers. The flagellin encoding gene (initially termed *hag* (from H antigen)) has different names in various bacterial species, for example, without limitation, *flaA, flaB* (e.g., in *Helicobacter spp., Camylobacter spp., Vibrio cholerae), fliC* (e.g., in *Pseudomonas aeruginosa, Salmonella spp.,* and *Eschericha spp*.). Depending on the targeted flagellated bacterial species, any of these flagellin genes and orthologs thereof may be employed in the herein described method, and the skilled person will be able to select the respective genes based on available literature.

The bacterial flagellar gene or bacterial chemotaxis gene may be from (a) *Helicobacter spp., preferably from Helicobacter pylori;* or (b) *Campylobacter spp.,* preferably *Campylobacter jejuni.* As shown in the appended Examples, the method successfully identified anti-motility compounds in the context of *Helicobacter pylori.* It is therefore expected that the approach will be effective for *Epsilonproteobacteria* in general, in particular for *Helicobacter spp. and Campylobacter spp.*.

The flagellated bacteria may be *Helicobacter spp.,* preferably *H. pylori,* and the "bacterial flagellar gene" may be one encoding the major flagellin FlaA.

The reporter gene product may be selected from the group consisting of *Vibrio* luciferase, firefly luciferase,

Gaussia luciferase (GLuc), *Renilla* luciferase (RLuc), *Oplophorus* luciferase (OLuc), NanoLuc luciferase (NLuc), jellyfish aequorin, green fluorescent protein (GFP), yellow fluorescent protein (YFP), cyan fluorescent protein (CFP), red fluorescent protein (RFP), mCherry, mOrange, mRaspberry, mKO, TagRFP, mKate, mRuby, FusionRed, mScariet, DsRED, DsRed-Express, chloramphenicol acetyltransferase (CAT), β-galactosidase, and alkaline phosphatase.

The reporter gene product may be a luciferase. As shown in the appended examples, the bacterial luxAB luciferase from *Vibrio harveyi* encoded by the luxAB operon proved to be particularly effective as a reporter gene product in the herein described method. It is thus expected that also other luciferase enzymes (e.g., as described above) are suitable.

The reporter gene product may be LuxAB luciferase from *Vibrio harveyi.*

The reporter gene construct may be (a) integrated into the bacterial genome; or (b) on a plasmid vector transformed into the bacteria.

As shown in the examples, a reporter gene construct which was integrated into the bacterial genome (the bacterial chromosome) was particularly effective for sensing the flagellar gene expression level. It is conceivable that a system where the reporter gene construct is instead present on a plasmid vector transformed into the flagellated bacteria will also be functional, although, such a plasmid-based system can be expected to be less stable. Thus, it is most preferred that the reporter gene construct is integrated into the bacterial genome. Methods for stably integrating a gene into a bacterial chromosome, e.g. by homologous recombination, are well known in the art.

It is understood that the promoter of the bacterial flagellar gene or of the bacterial chemotaxis gene comprised in the reporter gene construct corresponds to a promoter sequence that is either endogenously found in the respective flagellated bacteria subjected to the herein described method, or is at least so closely related to such an endogenously found promoter so that it is capable of being regulated by the same mechanisms/factors regulating the activity of said endogenous promoter.

Two or more different bacterial species may be employed in (i) steps (a) to (d); or in (ii) steps (c) to (d) of the method.

Conducting the method in the presence of two or more different bacterial species in steps (a) and (d) may allow to assess a potential effect of a test compound on several different target bacterial species in parallel (e.g. in a single reaction vessel, or in a single well of a micro-well plate). Such a setup may particularly be useful in high-throughput approaches for assessing whether a test compound is active against several different bacterial strains (e.g., different pathogenic bacterial strains; or one or more pathogenic strains and one or more non-pathogenic strains (e.g. any beneficial bacteria of the resident microbiota)). In such cases where several different bacterial strains are employed together, different reporter genes may be used in these different bacterial strains in order to allow to resolve/assign the observed effects of a test compound to the individual bacterial strains (e.g., by using reporter genes providing distinguishable reporter gene products (e.g., differently coloured fluorescence signals)).

Steps (a) and (b) and/or steps (c) and (d) are conducted in the presence of more than one test compound (e.g., a group of structurally related compounds). Such a setup can, for example, be used as a preliminary screening of a large number of test compounds in high-throughput; once an activity has been determined for a group of compounds, the method can be repeated to verify potential activities of individual compounds.

The culturing in steps (a) and (c) may be conducted in multi-well plates; and/or in the presence of more than one test compound.

The term "multi-well plate", "micro-well plate" or "micro-titer plate" as interchangeably used herein, refers to a two-dimensional array of addressable wells located on a substantially flat surface. More specifically, a multi-well may refer to a plate of a suitable material, such as polystyrene, which comprises a discrete number of "wells" (cavities) for taking up samples, e.g., the bacterial cells in culture medium. Common examples are multi-well plates having 6, 12, 24, 48, 96, 384 or 1536 of such wells.

The herein described, yet unclaimed method may further comprise importing the information whether the test compound has a bacterial anti-motility activity and/or information on the structure of said test compound and/or its target molecule into a contracting state of the EPC in which the patent, once granted, has been validated.

The term "infection", "bacterial infection" or "microbial infection" as interchangeably used herein, refers to the presence of bacteria in or on a subject's body, which presence has a negative impact on the subject's health or bears a risk for negatively affecting the subject's health.

It will be appreciated that the medical uses and therapeutic applications of the invention are useful in the fields of human medicine and veterinary medicine. Thus, the "subject" or "individual" in accordance with the medical uses of the invention is a mammal, preferably human, or other animals. For veterinary purposes, subjects include, for example, farm animals such as cows, sheep, pigs, horses, and goats, and poultry such as chickens, turkeys, ducks, and geese; companion animals such as dogs and cats; exotic and/or zoo animals; laboratory animals including mice, rats, rabbits, guinea pigs, and hamsters.

In general, bacterial infection with flagellated bacteria are known to be associated with a multitude of acute and chronic disease conditions involving nearly every organ system or tissue, and for many of bacterial pathogens, flagellar

motility has been shown to be a critical determinant for host colonization and pathogenicity.

The term "therapeutically effective amount", "pharmaceutically effective amount" or "effective amount" as interchangeably used herein, refers to an amount, which has a therapeutic effect or is the amount required to produce a therapeutic effect in a subject. For example, a therapeutically effective amount of a compound or a pharmaceutical composition thereof is the amount of the compound or the pharmaceutical composition required to produce a desired therapeutic effect as may be judged by clinical trial results, model animal infection studies, and/or *in vitro* studies (e.g. in agar or broth media). The pharmaceutically effective amount depends on several factors, including but not limited to, the particular microorganism (e.g., bacteria) involved, characteristics of the subject (for example, weight, gender, age, and medical history), severity of infection and the particular type of the agent used. For prophylactic (i.e., preventive) application (or treatment), a therapeutically or prophylactically effective amount is that amount which would be effective in preventing a bacterial infection or in preventing of not yet occurring disease symptoms of an already prevalent infection.

The compound may be formulated for administration according to the invention as part of a pharmaceutical composition. For oral administration, the pharmaceutical composition may be formulated into solid dosage forms such as tablets, pills, powders, granules or capsules, etc., and these solid dosage forms may be prepared by mixing the compound of the invention with one or more excipients such as starch, calcium carbonate, sucrose or lactose, gelatine, etc. Moreover, lubricants such as magnesium stearate, talc, etc. can be used in addition to simple excipients. Furthermore, the pharmaceutical composition may be formulated into liquid dosage forms such as suspensions, liquid for internal use, emulsions, syrups, etc., and various excipients such as humectants, sweeteners, aromatics, preservatives, etc. in addition to water and liquid, paraffin may be used for the formulation of liquid dosage forms. For parenteral administration, the pharmaceutical composition is formulated generally by mixing the compound of the invention at the desired degree of purity, in a unit dosage injectable form (solution, suspension, or emulsion), with a pharmaceutically acceptable carrier, *i.e.*, one that is non-toxic to recipients at the dosages and concentrations employed and is compatible with other ingredients of the formulation.

Generally, the formulations are prepared by contacting the components of the pharmaceutical composition uniformly and intimately with liquid carriers or finely divided solid carriers or both. Then, if necessary, the product is shaped into the desired formulation. Preferably the carrier is a parenteral carrier, more preferably a solution that is isotonic with the blood of the recipient. Examples of such carrier vehicles include water, saline, Ringer's solution, and dextrose solution. Non-aqueous vehicles such as fixed oils and ethyl oleate are also useful herein, as well as liposomes. The carrier suitably contains minor amounts of additives such as substances that enhance isotonicity and chemical stability. Such materials are non-toxic to recipients at the dosages and concentrations employed, and include buffers such as phosphate, citrate, succinate, acetic acid, and other organic acids or their salts; antioxidants such as ascorbic acid; low molecular weight (less than about ten residues) peptides, e.g., polyarginine or tripeptides; serum albumin, gelatin; hydrophilic polymers such as polyvinylpyrrolidone; amino acids, such as glycine, glutamic acid, aspartic acid, or arginine; monosaccharides, disaccharides, and other carbohydrates including cellulose or its derivatives, glucose, mannose, or dextrins; chelating agents such as EDTA; sugar alcohols such as mannitol or sorbitol; counterions such as sodium; and/or nonionic surfactants such as polysorbates, poloxamers, or PEG.

The components of the pharmaceutical composition to be used for therapeutic administration must be sterile. Sterility is readily accomplished by filtration through sterile filtration membranes (e.g., 0.2 micron membranes). Therapeutic components of the pharmaceutical composition generally are placed into a container having a sterile access port, for example, an intravenous solution bag or vial having a stopper pierceable by a hypodermic injection needle.

The components of the pharmaceutical composition ordinarily will be stored in unit or multi-dose containers, for example, sealed ampoules or vials, as an aqueous solution or as a lyophilized formulation for reconstitution. As an example of a lyophilized formulation, 10-ml vials are filled with 5 ml of sterile-filtered 1% (w/v) aqueous solution, and the resulting mixture is lyophilized. The infusion solution is prepared by reconstituting the lyophilized compound(s) using bacteriostatic Water-for-Injection.

The compound according to the invention (or pharmaceutical composition comprising said compound) can be administered systemically (e.g., orally, rectally, parenterally (e.g., intravenously), intramuscularly, intraperitoneally, transdermally (e.g., by a patch), topically (as by powders, ointments, drops or transdermal patch), bucally, or as an oral or nasal spray, by inhalation, subcutaneously or the like), by administration into the central nervous system (e.g., into the brain (e.g., intracerebrally, intraventricularly or intracerebroventricular) or spinal cord or into the cerebrospinal fluid), or any combination thereof. However, oral administration routes are typically preferred, for reasons of a more convenient (e.g., less-invasive) administration (e.g., the possibility of self-administration) and particularly in cases where the targeted pathogenic bacteria (e.g., *H. pylon*) are present in the gastrointestinal tract.

The compound may be administered in one or more dose administrations, at least once daily, for one or more days (e.g., 1, 2, 3, 4, 5, 6, 7 days; 2, 3, 4 weeks; 2, 3, 4, 5, 6, 7, 8, 9, 10, 11, 12 months; 2 years, 3 years, 4 years, or longer). However, the skilled person understands that the duration of the administration, in accordance with the present invention, can optionally be sustained for the remaining lifetime of the subject. The compound may be administered daily or less frequently, for example, every-other-day, semi-weekly, weekly, biweekly, semi-monthly, monthly, bimonthly, etc.

An effective amount of the compound may be administered to a subject in need thereof once every 6 hours to once every 60 hours, more preferably once every 12 hours to once every 48 hours, and most preferred once every about 24 hours. In connection with the present invention, it was found that the treatment of *H. pylori*-infected mice with the compound was effective when administered once daily, as detailed in Example 3.

The compound may be administered for a period of at least 3 days, preferably at least 5 days, more preferably at least 7 days. As also apparent from Example 3, compound active 2/BL2 was therapeutically active (as concluded, for example, from an observed reduction of cfu counts in *H. pylori* infected BL2-treated mice) when the compound was administered once daily (at a single dose of 10 mg/kg/day) for 7 consecutive days.

The compound of the invention can be administered to the subject at a suitable dose and/or a therapeutically effective amount. This will be further discussed herein below.

The dosage of the compound of the invention required will somewhat vary from subject to subject, depending on the species, age, weight, and general condition of the subject, the particular active agent and pharmaceutical carrier used, the mode of administration and the like. For example, the dose of the compound when administered parenterally may be in the range of about 0.01 mg/kg to 1000 mg/kg of patient body weight (bd wt), although, as noted above, this will be subject to therapeutic discretion. More preferably, this dose is at least 0.5 mg/kg/day, and most preferably for humans between about 5 and 100 mg/kg/day. If given continuously, the pharmaceutical composition is typically administered at a dose rate of about 1 mg/kg/hour to about 50 mg/kg/hour, either by 1-4 injections per day or by continuous subcutaneous infusions, for example, using a mini-pump. An intravenous bag solution may also be employed. The length of treatment needed to observe changes and the interval following treatment for responses to occur appears to vary depending on the desired effect. The particular amounts may be determined by conventional tests which are well known to the person skilled in the art.

For determining therapeutically effective dosages for particular pharmaceutical formulations, for example, animal models (e.g., mice, gerbils, etc.) of bacterial infection (e.g., *H. pylori* chronic infection) can be used to monitor levels of response. Therapeutic efficacy can then be determined, for example, from a reduction of colony-forming units (cfus) of bacteria in infected and treated animals, over infected, non-treated animals; and potential effects on the residual microbiota can be assessed, for example, by 16S rRNA amplicon sequencing as described in the herein disclosed examples (cf. Example 1 (Methods) and Example 3).

As a general rule, the dosage ranges for the administration of the compound formulations or pharmaceutical compositions are those large enough to produce the desired effect in which the symptoms of the disease are affected. For example, functional improvement, histological improvement, gene expression, and the like can be affected and monitored to determine the desired outcome. The dosage should not be so large as to cause adverse side effects, such as unwanted cross-reactions and unwanted inflammatory reactions. Dosage can vary, dependent on the route of administration used.

The compound may be administered at a dose of between 0.1 mg/kg body-weight and 2000 mg/kg body-weight. In context of the present invention, the treatment of the mouse model with the compound was found to be effective at the tested dosage of 10 mg/kg mouse body-weight, as detailed in Example 3. It is, hence, expected that comparable dosages may generally be therapeutically effective in mammalian subjects, in particular in human subjects.

In accordance with the invention, the compound has an "anti-motility activity" (as defined herein above), preferably an "anti-flagellar activity" (as defined herein above). Means and methods for assaying the presence or absence of such activities are known in the art and described herein. For example, the herein disclosed *flaA*-luciferase assay (e.g., the luciferase / bioluminescence assay described in Examples 1 and 2) may be used for that purpose. Alternatively, the presence or absence of such activities can also be determined, for example, by (microscopical) motility tracking, e.g., as also described in the herein disclosed examples.

The compound may have a detectable inhibitory activity (*i.e.*, "anti-motility activity"), when assayed in an anti-motility assay as disclosed herein, for example, in the herein disclosed *flaA*-luciferase assay (e.g., described in Examples 1 and 2), wherein a decrease of a detected reporter gene signal (detected bioluminescence) in the presence of the compound as compared to the absence of said compound is indicative of an inhibitory activity of said compound. The skilled person knows that an $IC_{50}$ of such an assay can be determined as a measure of such an inhibitory activity.

The $IC_{50}$ (*i.e.*, the half maximal inhibitory concentration) is a commonly applied measure of the potency of a substance in inhibiting a specific biological or biochemical function (e.g., expression of the reporter gene in the *flaA*-reporter strain assayed by detection of the bioluminescence reporter signal). $IC_{50}$ is a quantitative measure that indicates how much of a particular inhibitory substance (e.g., drug/compound) is needed to inhibit, *in vitro,* a given biological process or biological component by 50%.

In accordance with the present disclosure, the term "detectable inhibitory activity" means that a corresponding compound has a maximum $IC_{50}$ of, with increasing preference, 100 μg/ml, 75 μg/ml, 50 μg/ml, 40 μg/ml, 30 μg/ml, 20 μg/ml, 10 μg/ml, 5 μg/ml, 4 μg/ml, 3 μg/ml, 2 μg/ml, 1 μg/ml, or 0.5 μg/ml, wherein the $IC_{50}$ is determined by an anti-motility assay, for example, using any of the herein disclosed *H. pylori* flaA-luciferase reporter strains for assaying the reporter signal in the presence (of gradually increased concentrations) or absence of a test compound (e.g. as described

in Example 1). The skilled person will be able to adapt such assays for assessing the presence or absence of an inhibitory activity of a compound in other target bacterial species.

For example, as described in Example 4 (using *H. pylori* flaA-luciferase reporter strain N6), Table 6, for compounds Active 2, DJ-1, DJ-2, DJ-5, DJ-6, DJ-7, DJ-8, DJ-9 and DJ-12, $IC_{50}$ values of below 4.5 $\mu$g/ml were determined; and for compound DJ-2 an $IC_{50}$ of 0.44 $\mu$g/ml was determined. For compounds DJ-10 and DJ-11 $IC_{50}$ values of 10.83 $\mu$g/ml and 10.56 $\mu$g/ml, respectively, were determined. Since lead compound Active 2 having an $IC_{50}$ of 2.75 $\mu$g/ml was particularly effective, it is expected that close derivatives from this initial lead compound will also be effective, particularly those compounds having $IC_{50}$ values in similar range. Similar $IC_{50}$ ranges were determined also for compounds Active 2 and Active 2a (DJ-1) assayed with a *H. pylori* flaA-luciferase reporter strain derived from the *H. pylori* strain L7 (cf. Example 6).

The compound may have an anti-motility activity (preferably, anti-flagellar activity) and no, or substantially no anti-viability effects (e.g., growth-inhibitory activity (bactericidal and/or bacteriostatic activity). The compound may have anti-motility (preferably anti-flagellar) and no (or substantially no) bacteriostatic activity and no bactericidal activity.

Means and methods for assaying the presence or absence of bacteriostatic or bactericidal activity of a compound on selected bacterial target species are known in the art and disclosed herein.

Known gastro-intestinal and/or gastro-duodenal pathogenic bacteria include, without limitation, *Helicobacter spp.,* (e.g. *Helicobacter pylon*), *Campylobacter spp.* (e.g. *Campylobacter jejuni*), *Staphylococcus spp.* (*Staphylococcus aureus*), *Bacillus subtilis,* enterohemorrhagic *Escherichia coli* (EHEC), enterotoxigenic *Escherichia coli* (ETEC), enteropathogenic *Escherichia coli* (EPEC), Shiga toxin-producing *Escherichia coli* (STEC), *Klebsiella pneumonia, Clostridium difficile, Salmonella spp., Shigella spp., Vibrio spp.,* and *Yersinia spp.* (e.g. *Yersinia enterocolitica*).

Urogenital pathogenic bacteria include, without limitation, $\beta$-hemoiytic *streptococci*, *Candida albicans, Klebsiella pneumoniae, Escherichia coli, Corynebacterium pyogenes, C. vaginale, Campylobacter spp., Chlamydia trachomatis, Neisseria gonorrhoeae.*

The optional limitation with respect to the flagellated bacteria to be "not susceptible to antibiotic treatment", refers to such bacteria which either (*i*) have acquired resistance to at least one antibiotic, wherein said antibiotic cannot exert its bactericidal and/or bacteriostatic effect it usually has on bacteria of the same species and/or genus without acquired resistance; or (*ii*) to such bacteria against which no known antibiotic is available.

Besides the most prominent representative *H. pylori,* other pathogenic *Helicobacter* species have been found in the stomach, intestine and biliary tract. For example, *H. heilmannii,* which is known to comprise several species including *H. bizzozeronii* and *H. salmonis,* has been found in the stomach and is also suspected to cause the same diseases as *H. pylori.* Other species that infect humans include *H. cinaedi, H. pullorum, H. canis, H. canadensis, H. fennelliae, H. rappini, H. bilis, H. hepaticus,* and *H. suis* (Testerman et al., World J Gastroenterol (2014); 20(36): 12781-12808). As shown in appended Example 3, the compound (BL2) identified by the present invention proved to be therapeutically effective in the treatment of a *H. pylori* chronic infection animal model. It is hence expected that the same compound or closely related derivatives will also be effective in the treatment of other pathogenic *Helicobacter spp.*.

*Campylobacter species* are Gram-negative, microaerophilic, non-fermenting, flagellated bacteria of the class *Epsilonproteobacteria. Campylobacter spp.* cause acute gastroenteritis with diarrhea, abdominal pain, fever, nausea, and vomiting. Recently, *Campylobacter* infections have been identified as the most common antecedent to an acute neurological disease, the Guillain-Barré syndrome. These bacteria colonize the small and large intestines, causing inflammatory diarrhea with fever. *C. jejuni* and *C. coli* infections are endemic worldwide and hyperendemic in developing countries. Infants and young adults are most often infected. Disease incidence peaks in the summer. Domestic and wild animals are the reservoirs for the organisms. Outbreaks are associated with contaminated animal products or water (Medical Microbiology. 4th Ed., Univ. of Texas Medical Branch at Galveston, 1996. Chapter 23; Rosner BM, et al., Sci Rep. 2017;7(1):5139). In addition, *Campylobacter* infection can also cause neurological complications, such as reactive arthritis, Guillain-Barré Syndrome (GBS) and Miller-Fisher Syndrome. The latter two can be very severe and even lead to death of the patients.

The bacterial pathogen *Helicobacter pylori (H. pylon)* commonly colonizes the human gastric mucosa during early childhood and persists throughout life. This organism has evolved multiple mechanisms for evading clearance by the immune system and, mainly by inducing inflammation in the stomach, is thought to be causative for a multitude of serious disease conditions (Testerman et al., World J Gastroenterol (2014); 20(36): 12781-12808; White et al., J Inflamm Res. (2015); 8: 137-147; Suerbaum and Michetti, N Engl J Med. 2002;347(15):1175-86).

For example, *H. pylori* infection is known as leading cause of peptic ulcer disease and gastric cancer. Peptic ulcers are sores that develop in the lining of the stomach or upper part of the small intestine (duodenum). An ulcer in the stomach is called a gastric ulcer, and an ulcer in the duodenum is called a duodenal ulcer. *H. pylori* are believed to be transmitted from person to person through close contact and exposure to fecal matter or vomit. The resulting infection weakens the protective mucus in the stomach and duodenum, allowing acid to get through to the sensitive lining beneath. Both the acid and bacteria irritate the lining and cause an ulcer to form.

If left untreated, a *H. pylori* infection can cause gastritis (*i.e.*, inflammation of the lining of the stomach) or

duodenitis (*i.e.*, inflammation of the duodenum). Gastritis can occur suddenly (acute gastritis) or gradually (chronic gastritis). An untreated *H. pylori* infection may also progress into peptic ulcer disease or stomach cancer later in life. Erosive gastritis is gastric mucosal erosion caused by damage to mucosal defenses. Chronic gastritis refers to a wide range of problems of the gastric tissues. In the case of gastritis induced by *H. pylori* infection and/or colonization, acute infection may appear as an acute gastritis with abdominal pain (stomach ache) or nausea. Where this develops into chronic gastritis, the symptoms, if present, are often those of non-ulcer dyspepsia: stomach pains, nausea, bloating, belching, and sometimes vomiting or black stool. Individuals infected with *H. pylori* have a 10 to 20% lifetime risk of developing peptic ulcers and a 1 to 2% risk of acquiring gastric (stomach) cancer, including, *inter alia,* gastric adenocarcinoma and MALT lymphoma (official name: extranodal marginal zone B cell lymphoma of mucosa-associated lymphoid tissue).

Gastric adenocarcinoma is divided into intestinal subtype and diffuse subtype. Intestinal type adenocarcinoma is more common. The sequence of pathological changes leading to intestinal type cancer starts with gastritis, followed by gastric atrophy (loss of glandular structure) and progressing to intestinal metaplasia, dysplasia, and finally carcinoma. This progression occurs over many years; consequently, most patients are middle aged or older. Environmental factors, such as smoking and diet, increase the risk of developing gastric adenocarcinoma. *H. pylori*-associated adenocarcinoma mostly occurs in the distal (non-cardia) stomach. In addition, *H. pylori* infection has been identified as a potential cause, or at least to contributing to the development of diffuse type adenocarcinoma, which is characterized by scattered tumor cells and lack of glandular organization (Testerman et al., World J Gastroenterol (2014); 20(36): 12781-12808).

*H. pylori* has further been reported to be responsible for 92%-98% of gastric MALT lymphoma (Testerman et al., World J Gastroenterol (2014); 20(36): 12781-12808). Most gastric MALT lymphomas are low grade when discovered and are dependent upon continued antigen stimulation. It is thought that the immunological response caused by the bacterial infection may lead to chronic gastritis with formation of lymphoid follicles within the stomach. These lymphoid follicles are composed of reactive T cells and activated plasma cells and B cells. The B cells are responsible for initiating a clonal expansion of centrocyte-like cells that form the basic histology of MALT lymphoma. Clinical symptoms are vague and varied, with abdominal pain being a common presenting complaint. *H. pylori* infection has further been implicated with other lymphoma types, including diffuse large B cell lymphoma (DLBCL).

In addition, *H. pylori* infection has been causatively linked to enterocolitis, an inflammation of the digestive tract, involving enteritis of the small intestine and colitis of the colon. Common clinical manifestations of enterocolitis are frequent diarrheal defecations, with or without nausea, vomiting, abdominal pain, fever, chills, and alteration of general condition. General manifestations are given by the dissemination of the infectious agent or its toxins throughout the body, or - most frequently - by significant losses of water and minerals, the consequence of diarrhea and vomiting.

For example, for its use in treating and/or preventing of *H. pylori* infection or associated condition, the compound of the invention may be administered in combination, or sequentially, with the constituents of a triple or quadruple therapy (according to the currently recommended treatment standard), wherein, preferably, the dosage(s) of one or more of the constituents of said triple or quadruple therapies, e.g., one or both of the two applied antibiotics, are lower (e.g., at least about 5%, 10%, 15%, 20%, 30%, 40%, 50%, 60%, 70%, 80%, or 90% lower) as compared to their dosage currently applied in the standard triple or quadruple therapies (see O'Connor A et al., Helicobacter. 2019;24 Suppl 1:e12640. Review: "Treatment of Helicobacter pylori Infection 2019"). It is expected that corresponding treatment regimens, wherein lowered dosages of one or more antibiotics are applied, can reduce antibiotic stress responses of the bacteria (including the (defence) mechanisms which drive development of persistence), while at the same time benefiting from the anti-virulence (i.e., antimotility) effects of the compound of the invention, and thus be particularly effective in the eradication of *H. pylori,* while reducing the risk of side effects.

The invention is herein described, by way of example only, with reference to the accompanying drawings for purposes of illustrative discussion of the preferred embodiments of the present invention.

Unless otherwise defined, all technical and scientific terms used herein have the same meaning as commonly understood by one of ordinary skill in the art to which this invention belongs. In case of conflict, the patent specification including definitions, will prevail.

Regarding the embodiments characterized in this specification, in particular in the claims, it is intended that each embodiment mentioned in a dependent claim is combined with each embodiment of each claim (independent or dependent) said dependent claim depends from. For example, in case of an independent claim 1 reciting 3 alternatives A, B and C, a dependent claim 2 reciting 3 alternatives D, E and F and a claim 3 depending from claims 1 and 2 and reciting 3 alternatives G, H and I, it is to be understood that the specification unambiguously discloses embodiments corresponding to combinations A, D, G; A, D, H; A, D, I; A, E, G; A, E, H; A, E, I; A, F, G; A, F, H; A, F, I; B, D, G; B, D, H; B, D, I; B, E, G; B, E, H; B, E, I; B, F, G; B, F, H; B, F, I; C, D, G; C, D, H; C, D, I; C, E, G; C, E, H; C, E, I; C, F, G; C, F, H; C, F, I, unless specifically mentioned otherwise.

Similarly, and also in those cases where independent and/or dependent claims do not recite alternatives, it is understood that if dependent claims refer back to a plurality of preceding claims, any combination of subject-matter covered thereby is considered to be explicitly disclosed. For example, in case of an independent claim 1, a dependent

claim 2 referring back to claim 1, and a dependent claim 3 referring back to both claims 2 and 1, it follows that the combination of the subject-matter of claims 3 and 1 is clearly and unambiguously disclosed as is the combination of the subject-matter of claims 3, 2 and 1. In case a further dependent claim 4 is present which refers to any one of claims 1 to 3, it follows that the combination of the subject-matter of claims 4 and 1, of claims 4, 2 and 1, of claims 4, 3 and 1, as well as of claims 4, 3, 2 and 1 is clearly and unambiguously disclosed.

The Figures show

**Fig. 1: Developing and establishing a screening system by primary luciferase-based reporter screen for flagellar expression and biosynthesis of *H. pylori* in multi-well plates and identification of active small compounds by the screen.** A) Results of luciferase flagellar reporter screen for one exemplary library of more than 1200 compounds; each dot represents one compound result. The x-axis shows the number of tested compounds, the y-axis shows reporter activity in percent of a positive non-inhibited control which was set to 100%. Inhibitory as well as enhancing activities of the compounds were observed. Yellow markings depict the borders of the Z-factor, corresponding to the three-fold standard error of the positive (non-inhibited) controls. Dots outside of the Z-factor margins are considered inhibitory or enhancing effects (active or hit compounds). B) Identification of active inhibitory compounds in five small compound libraries using the primary flagellar luciferase reporter screen. An upper cut-off of 30% inhibition was used to identify and enumerate the strongly inhibitory actives in the primary screen. Active compounds are given in percent of total screened compounds in each library.

**Fig. 2: antibacterial characteristics on *H. pylori* of a primary screen hit compound which had direct specific antibacterial effects against *H. pylori.*** This compound was an active/hit result, Active 1, which inhibited both, growth (determined by MIC/MBC) and flagellar reporter activity. In B) $IC_{50}$ determination of the reporter activity for this compound (primary screen), C) $IC_{50}$ determination for the metabolic activity (counter-screen) and D) MIC/MBC values for the same compound, which exhibits antibacterial activity against *H. pylori,* are given.

**Fig. 3: Chemical structure and antiflagellar activity of primary active compound Active 2 (BL2), antimotilin compound, against *H. pylori.*** Active 2 was a strong inhibitor in the primary anti-flagellar reporter screen, but did not inhibit growth or metabolic activity of *H. pylori* (MIC/MBC> 256 μg/ml) see also Table 3). Panel A) depicts the chemical identity of the compound (*top panel*) and a close chemical analog Active 2a (BL2a, *bottom panel*) with similar characteristics (one halogen residue replaced); Panel B) shows the $IC_{50}$ values for the BL2 compound in the primary luciferase-based *H. pylori* reporter assay.

**Fig. 4: Anti-motility activity on live *H. pylori* bacteria of two active/hit compounds *in vitro.*** Tracking assays with *H. pylori* were performed in liquid medium (RPMI1640 with 3% horse serum) and movies recorded in a CELL-R live imaging system (Olympus, see methods). A) Stops/reversals during the swimming runs of the bacteria as a measure of intact taxis and directional motility and curvilinear velocity (CLV) were quantitated as described in (Behrens et al., 2016). Significance of differences between the positive control (Control) and the compound-treated bacterial samples for 15 bacteria each are indicated by asterisks. * < 0.05. n.s. = non-significant difference.

**Fig. 5: Proof-of-principle therapeutic application of primary active antimotilin compound (BL2) in a preclinical chronic *H. pylori* mouse infection model revealed significant activity *in vivo.*** Panel A) shows a scheme of the experimental design. In panel B), the cfu values of four groups of *H. pylori*-infected mice with or without administration of BL2 compound in combination with metronidazole or after sole administration of BL2, are shown (see Table 4). All mice in the positive infected control group were cfu-positive; three animals in this group had low counts. C) microbiota analysis of all groups in this experiment by 16S amplicon analysis. The microbiota composition was not significantly different between the groups (multiple comparisons, two-way AMOVA). For detailed AMOVA results, see Table 5. Two groups which were not infected but only given BL2 or only mock-dosed are not shown in B. The detailed experimental setup is described in the methods and Table 4.

**Fig. 6: Western blot reveals reduction of flagellin protein FlaA upon incubation with active antimotilin compound Active 2 (BL2).** Two different time points of an *H. pylori* growth curve in liquid BHI medium (3% yeast extract, 5% horse serum), T1 (early exponential) T2 (late exponential growth) were monitored for differences in protein expression in the presence (+) or absence of Active 2 compound. Western immunoblots were performed on insoluble (IS), soluble (S) and surface-associated (OF) fractions of the bacteria. Blots were developed with anti-FlaA, anti-FlhA and anti-CagL antisera as fractionation controls, as indicated. The FlaA blot reveals a significant reduction of

main flagellin FlaA at time point T2 upon supplementation with Active 2 compound in the soluble and surface-associated bacterial fractions. FlhA serves as a fractionation control for the insoluble fraction. CagL, which was slightly increased by Active 2 compound, is shown as a second control for the insoluble fraction.

**Fig. 7: PCR identified strong residual bacterial load in a therapeutic *H. pylori* chronic infection model for the antibiotic metronidazole but not for the active pathoblocker compound BL2.** A) A polymerase chain reaction for a partial nucleotide segment of the *H. pylori cagL* gene was performed on stomach corpus homogenates from the chronic mouse infection and treatment experiment with BL2. PCR results from mice grouped in four groups (only HP-infected; groups separated by blue lines) (Table 4) are shown in the order of appearance. Group 1: mice 1 to 10; group 2: mice 11 to 20; group 3: mice 21 to 30; group 4 mice 31 to 40. Mouse numbers are shown above the PCR lanes. Highquality sequences obtained from *cagL* positive PCRs in the absence of positive culture are boxed; respective Sanger sequence reads are shown in B). Nucleotide sequences from mice 29, 36 and 38 contain SNPs in comparison to the input strain (red box), while the sequences from the other reisolates and homogenate PCR bands do not contain SNPs. N = negative control without DNA; P = positive PCR control using genomic DNA of mouse adapted *H. pylori* strain; L = nucleotide size ladder - 1 kb plus ladder Thermo Scientific. C) No significant change of plasma cytokine loads (measured by BioRad Bioplex assay) was determined in chronic mouse infection model with or without therapeutic intervention with BL2. Groups of mice (six groups, 54 mice) are grouped as depicted in the scheme in Fig. 5, Table 4.

The examples illustrate the invention:

## Example 1: Materials and Methods

### Bacterial strains and cultivation

*H. pylori* was cultured on blood agar plates (10% horse blood in Columbia blood agar base, Oxoid), supplemented with an antibiotic and antifungal combination under microaerobic conditions as described previously (Behrens et al., 2016). For growth inhibition assays, *H. pylori* was cultivated in broth culture (BHI broth, supplemented with 3% yeast extract and 5% horse serum), not supplemented with any antibiotics or antifungal agents. The microaerobic atmosphere required for growth was provided in air-tight incubation chambers supplemented with a specific gas mixture (5% $O_2$, 10% $CO_2$, 85% $N_2$) or in anaerobic jars with oxygen-depleting gas generator bags (Anaerocult C, Merck). Other bacterial strains used in the assays were *Escherichia coli* RP437 and *Campylobacter jejuni* 11186. *E. coli* were routinely cultivated on LB agar plates and liquid cultures for Minimum Inhibitory Concentration (MIC) assays performed in LB broth medium. *C. jejuni* was grown on blood agar plates with 5% sheep blood, and liquid culture for antibiotic testing was performed in brain-heart infusion (BHI) broth medium, supplemented with 3% yeast extract.

### Development of a *H. pylori* luminescence reporter strain as a screening tool for compounds having anti-flagellar effects

The *H. pylori flaA* promoter was fused with a luciferase operon (*luxAB*) from *Vibrio harveyi* in a *H. pylori* suicide plasmid (Niehus, Ye, Suerbaum, & Josenhans, 2002). The *flaA* reporter fusion was recombined into the *H. pylori* chromosome and the resulting strain tested to be strongly luminescence positive. The reporter strain (N6 *flaA-lux4B*) activated luminescence to about 40,000 to 60,000 counts per second using luciferase substrate at an $OD_{600}$ of 0.8 (mid-log phase) in liquid culture. The negative control value of a non-reporter strain or reporter strain without luminescence substrate was close to zero. Hence, the signal-to-noise ratio of the assay is between $10^4$ and $10^5$. The Z-Factor (Zhang et al., 1999) determined for the assay with various bacterial inhibitors measured in quadruplicates or triplicates was routinely between 0.6 and 0.7, with a confidence interval of 95%.

The reporter strain was validated using the compounds carbonyl cyanide m-chlorophenyl hydrazine (CCCP) (inhibitor of membrane potential essential for flagellar motility), rotenone (inhibitor of complex I of the respiratory chain), and the antibiotic ampicillin (cell wall biosynthesis inhibitor). While the first two metabolic/respiratory chain inhibitors inhibited the luminescence reporter activity, the cell wall antibiotic, which is highly effective against *H. pylori,* did not alter the readings in the luciferase reporter assay. This clearly distinguishes the screening assay from a classical antibacterial screen.

### Luciferase / Bioluminescence assay used during the screening procedure

*H. pylori* luminescence reporter strains were passaged on blood agar plates less than 22 hours prior to the start of the experiment. The passage number for these assays was not higher than five to preserve the strain's properties

and genetic stability. Luciferase reaction buffer was prepared as follows: 50 mM disodium phosphate ($Na_2HPO_4$) and 2% BSA were dissolved in HPLC water, filter sterilized, aliquoted and frozen at - 20°C.

Bacteria were harvested in brain-heart infusion medium (BHI, 3% yeast extract with 5% horse serum added) and adjusted to an $OD_{600}$ of 0.8 at room temperature. 50 μl of the bacterial suspension were added per well of a 96 well plate (non-binding, Greiner Bio-One 655901).

2 μl of each of the test compounds (provided as a 10 mM DMSO solution) were added to the sample wells. The same amount of pure, diluted DMSO was added to the control wells for a concentration of a maximum of 4% DMSO per well, since higher concentrations will interfere with metabolic functions of *H. pylori* and cause false negative results. The microwell plate was incubated inside a sterile sealed PM Gas Bag (BIOLOG #3032) together with a CampyGen Compact pack (Oxoid) to generate microaerobic conditions at 37°C and with shaking at 175 rpm for 4 h.

Shortly before taking the measurement, the luciferase substrate was prepared by diluting the luminescence substrate decanal (10% stock, Sigma Aldrich) at a 1 :2,000 dilution in luciferase reaction buffer. 60 μl of the substrate were added per well of a white 96 microwell plate (Thermo Scientific #236105) without the lid.

For the final luciferase measurement, 10 μl of bacterial solution were transferred with the epMotion 96 (Eppendorf) simultaneously to the wells containing the luciferase substrate and incubated on a microwell plate shaker for 10 s to lyse the bacteria. The measurements were recorded with a Perkin Elmer Victor 3/WNallac 1420 plate reader (luminometry, measurement time: 0.5 sec) and integrated to counts per sec. For the background measurement, 10 μl sterile medium containing 4% DMSO was added to the wells. For each measurement, at least four luciferase reporter positive control wells (without inhibitory compound) and four negative control wells (background without bacteria) were run on the same day and under the same assay conditions in parallel.

Calculation of the Z factor of the screening assay

The Z factor was calculated as described by Zhang *et al.* [Zhang et al., 1999]

$$Z = 1 - \frac{3\sigma_{\text{positive}} + 3\sigma_{\text{negative}}}{|\mu_{\text{positive}} - \mu_{\text{negative}}|}$$

$\sigma$ - standard deviation, $\mu$ - mean, positive - positive controls, negative - negative controls

**A Simple Statistical Parameter for Use in Evaluation and Validation of High Throughput Screening Assays** Ji-Hu Zhang, Thomas D. Y. Chung and Kevin R. Oldenburg J Biomol Screen 1999 4: 67

$$Z = 1 - \frac{3SD\text{ of sample} + 3SD\text{ of control}}{|\text{mean of sample} - \text{mean of control}|}*$$

| *Z-factor value* | *Structure of assay* | *Related to screening* |
|---|---|---|
| 1 | SD = 0 (no variation), or the dynamic range → ∞ | An ideal assay |
| 1 > Z ≥ 0.5 | Separation band is large | An excellent assay |
| 0.5 > Z > 0 | Separation band is small | A double assay |
| 0 | No separation band, the sample signal variation and control signal variation bands touch | A "yes/no" type assay |
| <0 | No separation band, the sample signal variation and control signal variation bands overlap | Screening essentially impossible |

*Note: For agonist/activation type assays the control data in the equation are substituted with the positive control (maximum activated signal) data; for antagonist/inhibition type assays the control data in the equation are substituted with the negative control (minimum signal) data, (see also Eq. 2 and Fig. 4

**Minimum inhibitory concentration (MIC), minimum bactericidal concentration (MBC) of the test compounds**

In order to distinguish compounds having anti-flagellar activity from compounds having classical antibiotic

effects (*i.e.*, bacteriostatic and/or bactericidal effects), the test compounds were additionally subjected to MIC/MBC testing. *Helicobacter pylori* were grown in liquid culture by harvesting bacteria from overnight-grown plates into brain-heart infusion medium (BHI, Oxoid) with 3% yeast extract and 5% horse serum, added to an initial inoculum of $OD_{600}$ = 0.05. 10 ml of this bacterial suspension were incubated as a preculture in a 50 ml Falcon tube with lid ajar inside of an air-tight incubation jar and a microaerobic atmosphere generated by Merck Anaerocult C gas generating packs at 37°C and shaking with 175 rpm overnight. On the following day, prior to the beginning of the experiment, the $OD_{600}$ of the liquid culture was routinely below 1, in mid-exponential growth phase.

Next, 200 μl of the bacterial suspension were added per well of a 24-well-plate (Greiner Cellstar #662160) diluted to an initial $OD_{600}$ of 0.07. Compounds (in DMSO) were added to the sample wells in duplicates in a serial dilution of starting at an upper limit of 256 μg/ml. Pure DMSO was added to all wells for a concentration of 2% DMSO per well. The multiwell plate (with lid) was incubated inside of an incubation jar and a microaerobic atmosphere generated by Merck Anaerocult C sachets at 37°C and shaking with 175 rpm for 24 h. For measurement, the duplicates were pooled after visual inspection, diluted 1:3 in medium and measured in plastic cuvettes as an OD at 600 nm. The detection limit of growth was defined as an $OD_{600}$ of 0.05. Equal or lower values were considered as indicators of completely inhibited growth and counted as the Minimum Inhibitory Concentration (MIC). This MIC determination method can also be downscaled, further automated and measured in 96- or 384-well plates in a micro-well plate incubator equipped with a gas chamber for microaerobic bacterial incubation.

MICs of known antibiotics (e.g. metronidazole) were also confirmed using Etest strips (Liofilchem) on blood agar plates.

For evaluation of the "minimum bactericidal concentration" (MBC), bacteria from the MIC experiment were streaked and plated at different serial dilutions on blood agar, including the lowest compound concentration where bacterial growth was still measurable by $OD_{600}$ ($OD_{600} \geq 0.05$), and three compound concentration levels above (if possible). The blood agar plates were incubated at 37°C in an anaerobic atmosphere, either inside an incubation jar (Oxoid) with an Anaerocult C gas pack (Merck), or in an anaerobic cabinet aerated with a microaerobic gas mix as outlined above (Scholzen).

For evaluation of the MBC, bacteria from the MIC experiment were plated as streaks or in appropriate dilutions on Columbia blood agar plates, using inoculation loops. The plates were incubated at 37°C overnight to detect growth.

**Microscopical motility tracking of *H. pylori* in the presence of test compounds**

For evaluating effects of a test compound on *H. pylori* motility, bacterial cells were tracked microscopically in the presence of the test compound. In particular, HEPES-buffered RPMI 1640 (Gibco) medium, supplemented with 3% fetal bovine serum (Biochrom) was pre-warmed to 37°C. *Helicobacter pylori* (strain N6) was harvested in medium to a test $OD_{600}$ of 0.033, distributed to cell culture flasks with filter lids (2 ml test volume each), and kept in a 37°C humidified incubation chamber at ambient atmosphere containing additionally 5% carbon dioxide. The pre-incubation for equilibration of the bacterial culture was carried out for 15 min prior to the beginning of the experiment. The microscope chamber (Olympus CELL-R system) was set to an atmosphere of 37°C, 5% carbon dioxide and 50% humidity. Compounds were added to the flasks, followed by gentle mixing. Motility of the bacteria was observed after 0, 15 and 120 min of incubation. Movies were recorded with *cell$^R$* software (Olympus) after 15 min of incubation with the compounds. Six bacterial cells visible for approximately 100 frames per movie were tracked using the *cell$^R$* system and software (Behrens et al., 2016; Schweinitzer et al., 2008). Velocity, number of stops/reversals and track lengths for each bacterial cell were observed and enumerated, with each back and forth motion (reversal) of a cell being considered as two stops.

**Active compound pre-testing for mouse toxicity (preparation of preclinical model)**

The active compounds BL1 and BL2, which were chosen to be tested in a preclinical model, were initially tested for toxicity in the mouse model by minimal toxic dose (MTD) testing. The MTD testing (MTD Tox56000 protocol, Eurofins Panlabs) was performed according to international standards, as will be briefly outlined in the following: Three mice were dosed with the compounds at each intended dose. BL1 active compound was prepared in a suitable stock concentration in 5% DMSO, or 5% DMSO, 10% Solutol, 85% PBS (for the 40 and 60 mg/kg/day), and dosed at 10, 30, 40, or 60 mg/kg/day by intraperitoneal injection (125 μl for each dose). BL2 active compound was dosed orally, at 10 mg/kg/day, 30 mg/kg/day, or 60 mg/kg/day, diluted in 5% DMSO, 2.5% carboxy-methyl-cellulose (CMC), in PBS, also at 125 μl per dose per animal. The dosing in each case started with the lowest dose in the three animals, followed by observation for up to 72 h. Subsequently, the next higher dose was administered to a different set of three mice, etc.. Both compounds to be used subsequently for the *in vivo* therapeutic administration were verified to be non-toxic to mice below an administered dose of 40 mg/kg per day. Compound BL1 showed variable toxicity at 60 mg/kg/day, which was mainly attributed to poor solubility of the compound, but showed no toxicity at 40 mg/kg/day. BL2 was very well soluble and did not show any toxicity up to 60 mg/kg/day.

### *H. pylori* infection and therapeutic compound administration in the mouse as a preclinical model for the anti-motility therapeutic principle

6-8 weeks old specific pathogen-free C57B/L6 mice were obtained from Charles River Laboratories. They were acclimatized to the new surroundings for one week before starting the assays. For the whole duration of the experiment, mice had access to sterile chow and sterile water ad libitum. In week one of the experiment, *H. pylori* was administered at $3\times10^8$ bacteria per mouse per inoculum (100 μl) on two days with one day of break in between. The proof-of-principle treatment was started two weeks after the end of the inoculation week, assuming a stable infection at this time point. The mice were treated in separate groups (8-14 animals per group), as outlined in the results, with compound(s), antibiotic (metronidazole at 14.3 mg/kg/day), a combination of both antibiotic and compound (for concentrations, see Results), or mock-treated once daily over seven consecutive days by intra-gastric gavage. After the treatment week, the mice were kept for another two weeks under normal housing and feeding conditions with sterilized chow and sterile water ad libitum. At the end of the assay period (week seven), the mice were necropsied by cervical dislocation under $CO_2$ anesthesia. The animal experiments were authorized under German federal law by the LAVES (Lower Saxony Government Authority). The mouse infection with *H. pylori* was persistent for more than six weeks (the total duration of the experiment) but did not cause any discernible pathological features in the mouse stomach in the here used model as assessed by experienced mouse pathologists.

### Microbiota amplicon sequencing and analysis from mouse feces as a measure of well-tolerated novel therapeutic active principle and compound to protect the microbiota

Lower bowel microbiota analysis was performed from fecal pellets collected from each animal shortly before the end of the treatment experiment. 16S rRNA amplicon sequencing was performed for the identification of microbiota composition. The preparation of total DNA, 16S amplicon library preparations, microbiota sequencing (Illumina MiSeq Sequencer) and final analysis were done using Illumina NexteraXT chemistry and the bacterial 16S rDNA v3-v4 region-specific primers for Amplicon generation as previously described (Yang et al., 2016). Paired sequencing reads from a MiSeq instrument (Illumina) were collected, analyzed and searched for various bacterial taxonomic groups using a standardized pipeline against several appropriate bacterial 16S databases.

### Cytokine analysis from mouse blood by Biorad Bioplex multiplex bead assay as a measure of well-tolerated compound as a novel treatment option

Before the infection and at the end of the treatment experiment, mouse blood was taken from the eye vein of a few selected mice (approximately 30 to 100 μl per mouse). The blood plasma was subsequently separated from cells using separation centrifugation devices (Sarstedt Microvette lithium-heparin). Plasma was diluted in Biorad Bioplex assay buffer to one fourth of the initial concentration and measured in the 23-Plex BioPlex bead-based multiplex cytokine assay (Biorad #m60009rdpd) according to the manufacturer's instructions and using the provided standards. Each sample was measured in a total volume of 50 μl in duplicate.

## Example 2: Screening and Identification of *H. pylori* anti-flagellar compounds

### Development of anti-flagellar screen and establishing and improving high-throughput library screening identifies numerous compounds active on a *H. pylori* flagellar assembly reporter strain

A *H. pylori* flagellar biogenesis reporter strain was developed based on a luciferase fusion to the flagellar *flaA* promoter of *H. pylori* as described in Example 1. Due to the position of the *flaA* gene as a so-called "late gene" (or "late-stage" gene) in the hierarchical cascade of flagellar gene regulation, this reporter construct is capable of detecting effects on multiple different pathways affecting flagellar gene regulation, assembly and substrate secretion (Josenhans et al., 2002; Niehus et al., 2004) that converge at the inhibition of flagellar motility, which is essential for the organism *in vivo* (Lertsethtakarn et al., 2011). The reporter construct was verified to possess a high sensitivity, high signal-to-noise ratio (low background) of 10,000 to 60,000 (determined in relative luminescence units (RLUs)) with a background value of 5 to 10, and a Z-factor of about 0.6 to 0.7. The positive control bacteria performed in 8 replicates per plate were always not inhibited (R2>95%), which makes the system readily amenable to medium- to high-throughput screening (HTS) (Methods).

For establishing the screen on the *H. pylori* luciferase reporter in High Throughput Screening format (HTS, 96-well plate format), approximately 4,000 compounds contained in five small-compound libraries were tested for effects targeting flagellar biosynthesis in *H. pylori.*

Screening the libraries for inhibitory effects on the *H. pylori* flagellar reporter strain, including at least two

biological screening repetitions (biological replicates), verified that the screening principle was able to single out inhibitory compounds reliably and efficiently. The screening identified numerous known and uncharacterized compounds that had a significant inhibitory effect (decreased bioluminescence) on the reporter strain (Fig. 1A). While many compounds showed inhibitory activity, some compounds increased the activity of the reporter construct (increased bioluminescence) (Fig. 1A). An observed inhibitory effect (decreased bioluminescence as compared to control) in the luciferase assay (primary screen) indicates a test compound to have an anti-flagellar effect and/or a bactericidal effect.

A counter-screen for growth inhibition was performed, also in multi-well plates, which demonstrated that the primary screen also detected a number of compounds which had both growth-inhibitory as well as anti-flagellar effects. The counter-screen also identified compounds which showed exclusively inhibitory effects in the anti-flagellar screen. Some compounds (including known antibiotics) did not show any effects in the anti-flagellar screen, which verified that the screen is specific for certain cellular effects and does not generally detect antibacterials. Overall, about 6% of all screened compounds showed activity (inhibition >30%) in the primary flagellar luminescence reporter screen (Fig. 1B). Approximately 1% of total actives were identified that had inhibitory effects only in the reporter luciferase activity, but did not show a direct antibacterial (bacteriostatic and/or bactericidal) effect.

**Numerous novel active compounds have combined anti-flagellar and anti-vital effects on *H. pylori in vitro***

Next, the $IC_{50}$ values for luminescence and viability inhibition were determined for a panel of selected compounds that were found to have an inhibitory activity in the primary screen. A wide range of $IC_{50}$ values was identified for the different compounds (Table 1, Fig. 2, Fig. 3). Many active compounds from the luciferase screens (on average, 80% of all actives) also had an antibacterial/antiviability effect (own unpublished data, and DZIF/Haas, personal communication). Therefore, MIC/MBC values were determined for a selected panel of strongly inhibitory compounds mostly from library gamma, collected in the screening approaches and also containing known therapeutically active compound classes, including known antibiotics. This methodology confirmed that most preselected active inhibitory compounds, which were further tested in detail, exerted a bactericidal or bacteriostatic effect on *H. pylori* at various concentrations (Table 1). Several compounds were determined to exhibit low MIC/MBC values, which highlighted them for further antibacterial studies. However, importantly, some other compounds from various libraries did not exhibit antibacterial activity, which singled those out for further studies on their anti-flagellar effects (Table 3).

**Novel anti-flagellar active compounds exert H. pylori-specific effects**

Instead of developing a broad-spectrum antibacterial compound, the goal of this study was to identify compounds which specifically target *H. pylori.* First, the compound effects were verified to not be strain-specific for one single strain of *H. pylori* by testing a second *H. pylori* strain (P12). The quantitative compound effects (MIC/MBC) were similar for both strains (Table 1).

Then, a panel of 12 preselected active compounds from library beta was tested according to the criterion that they showed >= 90% reduction of luminescence activity, for their antibacterial activity against other bacteria, including *C. jejuni* and *E. coli* using an assay for respiratory inhibition and MIC/MBC determination. Very weak antibacterial effects were observed with seven of the selected compounds on *C. jejuni,* and with only one compound on *E. coli* (Table 2). It can therefore be concluded that the selected compounds identified in the *H. pylori* anti-flagellar screen act rather selectively on *H. pylori* and not on other bacterial species.

**Identification of anti-flagellar compounds against *H. pylori* which do not have anti-vital (classical antibiotic) effects and selection/characterization of active patho-blocker compounds for further investigations**

Some compounds identified in the primary screen had no antibacterial effect as assessed by either the metabolic test (respiratory inhibition assay), ATP (BacTiter Glo) content assay, or MIC assay. A small number of compounds only modulated luminescence activity in the flagellar-targeted assay and did not show any inhibitory effect in any other secondary antibacterial assay. Selected compounds out of these actives were further tested in microscopic motility assays on *H. pylori* (as described in Example 1). Two compounds directly modified motility at low concentrations, both by reducing motility speed after a short time of incubation (between 5 and 15 min), and by changing the motility patterns (number of stops/reversals), (Fig. 3), which indicated that these compounds are directed selectively against motility-associated functions and might represent active patho-blockers. Subsequently, one promising compound (Active 2/BL2; Table 3), which did not exhibit classical antibacterial effects (MIC > 256 $\mu$g/ml) and had a low $IC_{50}$ of 2.75 $\mu$g/ml (Fig. 2) in the luciferase assay, was selected for further investigations. This compound also reduced the amounts of the major flagellin, FlaA (major protein subunit of motility organelles/flagella) as shown in Fig. 6.

Example 3: ***In vivo* preclinical evaluation of antimotilin compound BL2**

**The anti-flagellar and not anti-metabolic patho-blocker BL2 leads to substantial reduction of colonization when therapeutically administered in a *H. pylori* preclinical mouse model and confirms the activity of the novel therapeutic principle**

Compound BL2 was selected to perform a proof-of-principle preclinical treatment model against an established *H. pylori* infection, as an *in vivo* experiment in mice. As reported in Example 2, this compound was found to be not toxic to mice at doses below 60 mg/kg/day, given orally, which was a prerequisite for use in a preclinical model. For the whole duration of the experiment, mice had access to sterile chow and sterile water ad libitum.

Then, BL2 was further tested for therapeutic efficacy in a mouse model of chronic *H. pylori* infection (Fig. 5). Six groups of mice (10 mice per group in each of the four infected and/or treated groups, and six mice in both of the control groups) were established in this mouse treatment trial. For the treatment groups, mice were infected in week one (using ca. $3\times10^8$ bacteria per animal; Methods), and the chronic infection was manifested for two more weeks. The once-daily treatment (intragastric gavage) started in week four on seven consecutive days. For the treatment groups, three *H. pylori*-infected groups were evaluated in parallel. One dose of treatment was given daily.

Group one was treated with metronidazole (14.3 mg/kg/day) only, an established anti-*H. pylori* antibiotic. The antibiotic concentration corresponds to concentrations routinely used in human treatment. Group two was treated with metronidazole (dosed at 14.3 mg/kg/day) and BL2 (10 mg/kg/day) in combination, and group three received BL2 (10 mg/kg/day) only. In addition, one infected and mock-treated group was kept as positive control, and two mock-infected groups were set aside, one for administering BL2 only (10 mg/kg/day; microbiota compound control) and one which was mock-infected and mock-treated (microbiota null control).

The read-out of successful treatment was the reduction of colony-forming units (cfus) of bacteria in infected and treated mice, over infected, non-treated, mice, at the end of the experiment. A significant therapeutic activity was determined for compound BL2 against *H. pylori,* with a statistically significant reduction of cfu counts in the antrum for the infected BL2-treated group in comparison to the infected control (Fig. 5). The corpus also showed a trend towards reduced cfus in the infected and treated animals, but this trend was not statistically significant. As expected, the metronidazole-only group and the combined metronidazole and BL2 treatment group also had significantly reduced bacterial counts in comparison to the infected control (Fig. 5A,B). All mice in the infected positive control group were colonized (both cfu counts and PCR-positive), although the cfu counts in three positive animals were rather low.

When testing the presence of residual bacteria in stomach homogenates of cfu-negative mice using gene-specific PCR (as described in Methods; see Example 1), it was found that upon metronidazole treatment, or upon combination treatment of antibiotic plus BL2 compound, all mice still had detectable specific bacterial (PCR) signal in their stomachs (two weeks post-treatment; Fig. 7A). Sangersequencing of the specific PCR signal after DNA purification revealed correct *H. pylori* gene sequences (*cagL*; Fig. 7B) for all these PCR-amplified bands. Surprisingly, the infected mouse group that had been given BL2 only, had a strong reduction of gene-specific PCR signal at two weeks post-treatment in all but two mice (Fig. 7A).

Histopathology evaluation of all infected mouse groups, with or without treatment, did not show any elevated stomach pathology in any of the animals, as has been observed before with all *H. pylori* mouse models, due to unknown biological causes which generally limit pathology by *H. pylori* in the mouse.

Pathology can also be variable in humans and therefore is not a suitable read-out. Hence, since the hallmark of the here used model is reduction of bacterial cfus and not pathology, this does not change the interpretation of positive treatment outcome.

**Novel therapeutic principle is well-tolerated in preclinical model: Fecal microbiota composition and plasma cytokines are not significantly altered by a therapeutically effective anti-flagellar compound in the *H. pylori* infection and treatment mouse model**

From the experimental mouse treatment study, fecal pellets from all groups of mice were harvested at the end of the experiment (before necropsy) in order to assess the influence of *H. pylori* infection alone or of therapeutic compound administration on the fecal microbiota composition of all animals.

Microbiota was analyzed by fecal microbiota 16S rRNA amplicon sequencing of the V3-V4 variable regions.

Active compound BL2 alone did not exert a marked influence on fecal microbiota composition or richness (Fig. 5C) (Table 5). Also, the *H. pylori* infection alone, or a combination of infection and compound administration, did not lead to a marked difference in any of the fecal microbiota samples in comparison to the negative control groups in the treatment assay (Fig. 5C) (Table 5). Testing mouse plasma from the treatment assay for systemic cytokine production in blood by multiplex bead test (Methods) did not reveal any significant changes in cytokine amounts in blood between the control groups, the infected groups and the compound- and/or antibiotic-treated groups (Fig. 7).

Here, the present inventors developed a powerful luciferase-based screening system for diverse antiflagellar and antimotility effects against *H. pylori,* and established it to be applied in 96- or 384-well plates. Using this screening assay, numerous active compounds were identified that had strong antibacterial and/or anti-flagellar effects on *H. pylori.* Several primary actives had low (very effective) antibacterial MIC values and acted on *H. pylori* at different levels, probably via metabolic inhibition. In addition, some promising compounds were identified that did not inhibit *H. pylori* viability at all, but exclusively inhibited in the flagellar screen and assays of motility inhibition.

The therapeutic application of the selective anti-motility compound BL2 (together with its analog BL2a, both termed as antimotilin), identified by the selective screening approach, was not active against *H. pylori* in a classical antibacterial (antibiotic) manner. In a preclinical *H. pylori* mouse model of chronic infection, BL2 was significantly effective in reducing bacterial loads, both in the gastric corpus and the antrum. It also reduced molecular bacterial detection in the stomach by PCR at 2 weeks post treatment below detection levels, in contrast to single antibiotic therapy using metronidazole, which left no bacterial counts at the same time point, but where bacteria were still readily detectable by PCR. This is a promising result for the novel principle, which encourages further investments into the therapeutic concept of antimotilin patho-blockers against *H. pylori.*

The here reported results using the new anti-motility (antimotilin) and anti-virulence principle against *H. pylori* in the animal model provides a promising starting point for a new kind of combination or even single therapy against *H. pylori.* These results also indicate that antibiotic activity of compounds against *H. pylori* impose a strong selective pressure and longer bacterial survival times in the stomach, which may, over the time of persistence, lead to increasing bacterial resistance development and higher probability of relapse. In contrast, the anti-motility compound provides a more rapid clearance of the bacteria from the stomach, which was clearly visible by PCR at 2 weeks post-treatment. In addition, the therapeutic dose of the patho-blocker compound did not alter the microbiota composition or richness of resident intestinal microbiota after one week of daily therapeutic intervention, which may indicate that the novel therapeutic principle is well-tolerated with less prominent side effects on the gastrointestinal microbiota.

In conclusion, the present inventors developed a novel type of antimotility screen and identified promising patho-blocker compounds (antimotilins) which seem to be specifically active on a chronic *H. pylori* infection. It seems worthwhile to apply the antimotility screening assay to larger compound collections, and to further validate the novel therapeutic approach of motility inhibition of the chronic *H. pylori* infection as a specific anti-virulence therapy.

## Example 4: Additional active compound variants

Starting from Active 2 (BL2), identified by the herein disclosed screening method as initial lead compound, the present inventors designed and synthesized the following further compound variants/derivatives. The chemical synthesis, purification and structural characterization (by 1D NMR spectroscopy) of the compounds is described in Example 5, below.

The antiflagellar/antimotility activity of these further compounds was confirmed by applying the compounds in the herein disclosed luciferase-based *H. pylori* reporter assay (using the *H. pylori flaA*-luciferase reporter strain N6). The compounds and their respective determined $IC_{50}$ values are presented in Table 6, below.

As is evident from the obtained $IC_{50}$ data (Table 6), essentially all tested compounds variants/derivates (with exception of compounds DJ-3 and DJ-4) have inhibitory activity and with comparable $IC_{50}$ values as determined for the initial lead compound Active 2 (BL2). Among the variants tested, compounds DJ-1, -2, -5, -6, -7, -8, -9 and -12 have high inhibitory potency with all having $IC_{50}$ values <4.5 $\mu$g/ml; and with compound DJ-2 having the lowest determined $IC_{50}$ of 0.44 $\mu$g/ml indicative of a particularly high inhibitory activity. Compounds DJ-10 and DJ-11 were slightly less active with determined $IC_{50}$ values of 10.83 $\mu$g/ml and 10.56 $\mu$g/ml, respectively, and compounds DJ-13 and DJ-14 still having detectable yet weaker inhibitory activity. No inhibitory activity was detectable for compounds DJ-3 and DJ-4, likely to due significant differences in their chemical structures.

**Table 6:**

| **Compound ID§** | ***alias*** | **$IC_{50}$*** |
|---|---|---|
| 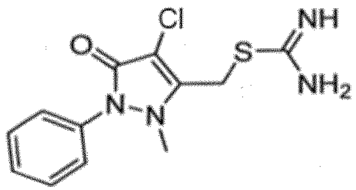 | Active 2 (BL2) | **$IC_{50}$ = 2.75 μg/ml** (*data shown in Figure 3B*) |
| 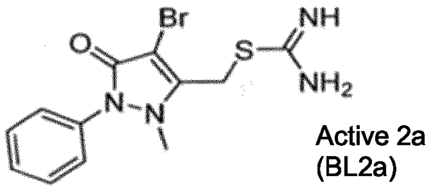 **Active 2a (BL2a)** | DJ-1 | $IC_{50}$ = 3.15 μg/ml 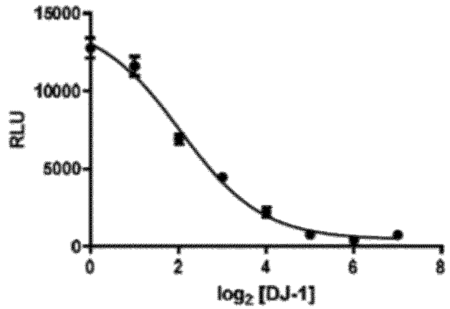 |
| 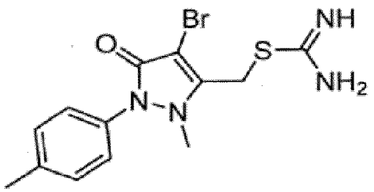 **ssa266-1** Chemical Formula: $C_{13}H_{15}BrN_4OS$ Exact Mass: 354,01 Molecular Weight: 355.25 | DJ-5 | $IC_{50}$ = 2.85 μg/ml 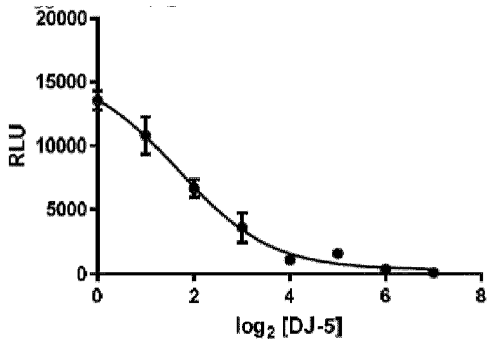 |

(continued)

| Compound ID§ | *alias* | $IC_{50}$* |
|---|---|---|
| 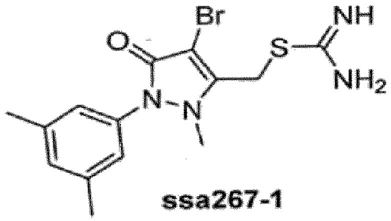 ssa267-1 Chemical Formula: $C_{14}H_{17}BrN_4OS$ Exact Mass: 368,03 Molecular Weight: 369,28 | DJ-6 | $IC_{50}$ = 2.29 μg/ml 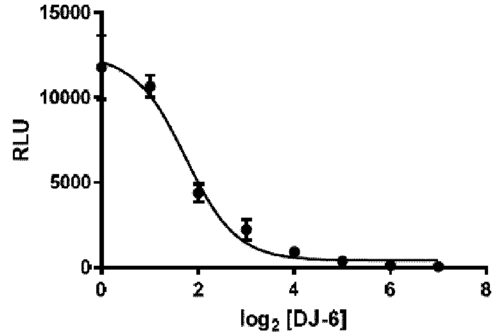  |
| 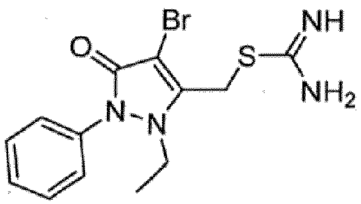 P29 Chemical Formula: $C_{13}H_{15}BrN_4OS$ Exact Mass: 354,01 Molecular Weight: 355.25 | DJ-11 | $IC_{50}$ = 10.56 μg/ml 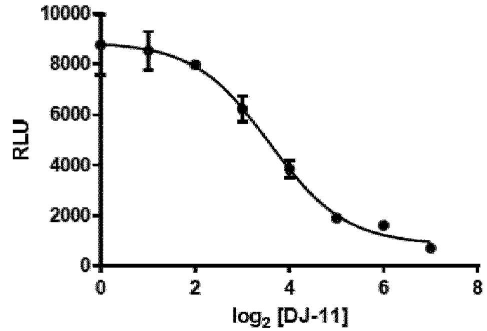  |
| 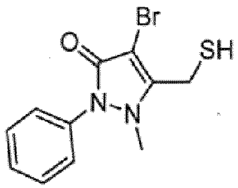 Chemical Formula: $C_{11}H_{11}BrN_2OS$ Exact Mass: 297.9775 Molecular Weight: 299.1860 | DJ-2 | $IC_{50}$ = 0.44 μg/ml 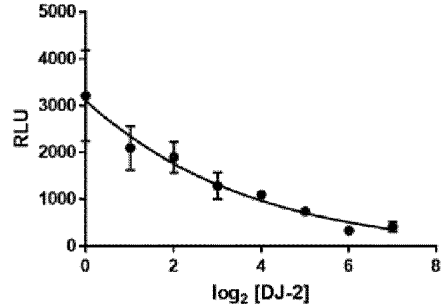  |

(continued)

| Compound ID§ | *alias* | $IC_{50}$* |
|---|---|---|
| 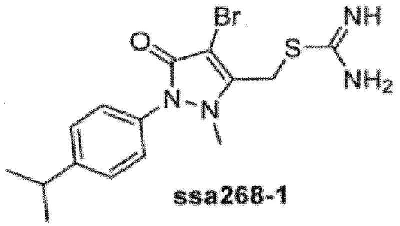 **ssa268-1** Chemical Formula: $C_{15}H_{19}BrN_4OS$ Exact Mass; 382,05 Molecular Weight: 383,31 | DJ-7 | $IC_{50}$ = 4.38 μg/ml 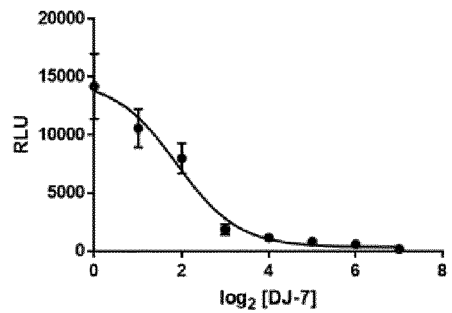  |
| 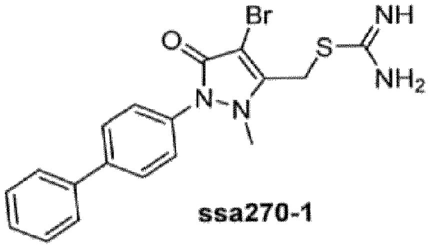 **ssa270-1** Chemical Formula: $C_{18}H_{17}BrN_4OS$ Exact Mass: 416,03 Molecular Weight: 417,33 | DJ-8 | $IC_{50}$ = 2.48 μg/ml 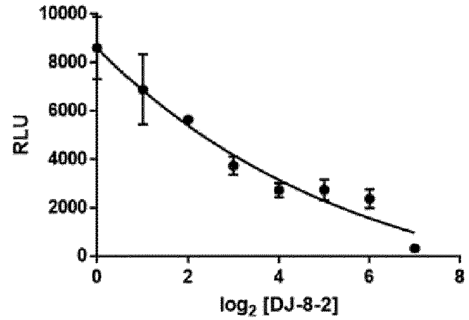  |
| 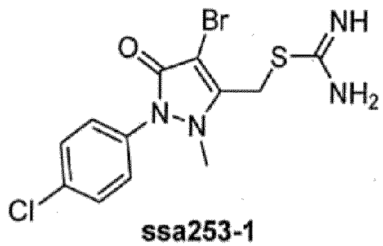 **ssa253-1** Chemical Formula: $C_{12}H_{12}BrClN_4OS$ Exact Mass: 373,96 Molecular Weight: 375,67 | DJ-9 | $IC_{50}$ = 3.15 ug/ml 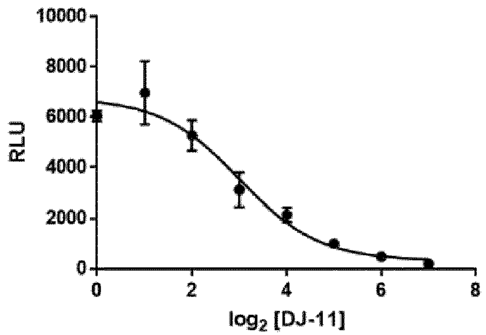  |

(continued)

| Compound ID§ | *alias* | $IC_{50}$* |
|---|---|---|
| 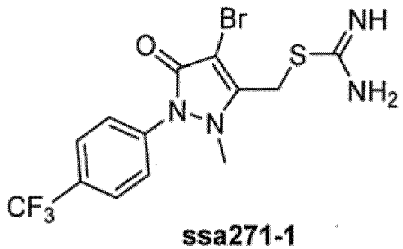 **ssa271-1** Chemical Formula: $C_{13}H_{12}BrF_3N_4OS$ Exact Mass: 407,99 Molecular Weight: 409,23 | DJ-10 | $IC_{50}$ = 10.83 μg/ml 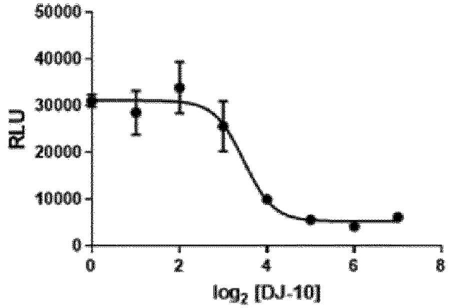  |
| 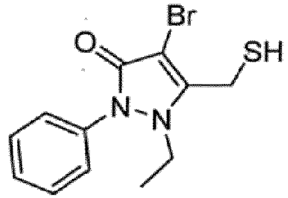 **P36** Chemical Formula: $C_{12}H_{13}BrN_2OS$ Exact Mass: 311,99 Molecular Weight: 313,21 | DJ-12 | $IC_{50}$ = 3.15 μg/ml 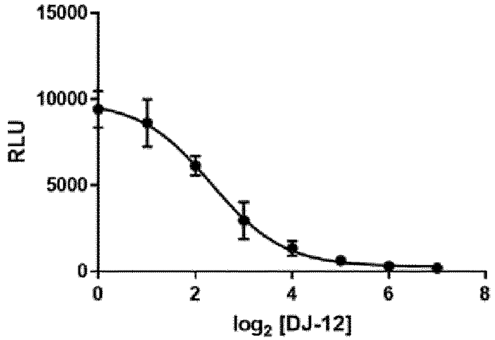  |
| 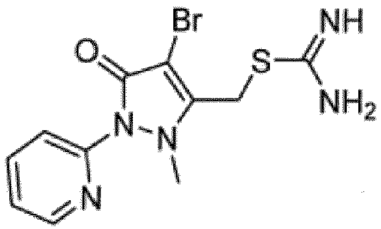 **P34** Chemical Formula: $C_{11}H_{12}BrN_5OS$ Exact Mass: 340,99 Molecular Weight: 342,22 | DJ-13 | Weak activity (+/- 50 % of non-inhibited control) ($IC_{50}$ not determined) |

(continued)

| Compound ID§ | *alias* | $IC_{50}$* |
|---|---|---|
| 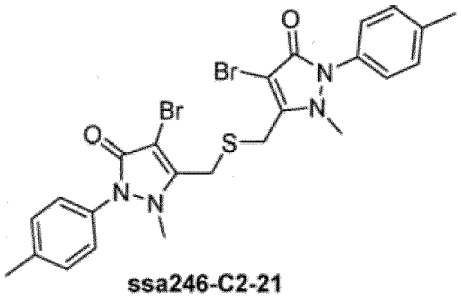 **ssa246-C2-21** Chemical Formula: $C_{24}H_{24}Br_2N_4O_2S$ Exact Mass: 590,00 Molecular Weight: 592,35 | DJ-14 | Weak activity (+/- 90 % of control) ($IC_{50}$ not determined) |
| 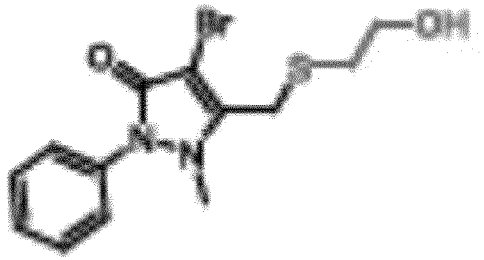 P19 | DJ-3 | No $IC_{50}$ 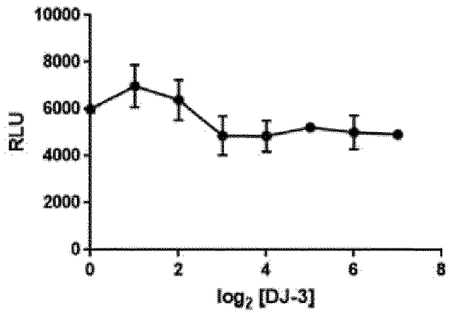  ➔ ***Not active*** |

(continued)

| Compound ID§ | *alias* | $IC_{50}$* |
|---|---|---|
| 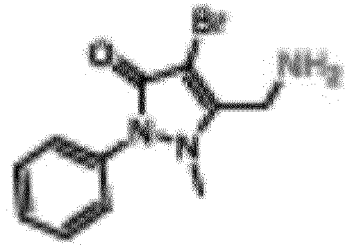 | DJ-4 | No $IC_{50}$ 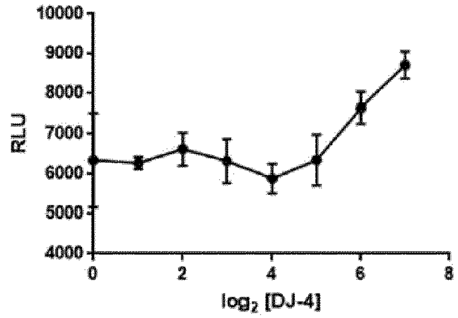 **➔ Not active** |

* The anti-flagellar assay for determination of the $IC_{50}$ for the test compounds was performed as described in the Examples (e.g. Example 1, Methods) using the *H. pylori flaA*-luciferase reporter strain.

§ Reference compound Active 2a was resynthesized as alias DJ-1 and subjected to the disclosed assay (with $IC_{50}$ determined as 3.15 µg/ml, consistent with the $IC_{50}$ determined for closely analogues lead compound Active 2 (BL2) of 2.75 µg/ml (see Figure 3B).

Further testing for potential antiviability effects in a secondary assay:

For further testing, all new compound variants which demonstrated inhibitory activity in the primary flaA-luciferase assay (*i.e.*, DJ-1 (= resynthesized Active 2a), DJ-2, DJ-5, DJ-6, DJ-7, DJ-8, DJ-9, DJ-10, DJ-11, DJ-12, and DJ-13, were tested for antibacterial activity on *H. pylori* (strain N6) in a secondary assay (growth assay) by monitoring $OD_{600}$ as a proxy of bacterial proliferation over time, at concentrations of 20 μg/ml.

Strinkingloy, in this secondary assay, all listed compounds did not exhibit any classical antibacterial (antibiotic - bactericidal or bacteriostatic) activity and therefore did not inhibit *H. pylori* proliferation, indicating that all these derivatives of Active 2a also act exclusively as inhibitors of bacterial motility without affecting viability.

**Example 5: Chemical Synthesis**

**General Route A**

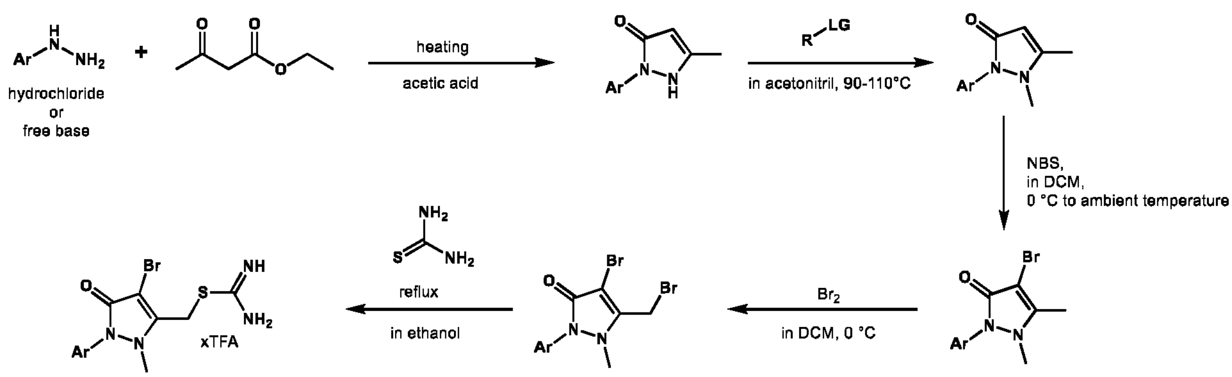


Procedure A1.1

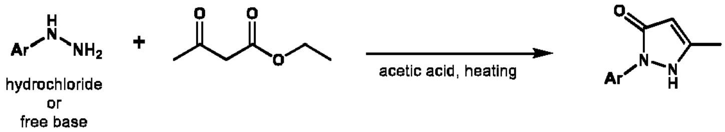


1.0 mmol of the respective hydrazine or its respective hydrochloride and 1 mmol of ethyl acetoacetate were dissolved in 2 ml acetic acid in a sealed tube. The reaction mixture was than stirred at ambient temperature for 3 h. The reaction mixture was than stirred at 100 °C for 3 h and afterwards at 80 °C for 15 h. After cooling down the reaction mixture was dissolved in ethylacetate. The organic phase was washed with saturated $NaHCO_3$ solution once and phases separated. The organic phase was dried over $Na_2SO_4$, filtered and the solvent removed under reduced pressure. The crude material was purified by normal phase column chromatography but could not purified completely. The crude product was used without further purification.

Example 5.1: 5-methyl-2-(p-tolyl)-1,2-dihydro-3H-pyrazol-3-one

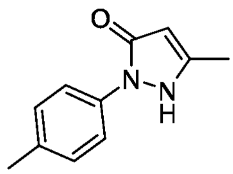

Synthesized according to procedure A1.1

Yield: 101 mg (54%)

$^1$H-NMR (500 MHz, methanol-d4) δ = 2.22 (s, 3H, $CH_3$), 2.36 (s, 3H, $CH_3$), 7.27 (dd, 2H, *J* = 8.7 Hz, *J* = 0.6 Hz, 2x CH), 7.46 (d, 2H, *J* = 8.5 Hz, 2x CH) ppm.

Low resolution LC/MS data (method A): 1.18 min, m/z 189 [M+H]+

Example 5.2: 2-(3,5-dimethylphenyl)-5-methyl-1,2-dihydro-3H-pyrazol-3-one

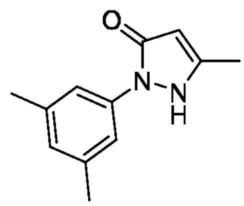

Synthesized according to procedure A1.1

Yield: 112 mg

$^1$H-NMR (500 MHz, methanol-d4) δ = 2.22 (s, 3H, $CH_3$), 2.34 (s, 6H, 2x $CH_3$), 6.94-6.97 (m, 1H, CH), 7.20-7.24 (m, 2H, 2x CH) ppm.

Low resolution LC/MS data (method A): 1.51 min, m/z 203 [M+H]+

Example 5.3: 2-(4-isopropylphenyl)-5-methyl-1,2-dihydro-3H-pyrazol-3-one

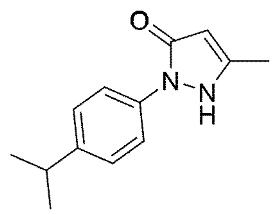

Synthesized according to procedure A1.1

Yield: 147 mg

$^1$H-NMR (500 MHz, methanol-d4) δ = 1.27 (d, 6H, *J* = 6.9 Hz, 2x $CH_3$), 2.22 (s, 3H, $CH_3$), 2.94 (sept., 1H, *J* = 6.9 Hz, CH), 7.33 (d, 2H, *J* = 8.5 Hz, 2x CH), 7.50 (d, 2H, *J* = 8.5 Hz, 2x CH) ppm.

Low resolution LC/MS data (method A): 1.51 min, m/z 203 [M+H]+

Procedure A1.2

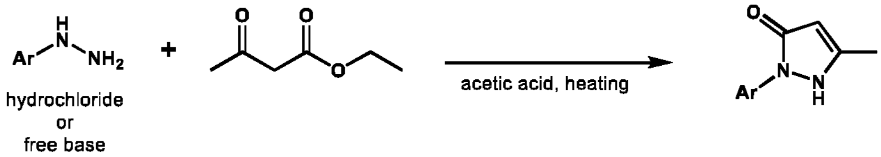


1.0 mmol of the hydrazine or its respective hydrochloride and 1 mmol of its respective hydrochloride were dissolved in 2 ml - 4 ml acetic acid in a sealed tube. The reaction mixture was at 110 °C for 16 h. After cooling down the reaction mixture was dissolved in ethyl acetate. The organic phase was washed with saturated $NaHCO_3$ solution once. The organic phase was dried over $Na_2SO_4$, filtered and the solvent removed under reduced pressure. The crude material was purified by normal phase column chromatography but could not purified completely. The crude product was used without further purification.

Example 5.4: 2-([1,1'-biphenyl]-4-yl)-5-methyl-1,2-dihydro-3H-pyrazol-3-one

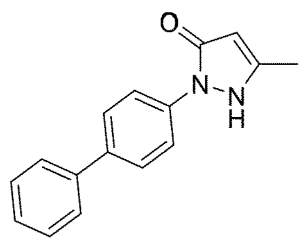

Synthesized according to procedure A1.2

Yield: 117 mg

$^1$H-NMR (500 MHz, methanol-d4) $\delta$ = 2.25 (s, 3H, $CH_3$), 7.33-7.37 (m, 1H, CH), 7.42-7.48 (m, 2H, 2x CH), 7.62-7.66 (m, 2H, 2x CH), 7.72 (s, 4H, 4x CH) ppm.

Low resolution LC/MS data (method A): 1.58 min, m/z 251 [M+H]+

Example 5.5: 2-(4'-chlorophenyl)-5-methyl-1,2-dihvdro-3*H*-pyrazol-3-one

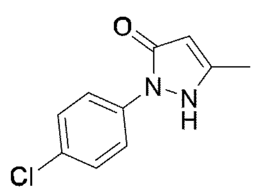

Synthesized according to procedure A1.2

Yield: 133 mg

$^1$H-NMR (700 MHz, methanol-d4) δ = 2.22 (s, 3H, $CH_3$), 7.44 (d, 2H, *J* = 8.9 Hz, CH), 7.64 (d, 2H, *J* = 8.9 Hz, 2x CH) ppm.

Low resolution LC/MS data (method A): 1.37 min, m/z 209 [M+H]+

Example 5.6: 5-methyl-2-(4'-(trifluoromethyl)phenyl)-1,2-dihydro-3*H*-pyrazol-3-one

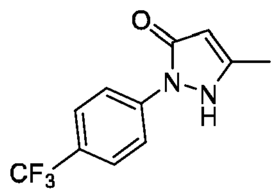

Synthesized according to procedure A1.2

Yield: 198 mg

$^1$H-NMR (700 MHz, methanol-d4) δ = 2.24 (s, 3H, $CH_3$), 7.74 (d, 2H, *J* = 8.6 Hz, 2x CH), 7.90 (d, 2H, *J* = 8.5 Hz, 2x CH) ppm.

Low resolution LC/MS data (method A): 1.60 min, m/z 243 [M+H]+

**Procedure A2**

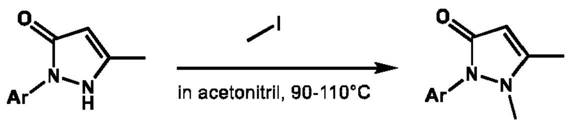


Reaction was carried out unter Argon atmosphere using Schlenk technique. 1.0 eq of the respective pyrazolidinone and an excess of iodomethane from at least 7.5 eq were dissolved in 2 ml dry acetonitrile. The reaction was than stirred for 2 h at 110 °C and then stirred for 12 h at 90 °C to 110 °C. After cooling to ambient temperature the mixture was dissolved in ethyl acetate. The organic phase was washed with saturated $NaHCO_3$ solution once. The phases were separated and the aqueous phase extracted once with ethyl acetate. The combined organic phases were dried over $Na_2SO_4$, filtered and the solvent removed under reduced pressure. The crude material was purified by normal phase column chromatography.

Example 5.7: 1,5-dimethyl-2-(p-tolyl)-1,2-dihvdro-3*H*-pyrazol-3-one

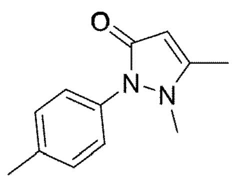

Synthesized according to procedure A2

Yield: 35% over two steps (69 mg)

$^1$H-NMR (700 MHz, methanol-d4) δ = 2.29 (d, 3H, *J* = 0.7 Hz, $CH_3$), 2.41 (s, 3H, $CH_3$), 3.18 (s, 3H, $CH_3$), 5.34 (s, 1H, CH) 7.23 (d, 2H, *J* = 8.3 Hz, 2x CH), 7.35 (d, 2H, *J* = 8.0 Hz, 2x CH) ppm.

Low resolution LC/MS data (method A): 1.15 min, m/z 203 [M+H]+

Example 5.8: 1,5-dimethyl-2-(3',5'-dimethylphenyl)-1,2-dihvdro-3*H*-pyrazol-3-one

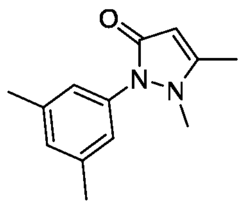

Synthesized according to procedure A2

Yield: 43% over two steps (69 mg)

$^1$H-NMR (700 MHz, methanol-d4) δ = 2.29 (d, 3H, *J* = 0.8 Hz, $CH_3$), 2.37 (s, 3H, $CH_3$), 2.37 (s, 3H, $CH_3$), 3.18 (s, 3H, $CH_3$), 5.03 (s, 1H, CH), 6.94-6.97 (m, 2H, 2x CH), 7.09-7.12 (m, 1H, CH) ppm.

Low resolution LC/MS data (method A): 1.26 min, m/z 217 [M+H]+

Example 5.9: 2-(4'-isopropylphenyl)-1,5-dimethyl-1,2-dihydro-3*H*-pyrazol-3-one

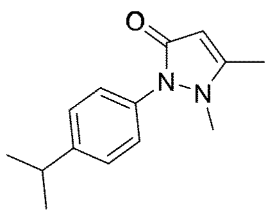

Synthesized according to procedure A2

Yield: 48 % over two steps (110 mg)

$^1$H-NMR (500 MHz, methanol-d4) δ = 1.29 (d, 6H, *J* = 7.0 Hz, 2x $CH_3$), 2.30 (s, 3H, $CH_3$), 2.99 (sept., 1H, *J* = 7.0 Hz, CH), 5.35 (s, 1H, CH), 7.27 (d, 2H, *J* = 8.5 Hz, 2x CH), 7.42 (d, 2H, *J* = 8.2 Hz, 2x CH) ppm.

Low resolution LC/MS data (method A): 1.36 min, m/z 231 [M+H]+

Example 5.10: 2-([1',1"-biphenyl1-4'-yl)-1,5-dimethyl-1,2-dihydro-3*H*-pyrazol-3-one

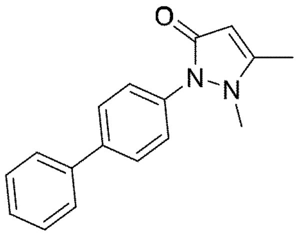

Synthesized according to procedure A2

Yield: 30% over two steps (77mg)

$^1$H-NMR (700 MHz, methanol-d4) δ = 2.32 (s, 3H, $CH_3$), 3.21 (s. 3H, $CH_3$), 5.39 (s, 1H, CH), 7.36-7.39 (m, 1H, CH), 7.44 (d, 2H, *J* = 8.6 Hz, 2x CH), 7.45-7.48 (m, 2H, 2x CH), 7.65-7.68 (m, 2H, 2x CH), 7.79 (d, 2H, *J*= 8.6 Hz, 2x CH) ppm.

Low resolution LC/MS data (method A): 1.45 min, m/z 265 [M+H]+

Example 5.11: 2-(4'-chlorophenyl)-1,5-dimethyl-1,2-dihvdro-3H-pyrazol-3-one

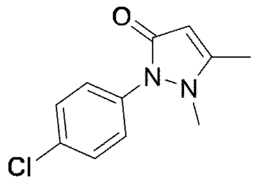

Synthesized according to procedure A2

Yield: 38% over two steps (83 mg)

$^1$H-NMR (500 MHz, methanol-d4): δ = 2.30 (s, 3H, $CH_3$), 3.18 (s 3H, $CH_3$), 5.37 (s, 1H, CH), 7.36 (d, 2H, *J* = 8.9 Hz, 2x CH), 7.54 (d, 2H, *J* = 8.9 Hz, 2x CH) ppm.

Low resolution LC/MS data (method A): 1.19 min, m/z 223 [M+H]+

Example 5.12: 1,5-dimethyl-2-(4'-(trifluoromethyl)phenyl)-1,2-dihydro-3*H*-pyrazol-3-one

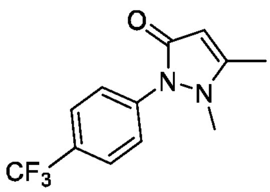

Synthesized according to procedure A2

Yield: 30% (83 mg)

$^1$H-NMR (500 MHz, methanol-d4) $\delta$ = 2.33 (d, 3H, $J$ = 0.8 Hz, $CH_3$), 3.20 (s, 3H, $CH_3$), 5.41 (s, 1H, CH), 7.58 (d, 2H, $J$ = 8.3 Hz, 2x CH), 7.83 (d, 2H, $J$ = 8.4 Hz, 2x CH) ppm.

Low resolution LC/MS data (method A): 1.35 min, m/z 257 [M+H]+

**Prodecure A3**

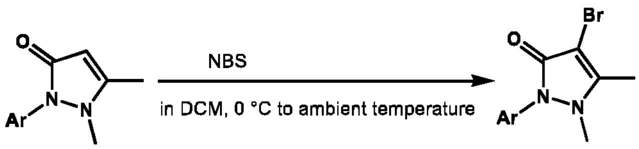


The reaction was carried out under Argon atmosphere using Schlenk technique. 1.0 eq of the respective methylated pyrazolidinone was dissolved in dry dichloromethane and the solution was cooled to 0 °C. 1.0 eq of N-bromsuccinimide (NBS) was added subsequently over 2 h. The reaction was further stirred for half an hour at 0 °C and then warmed up to ambient temperature. The reaction mixture was stirred at ambient temperature for 1 h. The solvent was removed under reduced pressure. The raw material was dissolved in ethyl acetate and the organic phase was washed three time with saturated $NaHCO_3$ solution and two times with water. The combined organic phases were dried over $Na_2SO_4$, filtered and the solvent removed under reduced pressure. The crude product was used without further purification.

Example 5.13: 4-bromo-1,5-dimethyl-2-(p-tolyl)-1,2-dihydro-3*H*-pyrazol-3-one

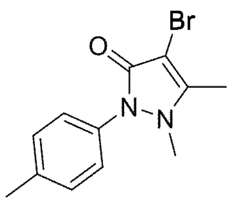

Synthesized according to procedure A3

low resolution LC/MS data (method A): 1.43 min, m/z 295 and 297 [M+H]+

Example 5.14: 4-bromo-2-(3',5'-dimethylphenyl)-1,5-dimethyl-1,2-dihydro-3*H*-pyrazol-3-one

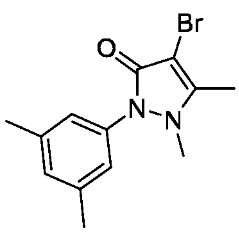

Synthesized according to procedure A3

low resolution LC/MS data (method A): 1.31 min, m/z 281 and 283 [M+H]+

Example 5.15: 4-bromo-2-(4'-isopropylphenyl)-1,5-dimethyl-1,2-dihydro-3*H*-pyrazol-3-one

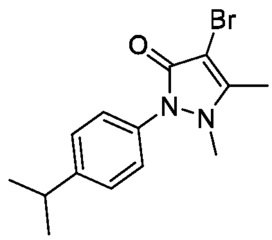

Synthesized according to procedure A3

low resolution LC/MS data (method A): 1.56 min, m/z 309 and 311 [M+H]+

Example 5.16: 2-([1',1"-biphenyl]-4'-yl)-4-bromo-1,5-dimethyl-1,2-dihydro-3*H*-pyrazol-3-one

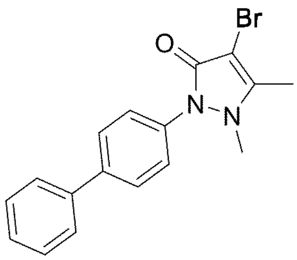

Synthesized according to procedure A3

low resolution LC/MS data (method A): 1.62 min, m/z 343 and 345 [M+H]+

Example 5.17: 4-bromo-2-(4'-chlorophenyl)-1,5-dimethyl-1,2-dihvdro-3H-pyrazol-3-one

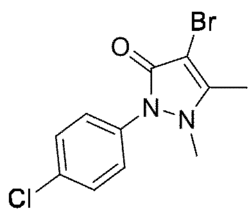

Synthesized according to procedure A3

low resolution LC/MS data (method A): 1.43 min, m/z 301 and 303 and 305 [M+H]+

Example 5.18: 4-bromo-1,5-dimethyl-2-(4'-(trifluoromethyl)phenyl)-1,2-dihvdro-3/-/-pyrazol-3-one

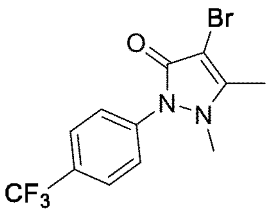

Synthesized according to procedure A3

low resolution LC/MS data (method A): 1.54 min, m/z 335 and 337 [M+H]+

**Procedure A4**

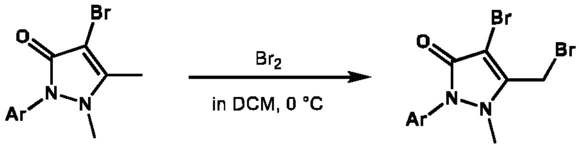


The reaction was carried out under Argon atmosphere using Schlenk technique. 1.0 eq of the respective mono-brominated pyrazolidinone was dissolved in dry dichloromethane and the solution was cooled to 0 °C. 1.0 eq of bromine, dissolved in dry dichloromethane was added dropwise over 2 h. The reaction was warmed up to ambient temperature and kept there for 1.75 h. The reaction was diluted with saturated $NaHCO_3$. The phases were separated and the aqueous was extracted two times with dichloromethane. The solvent of the combined organic phases was removed under reduced pressure. The crude material was purified by normal phase column chromatography but could not purified completely. The crude product was used without further purification.

Example 5.19: 4-bromo-5-(bromomethyl)-1-methyl-2-(p-tolyl)-1,2-dihydro-3*H*-pyrazol-3-one

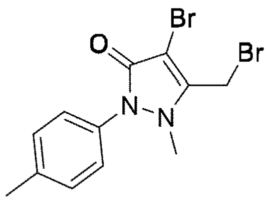

Synthesized according to procedure A4

low resolution LC/MS data (method A): 1.48 min, m/z 317 (100% intensity) [M+H]+

Example 5.20: 4-bromo-5-(bromomethyl)-2-(3',5'-dimethylphenyl)-1-methyl-1,2-dihydro-3*H*-pyrazol-3-one

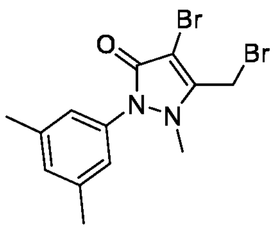

Synthesized according to procedure A4

low resolution LC/MS data (method A): 1.62 min, m/z 331 (100% intensity) [M+H]+

Example 5.21: 4-bromo-5-(bromomethyl)-2-(4'-isopropylphenyl)-1 -methyl-1,2-dihydro-3*H*-pyrazol-3-one

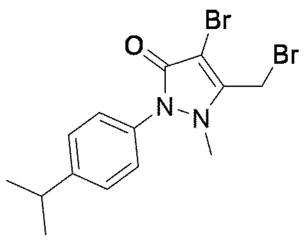

Synthesized according to procedure A4

low resolution LC/MS data (method A): 1.70 min, m/z 389 (100% intensity) [M+H]+

Example 5.22: 2-([1',1"-biphenyl]-4'-yl)-4-bromo-5-(bromomethyl)-1-methyl-1,2-dihydro-3*H*-pyrazol-3-one

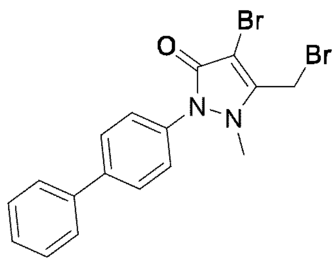

Synthesized according to procedure A4

low resolution LC/MS data (method A): 1.72 min, m/z 423 (100% intensity) [M+H]+

Example: 5.23: 4-bromo-5-(bromomethyl)-2-(4'-chlorophenyl)-1-methyl-1,2-dihvdro-3*H*-pyrazol-3-one

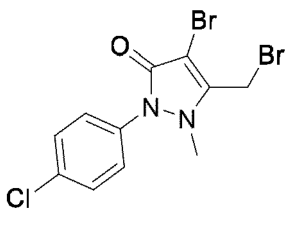

Synthesized according to procedure A4

low resolution LC/MS data (method A): 1.53 min, m/z 381 (100% intensity) [M+H]+

Example: 5.24: 4-bromo-5-(bromomethyl)-1-methyl-2-(4'-(trifluoromethyl)phenyl)-1,2-dihydro-3*H*-pyrazol-3-one

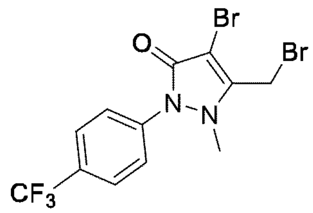

Synthesized according to procedure A4

low resolution LC/MS data (method A): 1.65 min, m/z 415 (100% intensity) [M+H]+

**Procedure A5.1**

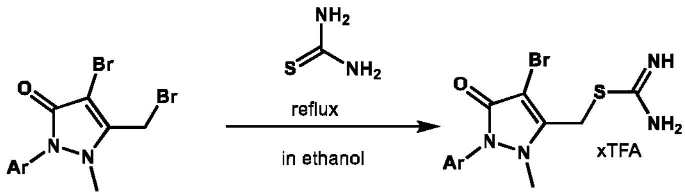


1.0 eq of the respective mono-brominated pyrazolidinone and 1.0 eq of thiourea were dissolved in ethanol in a sealed tube. The reaction mixture was heated to 80 °C and stirred at this temperature for 1 h. The reaction mixture was cooled down to ambient temperature and then stirred at ambient temperature for 12 h. The solvent was removed under reduced pressure and the resulting crude product purified by HPLC. After freeze drying the product was isolated as colorless solid, forming a TFA salt.

**Procedure A5.2**

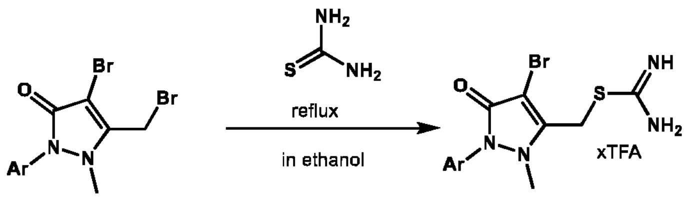


1.0 eq of the respective mono-brominated pyrazolidinone and 1.0 eq of thiourea were dissolved in ethanol in a sealed tube. The reaction mixture was heated to 80 °C and stirred at this temperature for 1.5 h. The reaction mixture was cooled down to ambient temperature. The solvent was removed under reduced pressure and the resulting crude product purified by HPLC. After freeze drying the product was isolated as colorless solid, forming a TFA salt.

Example 5.25: (4-bromo-2-methyl-5-oxo-1-(p-tolyl)-2,5-dihydro-1*H*-pyrazol-3-yl)methyl carbamimidothioate

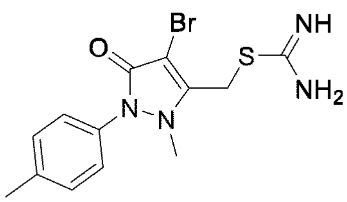

TFA salt

Synthesized according to procedure A5.2

$^{1}$H-NMR (500 MHz, methanol-d4): δ = 2.42 (s, 3H, $CH_3$), 3.30 (s, 3H, $CH_3$), 4.62 (s, 2H, $CH_2$), 7.26 (d, 2H, *J* = 8.3 Hz, 2x CH), 7.39 (d, 2H, *J* = 8.3 Hz, 2x CH) ppm.

$^{19}$F-NMR (500 MHz, methanol-d4): δ = -77.0 (s, $F_3CCOOH$) ppm.

$^{13}$C{$^{1}$H}-NMR (700 MHz, methanol-d4): δ=21.3, 27.4, 36.4, 90.3, 127.7, 131.5, 132.0, 141.2, 149.0, 162.8, 170.3 ppm.

Low resolution LC/MS data (method A): 0.40 min, m/z 355 and 357 [M+H]+

<u>Example 5.26: (4-bromo-1-(3',5'-dimethylphenyl)-2-methyl-5-oxo-2,5-dihydro-1*H*-pyrazol-3-yl)methyl carbamimidothioate</u>

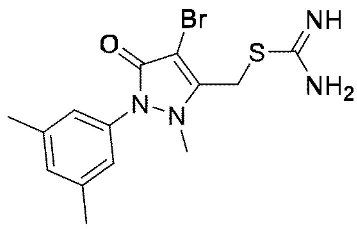

TFA salt

Synthesized according to procedure A5.2

$^{1}$H-NMR (500 MHz, methanol-d4) δ = 2.38 (s, 3H, $CH_3$), 2.38 (s, 3H, $CH_3$), 3.30 (s, 3H, $CH_3$), 4.62 (s, 2H, $CH_2$), 6.98-7.01 (m, 2H, 2x CH), 7.15-7.17 (m, 1H, 2x CH) ppm.

$^{19}$F-NMR (500 MHz, methanol-d4) δ = -77.0 (s, $F_3CCOOH$) ppm.

$^{13}$C{$^{1}$H}-NMR (700 MHz, methanol-d4) δ = 21.4, 27.4, 36.5, 90.5, 125.3, 132.0, 134.4, 141.2, 149.3, 162.8, 170.3 ppm.

Low resolution LC/MS data (method A): 0.84 min, m/z 369 and 371 [M+H]+

<u>Example 5.27: (4-bromo-1-(4'-isopropylphenyl)-2-methyl-5-oxo-2,5-dihydro-1H-pyrazol-3-vl)methyl carbamimidothioate</u>

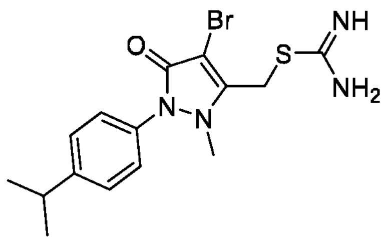

TFA salt

Synthesized according to procedure A5.2

$^1$H-NMR (500 MHz, methanol-d4) δ = 1.29 (d, 6H, *J* = 6.9 Hz, $CH_3$), 3.00 (sept., 1H, *J* = 6.9 Hz, CH), 3.31 (s, 3H, $CH_3$), 4.62 (s, 2H, $CH_2$), 7.31 (d, 2H, *J* = 8.4 Hz, 2x CH), 7.45 (d, 2H, *J* = 8.3 Hz, 2x CH) ppm.

$^{19}$F-NMR (500 MHz, methanol-d4) δ = -77.0 (s, $F_3CCOOH$) ppm.

$^{13}$C{$^1$H}-NMR (700 MHz, methanol-d4) δ = 24.4, 27.4, 35.4, 36.4, 90.4, 118.0 (q, *J* = 291 Hz), 127.9, 129.0, 132.1, 149.1, 152.0, 162.5 (q, *J* = 36 Hz), 162.8, 170.3 ppm.

Low resolution LC/MS data (method A): 0.98 min, m/z 383 and 385 [M+H]+

Example 5.28: (1-([1',1"-biphenyl]-4'-yl)-4-bromo-2-methyl-5-oxo-2,5-dihydro-1*H*-pyrazol-3-yl)methyl carbamimidothioate

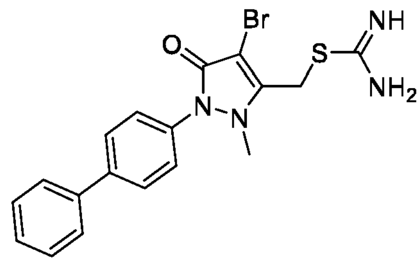

TFA salt

Synthesized according to procedure A5.1

$^1$H-NMR (700 MHz, methanol-d4) δ = 3.36 (s, 3H, $CH_3$), 4.65 (s, 2H, $CH_2$), 7.38-7.42 (m, 1H, CH), 7.46-7.51 (m, 4H, 4x CH), 7.65-7.69 (m, 2H, 2x CH), 7.81-7.85 (m, 2H, 2x CH) ppm.

$^{19}$F-NMR (500 MHz, methanol-d4) δ = -77.0 (s, $F_3CCOOH$) ppm.

$^{13}$C{$^1$H}-NMR (700 MHz, methanol-d4) δ = 27.5, 36.8, 91.0, 127.7, 128.3, 129.3, 129.4, 130.3, 133.8, 141.1, 143.5, 150.2, 163.0, 170.3 ppm.

Low resolution LC/MS data (method A): 1.08 min, m/z 417 and 419 [M+H]+

Example 5.29: (4-bromo-1-(4'-chlorophenyl)-2-methyl-5-oxo-2,5-dihydro-1*H*-pyrazol-3-vl)methyl carbamimidothioate

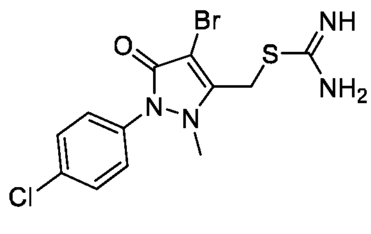

TFA salt

Synthesized according to procedure A5.1

$^1$H-NMR (700 MHz, methanol-d4) δ = 3.30 (s, 3H, $CH_3$), 4.63 (s, 2H, $CH_2$), 7.40 (d, 2H, *J* = 8.8 Hz, 2x CH), 7.58 (d, 2H, *J* = 8.8 Hz, 2x CH) ppm.

$^{19}$F-NMR (500 MHz, methanol-d4) δ = -77.0 (s, $F_3CCOOH$) ppm.

$^{13}$C{$^1$H}-NMR (700 MHz, methanol-d4) δ = 27.5, 36.9, 91.4, 118.2 (q, *J* = 292 Hz, $CF_3COOH$), 128.6, 131.0, 133.6, 135.8, 151.0, 162.8, 163.1, 170.3 ppm.

Low resolution LC/MS data (method A): 0.56 min, m/z 375 and 377 (100% int.) and 379 [M+H]+

Example 5.30: (4-bromo-2-methyl-5-oxo-1-(4'-(trifluoromethyl)phenyl)-2,5-dihvdro-1H-pyrazol-3-yl)methyl carbamimidothioate

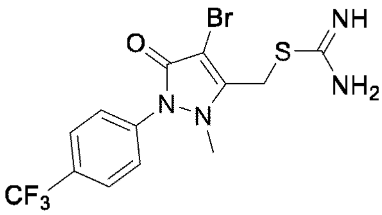

TFA salt

Synthesized according to procedure A5.1

$^1$H-NMR (500 MHz, methanol-d4): δ = 3.32 (s, 3H, $CH_3$), 4.64 (s, 2H, $CH_2$), 7.63 (d, 2H, *J* = 8.3 Hz, 2x CH), 7.88 (d, 2H, *J* = 8.4 Hz, 2x CH) ppm.

$^{19}$F-NMR (500 MHz, methanol-d4): δ = -77.0 (s, $F_3CCOOH$), -64.1 (s, $CF_3$-$C_q$) ppm.

$^{13}$C{$^1$H}-NMR (700 MHz, methanol-d4): δ = 27.6, 37.5, 92.5, 118.2 (q, *J* = 293 Hz, $CF_3COOH$), 125.4 (q, *J* = 272 Hz, $CF_3$), 126.4, 127.9 (q, J = 4 Hz, CH), 131.0 (q, *J* = 33 Hz, $C_q$), 138.5, 152.8, 152.9, 163.2 (q, *J* = 33 Hz, $CF_3COOH$), 163.2, 170.3 ppm

Low resolution LC/MS data (method A): 0.92 min, m/z 409 and 411 [M+H]+

Example 5.31: 5,5'-(thiobis(methylene))bis(4-bromo-1-methyl-2-(p-tolyl)-1,2-dihydro-3*H*-pyrazol-3-one)

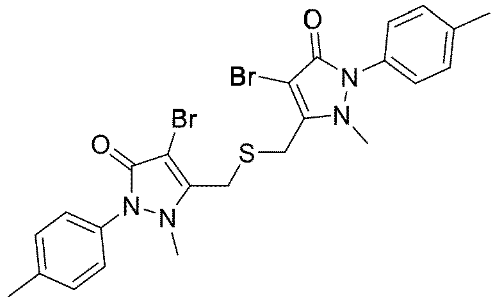

Synthesized according to procedure A5.1

$^{1}$H-NMR (500 MHz, methanol-d4) $\delta$ = 2.41 (s, 6H, 2x $CH_3$), 3.31 (s, 6H, 2x $CH_3$), 4.09 (s, 4H, 2x $CH_2$), 7.26 (m, 4H, 4x CH), 7.37 (m, 4H, 4x CH) ppm.

$^{13}$C{$^{1}$H}-NMR (700 MHz, methanol-d4) $\delta$ = 21.3, 26.9, 36.2, 88.96, 88.98, 127.9, 131.5, 132.2, 141.0, 151.5, 151.6, 163.1 ppm.

Low resolution LC/MS data (method A): 1.60 min, m/z 591 and 593 and 595 [M+H]+

## Route B

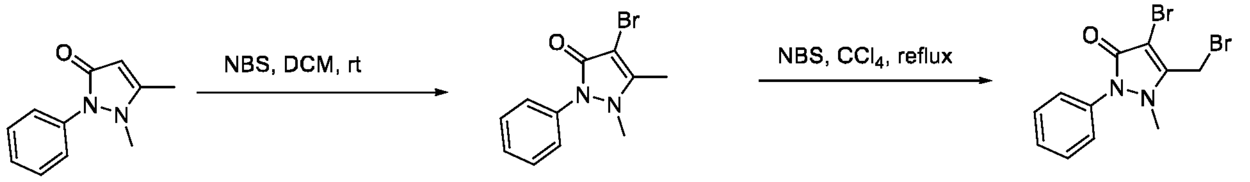


1 2 3

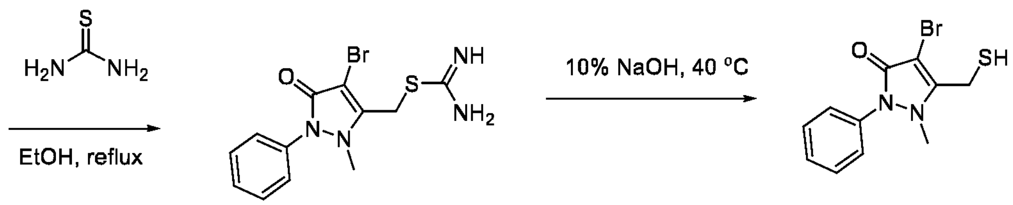


4 5

Example 5.32: 4-bromo-5-(bromomethyl)-1-methyl-2-phenyl-1,2-dihydro-3H-pyrazol-3-one, 3

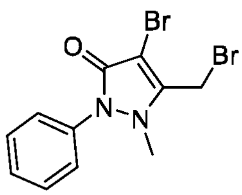

Antipyrene (0.94 g, 4.99 mmol) was dissolved in DCM (10 ml) and was allowed to stir in open atmosphere at

0 °C. NBS (0.889 g, 4.99 mmol) was added to the solution in three portions over 30 min. After complete addition, the reaction was stirred at room temperature for 30 min. After the reaction time was over, it was quenched with saturated aqueous $K_2CO_3$ solution (30 mL). The reaction mixture was extracted with ethyl acetate. The organic phase was washed with water two times and dried over $Na_2SO_4$. The organic portion was evaporated under vacuum to get the crude compound 4-bromo-1,5-dimethyl-2-phenyl-1,2-dihydro-3H-pyrazol-3-one 2.

2 (1.22 g, 4.57 mmol) was dissolved in $CCl_4$. NBS (70.0 mg, 0.39 mmol) was added to it in 6 portions over 3 h while refluxing. The reaction mixture was further refluxed for 4 h. The solid was filtered and extracted with 1 ml hot $CCl_4$. The filtrate were evaporated and the residue purified with 60 % ethyl acetate in petroleum ether to give 4-bromo-5-(bromomethyl)-1-methyl-2-phenyl-1,2-dihydro-3H-pyrazol-3-one .

Yield: 77 % (100 mg)

$^1$H NMR (400 MHz, methanol-$d_4$) $\delta$ = 7.57 (t, 2H, *J* = 7.6 Hz, 2x CH), 7.49 (t, 1H, *J* = 7.4 Hz, CH), 7.39 (d, 2H, *J* = 7.7 Hz, 2x CH), 4.64 (s, 2H, $CH_2Br$), 3.35 - 3.22 (m, 3H, $CH_3$) ppm.

$^{13}$C{$^1$H} NMR (101 MHz, methanol-$d_4$) $\delta$ = 148.5, 133.2, 129.4, 128.8, 125.9, 89.5, 35.2, 26.0 ppm.

Example 5.33: (4-bromo-2-methyl-5-oxo-1-phenyl-2,5-dihydro-1*H*-pyrazol-3-yl)methyl carbamimidothioate, **4**

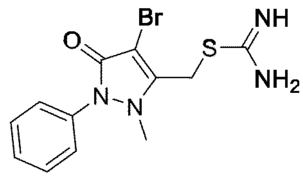

4-bromo-5-(bromomethyl)-1-methyl-2-phenyl-1,2-dihydro-3H-pyrazol-3-one, **3** (1.1 g, 3.18 mmol) in Ethanol (8ml) and thiourea (0.242 g, 3.18 mmol) in ethanol (4 ml) was added to it. The reaction mixture was refluxed for 1 h. The solvent was evaporated and the solid purified by HPLC to give (4-bromo-2-methyl-5-oxo-1-phenyl-2,5-dihydro-1*H*-pyrazol-3-yl)methyl carbamimidothioate as TFA salt.

$^1$H-NMR (400 MHz, methanol-$d_4$): $\delta$ = 7.60 (t, 2H, *J* = 7.6 Hz, 2x CH), 7.52 (t, 1H, *J* = 7.4 Hz, CH), 7.42 (d, 2H, *J* = 7.5 Hz, 2x CH), 4.65 (s, 2H, $CH_2$), 3.33 (s, 3H, $CH_3$) ppm.

$^{13}$C{$^1$H}-NMR (101 MHz, methanol-$d_4$): $\delta$ = 168.7, 161.4, 148.5, 133.2, 129.4, 128.8, 125.9, 89.3, 35.1, 25.9 ppm.

HRMS (ESI): calculated for $C_{12}H_{13}BrN_4OS$ ([M+Na]+): m/z 362 9891
measured: m/z 362.9892.

Example 5.34: 4-bromo-5-(mercaptomethyl)-1-methyl-2-phenyl-1,2-dihydro-3*H*-pyrazol-3-one, **5**

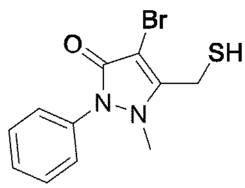

(4-bromo-2-methyl-5-oxo-1-phenyl-2,5-dihydro-1H-pyrazol-3-yl)methyl carbamimidothioate, **4** (90 mg, 0.264 mmol) was dissolved in 10% aqueous sodium hydroxide (0.5 ml, 0.26 mmol) and heated at 40 °C for 2 h. The reaction mixture was cooled and extracted with DCM (1 ml). The organic layer was dried and concentrated to give the crude product, which was purified by HPLC to yield **5**.

Yield: 13 % (10 mg)

$^1$H-NMR (400 MHz, $CDCl_3$): δ = 7.48 (t, 2H, $J$ = 7.7 Hz, 2x CH), 7.43 - 7.29 (m, 3H, 3x CH), 3.71 (d, 2H, $J$ = 8.0 Hz, $CH_2SH$), 3.22 (s, 3H, $CH_3$), 2.11 (t 1H, $J$ = 8.0 Hz, SH) ppm.

$^{13}C\{^1H\}$-NMR (101 MHz, $CDCl_3$): δ = 161.7, 154.1, 133.8, 129.6, 129.5, 128.0, 125.1, 89.5, 36.4, 18.8 ppm.

high resolution MS (ESI): calculated for $C1_1H_{11}BrN_2OS$ ([M+Na]+): m/z 320.9673
measured: m/z 320.9679.

**Route C**

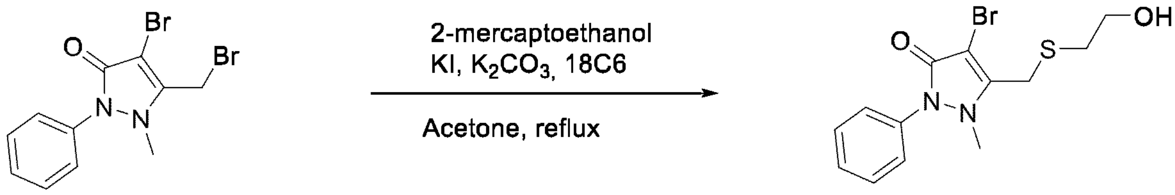


**3** **6**

Example 5.35: 4-bromo-5-(((2-hydroxyethyl)thio)methyl)-1-methyl-2-phenyl-1,2-dihydro-3*H*-pyrazol-3-one, **6**

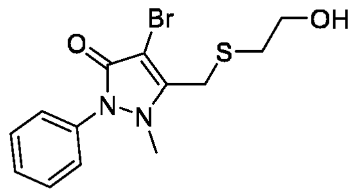

4-bromo-5-(bromomethyl)-1-methyl-2-phenyl-1,2-dihydro-3*H*-pyrazol-3-one, **5** (100 mg, 0.29 mmol) and 2-mercaptoethan-1-ol (20.95 μl, 0.30 mmol) were added to a stirred suspension of potassium carbonate (100 mg, 0.72 mmol), 18-crown-6 (0.806 μl, 3.78 μmol) and potassium iodide (1 mg, 6.02 μmol) in dry acetone. The mixture refluxed for 15 h, cooled to ambiente temeratrue, filtered and concentrated under reduced pressure. Water was then added to the residue and the mixture was then extracted three times with diethylether. The combined organic layers were washed with water (2 ml), saturated NaCl solution (2 ml) and dried over $MgSO_4$. This was then concentrated under reduced pressure to give the crude product. The crude product was purified by HPLC to give 4-bromo-5-(((2-hydroxyethyl)thio)methyl)-1-methyl-2-phenyl-1,2-dihydro-3*H*-pyrazol-3-one.

Yield: 5 % (5 mg)

$^1$H-NMR (400 MHz, $CDCl_3$): δ = 7.50 (t, 2H, $J$ = 7.6 Hz, 2x CH), 7.39 (t, 3H, $J$ = 8.8 Hz, 3x CH), 3.85 (t, 2H, $J$ = 5.9 Hz, $CH_2OH$), 3.79 (s, 2H, $CH_2S$), 3.27 (s, 3H, $CH_3$), 2.85 (t, 2H, $J$ = 5.9 Hz, $CH_2$) ppm.

$^{13}C\{^1H\}$-NMR (101 MHz, $CDCl_3$) δ = 151.5, 133.7, 129.6, 128.4, 125.5, 60.9, 36.1, 34.7, 25.6 ppm.

high resolution MS (ESI): calculated for $C_{13}H_{15}BrN_2O_2S$ ([M+Na]+): 364.9936

measured m/z 364.9926.

## Route D

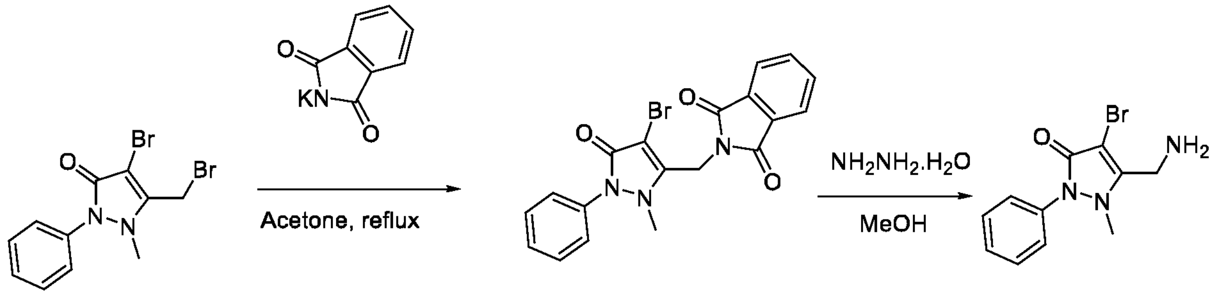


2 7 8

Example 5.36: 2-((4-bromo-2-methyl-5-oxo-1-phenyl-2,5-dihydro-1*H*-pyrazol-3-yl)methyl)isoindoline-1,3-dione, **7**

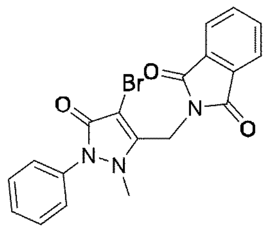

A solution of 4-bromo-5-(bromomethyl)-1-methyl-2-phenyl-1,2-dihydro-3H-pyrazol-3-one (467 mg, 1.35 mmol) and potassium 1,3-dioxoisoindolin-2-ide (250 mg, 1.35 mmol) in acetone (0.54 ml) was refluxed for 12 h. The solid was filtered off and the filterate concentrated under reduced pressure. The crude product was purified by column chromatograohy 60% ethyl acetate in petroleum ether to give 2-((4-bromo-2-methyl-5-oxo-1-phenyl-2,5-dihydro-1*H*-pyrazol-3-yl)methyl)isoindoline-1,3-dione as a colourless solid.
Yield: 13 % (70 mg)

Example 5.37: 5-(aminomethyl)-4-bromo-1-methyl-2-phenyl-1,2-dihydro-3*H*-pyrazol-3-one, **8**

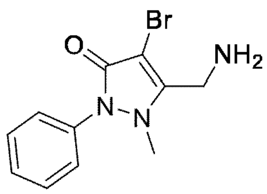

To a solution of 2-((4-bromo-2-methyl-5-oxo-1-phenyl-2,5-dihydro-1*H*-pyrazol-3-yl)methyl)isoindoline-1,3-dione (40 mg, 0.097 mmol) in methanol was added hydrazine hydrate (0.016 ml, 0.310 mmol) and the reaction mixture stirred for half an hour. 5 % HCl (1 ml) was added to the reaction mixture and the reaction stirred over night. The reaction mixture was purified by HPLC to give the product 5-(aminomethyl)-4-bromo-1-methyl-2-phenyl-1,2-dihydro-3*H*-pyrazol-3-one as a colorless TFA salt.

Yield 37 % (10 mg)

$^1$H-NMR (400 MHz, methanol-$d_4$): $\delta$ = 7.59 (t, 2H, *J* = 7.6 Hz, 2x CH), 7.50 (t, 1H, *J* = 7.4 Hz, CH), 7.44 (d, 2H, *J* = 7.6 Hz, 2x CH), 4.06 (s, 2H, $CH_2$), 3.32 (s, 3H, $CH_3$) ppm.

$^{13}C\{^{1}H\}$-NMR (101 MHz, methanol-$d_4$) $\delta$ 161.7, 152.1, 133.4, 129.3, 128.6, 126.0, 87.5, 34.8, 34.7 ppm.

high resolution MS (ESI): calculated for $C_{11}H_{12}BrN_3O$ ([M+H]+): m/z 282.0242
measured: m/z 282.0243.

Literature:

Fulmer *et al*.: Fulmer et al., Organometallics, 2010, 29, 2176-2179

**Example 6:**

*H. pylori* strains can be genetically and phenotypically diverse. The main clinical test strain *H. pylori* N6 is of European origin - originally isolated in France from a patient with stomach inflammation/gastritis - and has genetic and phenotypical differences to other globally collected clinical isolates, for example of African, South American or Asian origin (reference Olbermann et al.). In order to verify whether/demonstrate that the herein disclosed antimotilin compounds are also active (in terms of anti-motility/anti-flagellar activity) on another clinically relevant *H. pylori* strain originating from different ethnical background, the present inventors also assayed the identified primary active compounds Active 2 and Active 2a in *flaA*-luciferase reporter assays with a reporter strain derived from a second clinical isolate *H. pylori* strain, "L7" (generated as described for N6 in Example 1). This second clinical isolate L7 (reference Olbermann et al.), originates from Asia and is genetically very remote from strain N6. The flaA-luxAB luciferase reporter was newly engineered to express in strain L7 and assayed for luciferase inhibition as shown below.

Results of flaA-luc reporter strain of second clinical isolate of H. pylori (strain L7*)

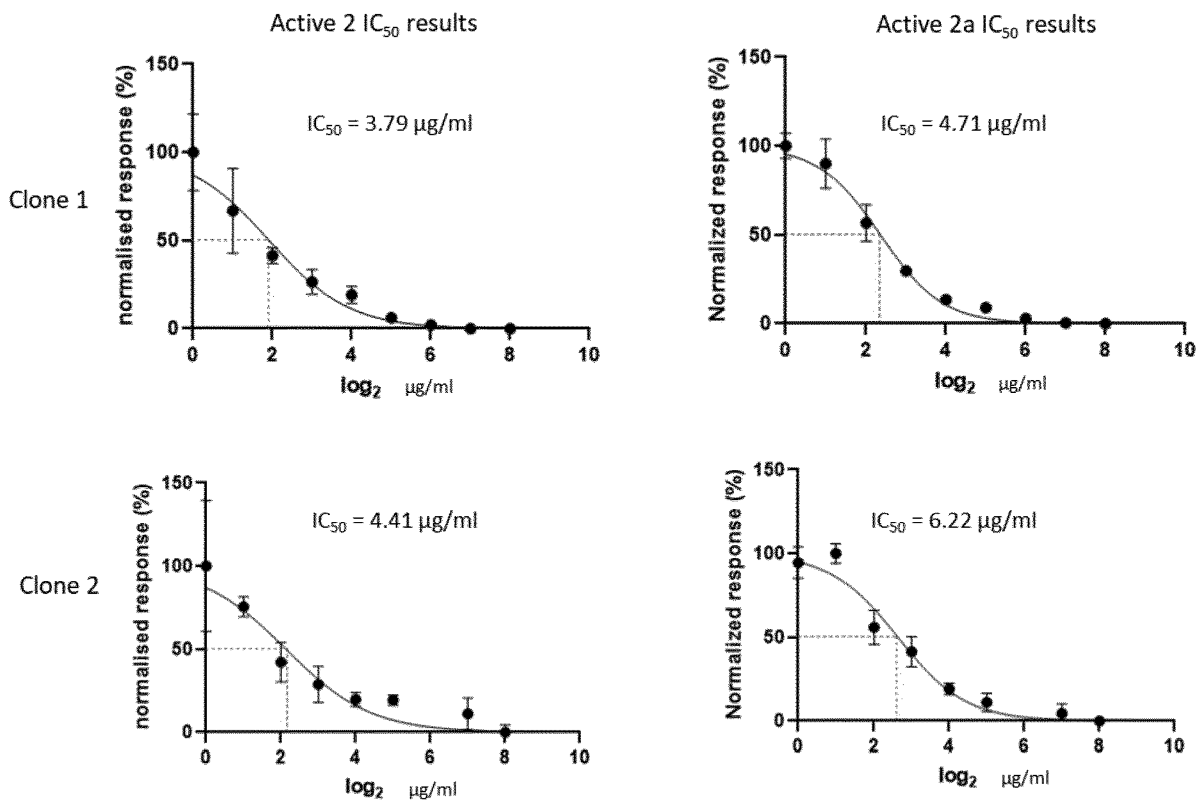

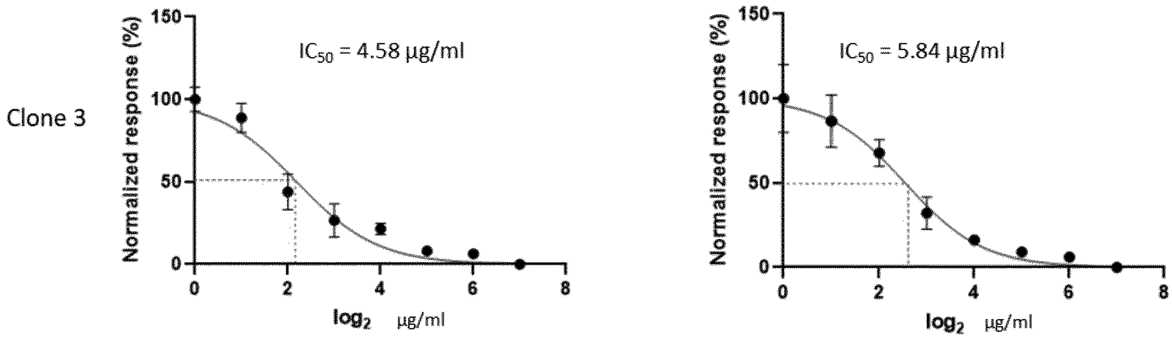


Shown are normalized results (highest respective luciferase value was set to 100%) of three clones of *H. pylori* strain L7 flaA-luxAB reporter strain; L7 reporter strain was newly generated accordingly as described for strain N6 (cf. Example 1). The assay method (Example 1) for luciferase determination was performed as described for reporter strain N6 (Example 1, Example 4). Three independently generated clones (clone 1, clone 2, clone 3) of the same reporter strain are shown for comparison.

The two compounds tested showed similar activities ($IC_{50}$ in comparable ranges) both, in the original reporter strain N6 flaA-luxAB and the second reporter strain L7 flaA-luxAB, thus confirming the general applicability of the invention for the species *H. pylori.*

*a second clinical strain, L7, of Asian origin, which was used to generate the second flaA reporter strain, is referenced in: Olbermann P, Josenhans C, Moodley Y, Uhr M, Stamer C, Vauterin M, Suerbaum S, Achtman M, Linz B., PLoS Genet. 2010;6(8):e1001069. doi: 10.1371/journal.pgen.1001069.

## Further References

Aebischer, T., Walduck, A., Schroeder, J., Wehrens, A., Chijioke, O., Schreiber, S., & Meyer, T. F. (2008). A vaccine against Helicobacter pylori: towards understanding the mechanism of protection. Int J Med Microbiol, 298(1-2), 161-168. doi:10.1016/j.ijmm.2007.07.009

Agudo, S., Alarcon, T., Cibrelus, L., Urruzuno, P., Martinez, M. J., & Lopez-Brea, M. (2009). [High percentage of clarithromycin and metronidazole resistance in Helicobacter pylori clinical isolates obtained from Spanish children]. Rev Esp Quimioter, 22(2), 88-92.

Allen, R. C., Popat, R., Diggle, S. P., & Brown, S. P. (2014). Targeting virulence: can we make evolution-proof drugs? Nat Rev Microbiol, 12(4), 300-308. doi:10.1038/nrmicro3232

Andermann, T. M., Chen, Y. T., & Ottemann, K. M. (2002). Two predicted chemoreceptors of Helicobacter pylori promote stomach infection. Infect Immun, 70(10), 5877-5881.

Angeli, A., Pinteala, M., Maier, S. S., Del Prete, S., Capasso, C., Simionescu, B. C., & Supuran, C. T. (2019). Inhibition of bacterial alpha-, beta- and gamma-class carbonic anhydrases with selenazoles incorporating benzenesulfonamide moieties. J Enzyme Inhib Med Chem, 34(1), 244-249. doi:10.1080/14756366.2018.1547287

Behrens, W., Schweinitzer, T., Bal, J., Dorsch, M., Bleich, A., Kops, F., ... Josenhans, C. (2013). Role of energy sensor TlpD of Helicobacter pylori in gerbil colonization and genome analyses after adaptation in the gerbil. Infect Immun, 81(10), 3534-3551. doi:10.1128/IAI.00750-13

Behrens, W., Schweinitzer, T., McMurry, J. L., Loewen, P. C., Buettner, F. F., Menz, S., & Josenhans, C. (2016). Localisation and protein-protein interactions of the Helicobacter pylori taxis sensor TlpD and their connection to metabolic functions. Sci Rep, 6, 23582. doi:10.1038/srep23582

Blaser, M. J. (2016). Antibiotic use and its consequences for the normal microbiome. Science, 352(6285), 544-545. doi:10.1126/science.aad9358

Chevance, F. F., & Hughes, K. T. (2008). Coordinating assembly of a bacterial macromolecular machine. Nat Rev Microbiol, 6(6), 455-465. doi:10.1038/nrmicro1887

Dethlefsen, L., Huse, S., Sogin, M. L., & Relman, D. A. (2008). The pervasive effects of an antibiotic on the human gut microbiota, as revealed by deep 16S rRNA sequencing. PLoS Biol, 6(11), e280. doi:10.1371/journal.pbio.0060280

Duck, W. M., Sobel, J., Pruckler, J. M., Song, Q., Swerdlow, D., Friedman, C., ... Gold, B. D. (2004). Antimicrobial resistance incidence and risk factors among Helicobacter pylori-infected persons, United States. Emerg Infect Dis, 10(6), 1088-1094. doi:10.3201/eid1006.030744

Eaton, K. A., Morgan, D. R., & Krakowka, S. (1992). Motility as a factor in the colonisation of gnotobiotic piglets by Helicobacter pylori. J Med Microbiol, 37(2), 123-127. doi:10.1099/00222615-37-2-123

Erhardt, M. (2016). Strategies to Block Bacterial Pathogenesis by Interference with Motility and Chemotaxis. Curr Top Microbiol Immunol, 398, 185-205. doi:10.1007/82_2016_493

Estibariz, I., Overmann, A., Ailloud, F., Krebes, J., Josenhans, C., & Suerbaum, S. (2019). The core genome m5C methyltransferase JHP1050 (M.Hpy99III) plays an important role in orchestrating gene expression in Helicobacter pylori. Nucleic Acids Res. doi:10.1093/nar/gky1307

Ferrero, R. L., Thiberge, J. M., Huerre, M., & Labigne, A. (1994). Recombinant antigens prepared from the urease subunits of Helicobacter spp.: evidence of protection in a mouse model of gastric infection. Infect Immun, 62(11), 4981-4989.

Gavrish, E., Shrestha, B., Chen, C., Lister, I., North, E. J., Yang, L., ... LaFleur, M. D. (2014). In vitro and in vivo activities of HPi1, a selective antimicrobial against Helicobacter pylori. Antimicrob Agents Chemother, 58(6), 3255-3260. doi:10.1128/AAC.02573-13

Guttner, Y., Windsor, H. M., Viiala, C. H., Dusci, L., & Marshall, B. J. (2003). Nitazoxanide in treatment of Helicobacter pylori: a clinical and in vitro study. Antimicrob Agents Chemother, 47(12), 3780-3783.

Herrera, V., & Parsonnet, J. (2009). Helicobacter pylori and gastric adenocarcinoma. Clin Microbiol Infect, 15(11), 971-976. doi:10.1111/j.1469-0691.2009.03031.x

Hsu, P. I., Pan, C. Y., Kao, J. Y., Tsay, F. W., Peng, N. J., Kao, S. S., ... Tsai, K. W. (2018). Helicobacter pylori eradication with bismuth quadruple therapy leads to dysbiosis of gut microbiota with an increased relative abundance of Proteobacteria and decreased relative abundances of Bacteroidetes and Actinobacteria. Helicobacter, 23(4), e12498. doi:10.1111/hel.12498

Johnson, J. G., Yuhas, C., McQuade, T. J., Larsen, M. J., & DiRita, V. J. (2015). Narrow-spectrum inhibitors of Campylobacter jejuni flagellar expression and growth. Antimicrob Agents Chemother, 59(7), 3880-3886. doi:10.1128/AAC.04926-14

Josenhans, C., Niehus, E., Amersbach, S., Horster, A., Betz, C., Drescher, B., ... Suerbaum, S. (2002). Functional characterization of the antagonistic flagellar late regulators FliA and FlgM of Helicobacter pylori and their effects on the H. pylori transcriptome. Mol Microbiol, 43(2), 307-322.

Josenhans, C., & Suerbaum, S. (2002). The role of motility as a virulence factor in bacteria. Int J Med Microbiol, 291(8), 605-614. doi:10.1078/1438-4221-00173

Kavermann, H., Burns, B. P., Angermuller, K., Odenbneit, S., Fischer, W., Melchers, K., & Haas, R. (2003). Identification and characterization of Helicobacter pylori genes essential for gastric colonization. J Exp Med, 197(7), 813-822. doi:10.1084/jem.20021531

Kennedy, A. J., Bruce, A. M., Gineste, C., Ballard, T. E., Olekhnovich, I. N., Macdonald, T. L., & Hoffman, P. S. (2016). Synthesis and Antimicrobial Evaluation of Amixicile-Based Inhibitors of the Pyruvate-Ferredoxin Oxidoreductases of Anaerobic Bacteria and Epsilonproteobacteria. Antimicrob Agents Chemother, 60(7), 3980-3987.

doi:10.1128/AAC.00670-16

Kim, S. E., Roh, J. H., Park, M. I., Park, S. J., Moon, W., Kim, J. H., ... Heo, J. J. (2019). Effect of 7-day Bismuth Quadruple Therapy versus 14-day Moxifloxacin Triple Therapy for Second-line Helicobacter pylori Eradication Therapy. Korean J Gastroenterol, 73(1), 26-34. doi: 10.4166/kjg .2019.73.1.26

Ko, S. W., Kim, Y. J., Chung, W. C., & Lee, S. J. (2019). Bismuth supplements as the first-line regimen for Helicobacter pylori eradication therapy: Systemic review and meta-analysis. Helicobacter, e12565. doi:10.1111/hel.12565

Lee, C. W., Rickman, B., Rogers, A. B., Ge, Z., Wang, T. C., & Fox, J. G. (2008). Helicobacter pylori eradication prevents progression of gastric cancer in hypergastrinemic INS-GAS mice. Cancer Res, 68(9), 3540-3548. doi:10.1158/0008-5472.CAN-07-6786

Lertsethtakarn, P., Ottemann, K. M., & Hendrixson, D. R. (2011). Motility and chemotaxis in Campylobacter and Helicobacter. Annu Rev Microbiol, 65, 389-410. doi:10.1146/annurev-micro-090110-102908

Liu, Q., Shi, W. K., Ren, S. Z., Ni, W. W., Li, W. Y., Chen, H. M., ... Zhu, H. L. (2018). Arylamino containing hydroxamic acids as potent urease inhibitors for the treatment of Helicobacter pylori infection. Eur J Med Chem, 156, 126-136. doi:10.1016/j.ejmech.2018.06.065

Lobo, A. J., McNulty, C. A., Uff, J. S., Dent, J., Eyre-Brook, I. A., & Wilkinson, S. P. (1994). Preservation of gastric antral mucus is associated with failure of eradication of Helicobacter pylori by bismuth, metronidazole and tetracycline. Aliment Pharmacol Ther, 8(2), 181-185.

Luther, J., Higgins, P. D., Schoenfeld, P. S., Moayyedi, P., Vakil, N., & Chey, W. D. (2010). Empiric quadruple vs. triple therapy for primary treatment of Helicobacter pylori infection: Systematic review and meta-analysis of efficacy and tolerability. Am J Gastroenterol, 105(1), 65-73. doi:10.1038/ajg.2009.508

Malfertheiner, P., Megraud, F., O'Morain, C. A., Gisbert, J. P., Kuipers, E. J., Axon, A. T., ... Consensus, p. (2017). Management of Helicobacter pylori infection-the Maastricht V/Florence Consensus Report. Gut, 66(1), 6-30. doi:10.1136/gutjnl-2016-312288

Malfertheiner, P., Selgrad, M., Wex, T., Romi, B., Borgogni, E., Spensieri, F., ... Del Giudice, G. (2018). Efficacy, immunogenicity, and safety of a parenteral vaccine against Helicobacter pylori in healthy volunteers challenged with a Cag-positive strain: a randomised, placebo-controlled phase 1/2 study. Lancet Gastroenterol Hepatol, 3(10), 698-707. doi:10.1016/S2468-1253(18)30125-0

McGee, D. J., Langford, M. L., Watson, E. L., Carter, J. E., Chen, Y. T., & Ottemann, K. M. (2005). Colonization and inflammation deficiencies in Mongolian gerbils infected by Helicobacter pylori chemotaxis mutants. Infect Immun, 73(3), 1820-1827. doi:10.1128/IAI.73.3.1820-1827.2005

Menard, R., Schoenhofen, I. C., Tao, L., Aubry, A., Bouchard, P., Reid, C. W., ... Logan, S. M. (2014). Small-molecule inhibitors of the pseudaminic acid biosynthetic pathway: targeting motility as a key bacterial virulence factor. Antimicrob Agents Chemother, 58(12), 7430-7440. doi:10.1128/aac.03858-14

Niehus, E., Gressmann, H., Ye, F., Schlapbach, R., Dehio, M., Dehio, C., ... Josenhans, C. (2004). Genome-wide analysis of transcriptional hierarchy and feedback regulation in the flagellar system of Helicobacter pylori. Mol Microbiol, 52(4), 947-961. doi:10.1111/j.1365-2958.2004.04006.x

Niehus, E., Ye, F., Suerbaum, S., & Josenhans, C. (2002). Growth phase-dependent and differential transcriptional control of flagellar genes in Helicobacter pylori. Microbiology, 148(Pt 12), 3827-3837. doi:10.1099/00221287-148-12-3827

O'Morain, C., Borody, T., Farley, A., De Boer, W. A., Dallaire, C., Schuman, R., ... International multicentre, s. (2003). Efficacy and safety of single-triple capsules of bismuth biskalcitrate, metronidazole and tetracycline, given with omeprazole, for the eradication of Helicobacter pylori: an international multicentre study. Aliment Pharmacol Ther, 17(3), 415-420.

O'Morain, N. R., Dore, M. P., O'Connor, A. J. P., Gisbert, J. P., & O'Morain, C. A. (2018). Treatment of Helicobacter pylori infection in 2018. Helicobacter, 23 Suppl 1, e12519. doi:10.1111/hel.12519

Pilotto, A., Franceschi, M., Rassu, M., Leandro, G., Bozzola, L., Furlan, F., & Di Mario, F. (2000). Incidence of secondary Helicobacter pylori resistance to antibiotics in treatment failures after 1-week proton pump inhibitor-based triple therapies: a prospective study. Dig Liver Dis, 32(8), 667-672.

Rasko, D. A., & Sperandio, V. (2010). Anti-virulence strategies to combat bacteria-mediated disease. Nat Rev Drug Discov, 9(2), 117-128. doi:10.1038/nrd3013

Rust, M., Borchert, S., Niehus, E., Kuehne, S. A., Gripp, E., Bajceta, A., Josenhans, C. (2009). The Helicobacter pylori anti-sigma factor FlgM is predominantly cytoplasmic and cooperates with the flagellar basal body protein FlhA. J Bacteriol, 191(15), 4824-4834. doi:10.1128/JB.00018-09

Salama, N. R., Shepherd, B., & Falkow, S. (2004). Global transposon mutagenesis and essential gene analysis of Helicobacter pylori. J Bacteriol, 186(23), 7926-7935. doi:10.1128/JB.186.23.7926-7935.2004

Schreiber, S., Konradt, M., Groll, C., Scheid, P., Hanauer, G., Werling, H. O., ... Suerbaum, S. (2004). The spatial orientation of Helicobacter pylori in the gastric mucus. Proc Natl Acad Sci U S A, 101(14), 5024-5029. doi:10.1073/pnas.0308386101

Schweinitzer, T., Mizote, T., Ishikawa, N., Dudnik, A., Inatsu, S., Schreiber, S., ... Josenhans, C. (2008). Functional characterization and mutagenesis of the proposed behavioral sensor TlpD of Helicobacter pylori. J Bacteriol, 190(9), 3244-3255. doi:10.1128/JB.01940-07

Sharma, C. M., Hoffmann, S., Darfeuille, F., Reignier, J., Findeiss, S., Sittka, A., ... Vogel, J. (2010). The primary transcriptome of the major human pathogen Helicobacter pylori. Nature, 464(7286), 250-255. doi:10.1038/nature08756

Srikhanta, Y. N., Gorrell, R. J., Power, P. M., Tsyganov, K., Boitano, M., Clark, T. A., ... Kwok, T. (2017). Methylomic and phenotypic analysis of the ModH5 phasevarion of Helicobacter pylori. Sci Rep, 7(1), 16140. doi:10.1038/s41598-017-15721-x

Suerbaum, S., & Michetti, P. (2002). Helicobacter pylori infection. N Engl J Med, 347(15), 1175-1186. doi:10.1056/NEJMra020542

Theuretzbacher U., Piddock L.J.V.. (2019) Non-traditional Antibacterial TherapeuticOptions and Challenges. Cell Host Microbe 26(1):61-72. doi:10.1016/j.chom.2019.06.004.

Totsika, M. (2017). Disarming pathogens: benefits and challenges of antimicrobials that target bacterial virulence instead of growth and viability. Future Med Chem, 9(3), 267-269. doi:10.4155/fmc-2016-0227

van Zanten, S. J., Kolesnikow, T., Leung, V., O'Rourke, J. L., & Lee, A. (2003). Gastric transitional zones, areas where Helicobacter treatment fails: results of a treatment trial using the Sydney strain mouse model. Antimicrob Agents Chemother, 47(7), 2249-2255.

Xia, H. X., Talley, N. J., Keane, C. T., & O'Morain, C. A. (1997). Recurrence of Helicobacter pylori infection after successful eradication: nature and possible causes. Dig Dis Sci, 42(9), 1821-1834.

Yang, I., Woltemate, S., Piazuelo, M. B., Bravo, L. E., Yepez, M. C., Romero-Gallo, J., ... Suerbaum, S. (2016). Different gastric microbiota compositions in two human populations with high and low gastric cancer risk in Colombia. Sci Rep, 6, 18594. doi:10.1038/srep18594

Ye, F., Brauer, T., Niehus, E., Drlica, K., Josenhans, C., & Suerbaum, S. (2007). Flagellar and global gene regulation in Helicobacter pylori modulated by changes in DNA supercoiling. Int J Med Microbiol, 297(2), 65-81. doi:10.1016/j.ijmm.2006.11.006

Zeng, M., Mao, X. H., Li, J. X., Tong, W. D., Wang, B., Zhang, Y. J., ... Zou, Q. M. (2015). Efficacy, safety, and

immunogenicity of an oral recombinant Helicobacter pylori vaccine in children in China: a randomised, double-blind, placebo-controlled, phase 3 trial. Lancet, 386(10002), 1457-1464. doi:10.1016/S0140-6736(15)60310-5

Zhang, S., Lee, D. S., Morrissey, R., Aponte-Pieras, J. R., Rogers, A. B., & Moss, S. F. (2015). Early or late antibiotic intervention prevents Helicobacter pylori-induced gastric cancer in a mouse model. Cancer Lett, 359(2), 345-351.

Citation of the above patents, patent applications, publications and documents is not an admission that any of the foregoing is pertinent prior art, nor does it constitute any admission as to the contents or date of these publications or documents. Their citation is not an indication of a search for relevant disclosures. All statements regarding the date(s) or contents of the documents is based on available information and is not an admission as to their accuracy or correctness.

The term "a" or "an" can refer to one of or a plurality of the elements it modifies (e.g., "a carrier" can mean one or more carriers) unless it is contextually clear either one of the elements or more than one of the elements is described. The term "about" as used herein refers to a value within 10% of the underlying parameter (i.e., plus or minus 10%), and use of the term "about" at the beginning of a string of values modifies each of the values (i.e., "about 1, 2 and 3" refers to about 1, about 2 and about 3). For example, a weight of "about 100 grams" can include weights between 90 grams and 110 grams. Further, when a listing of values is described herein (e.g., about 50%, 60%, 70%, 80%, 85% or 86%) the listing includes all intermediate and fractional values thereof (e.g., 54%, 85.4%).

Certain embodiments of the technology are set forth in the claim(s) that follow(s)

Table 1

**Table 1:** comparative antibacterial activity of preselected panel of primary actives (A1-A15) discovered in *H. pylori* anti-motility screen against two different *H. pylori* strains, N6 and P12. Secondary tests included $IC_{50}$ of antimotility assay and $IC_{50}$ of vitality testing (antirespiratory), and MIC/MBC. For the second strain, only MIC/MBC were performed, since all except one preselected hit compound exhibited killing activity against *H. pylori.* The determined activities were similar in the two strains.

***H. pylori* N6**

| Compound | Luminescence | $IC_{50}$ luc | $IC_{50}$ vit | MIC | MBC |
|---|---|---|---|---|---|
| A1 | 18 % | 0,34 µg/ml, 1,1 µM | 0,03 µg/ml, 0,09 µM | 1 µg/ml, 3,2 µM | 4 µg/ml, 12,6 µM |
| A2 | 5 % | 0,87 µg/ml, 1,6 µM / 0,9 µg/ml, 1,7 µM | 0,58 µg/ml, 1,1 µM | 1 µg/ml, 1,9 µM | >8 µg/ml, 15,2 µM ? |
| A3 | 20 % | 2,13 µg/ml, 3,1 µM / 2, 7 µg/ml, 3,9 µM | 0,13 µg/ml, 0,19 µM / 0,16 µg/ml, 0,23 µM | 2 µg/ml, 2,9 µM | 4 µg/ml, 5,8 µM |
| A4 | 12 % | 3,8 µg/ml, 11,5 µM /4 µg/ml, 12,1 µM | 5,4 µg/ml, 16,3 µM | 0,5 µg/ml, 1,5 µM | 4 µg/ml, 12,1 µM |
| A5 | 15 % | 2,6 µg/ml, 9,5 µM | 2,9 µg/ml, 10,6 µM | 4 µg/ml, 14,6 µM | 4 µg/ml, 14,6 µM |
| A6 | 9 % | 3,7 µg/ml, 16,8 µM | precipitate | 8 µg/ml, 36,3 µM | >64 µg/ml ? |
| A7 | 9 % | 0,04 µg/ml, 0,076 µM | 0,01 µg/ml, 0,02 µM | 0,25 µg/ml, 0,5 µM | 0,5 µg/ml, 1 µM |
| A8 | 1 % | 0,46 µg/ml, 1,48 µM | 0,18 µg/ml, 0,58 µM | 0,5 µg/ml, 1,6 µM | 1 µg/ml, 3,2 µM |
| A9 | 7 % | 0,27 µg/ml, 1,11 µM | 0,1 µg/ml, 0,4 µM | 0,25 µg/ml, 1 µM | ≥ 1 µg/ml, 4,1 µM |
| A10 | 3 % | not applicable | 39,8 µg/ml, 94,7 µM | 256 µg/ml, 609 µM | > 256 µg/ ml ? |
| A11 | 1 % | 0,22 µg/ml, 0,72 µM | 6,13 µg/ml, 20,1 µM | 4 µg/ml, 13,1 µM | 16 µg/ml, 52,6 µM |
| A12 | 1% | 0,7 µg/ml, 2,8 µM / 0,9 µg/ml, 3,6 µM | 0,48 µg/ml, 1,9 µM | 0,25 µg/ml, 1 µM | 0,5 µg/ml, 2 µM |

(continued)

**Table 1:** comparative antibacterial activity of preselected panel of primary actives (A1-A15) discovered in *H. pylori* anti-motility screen against two different *H. pylori* strains, N6 and P12. Secondary tests included $IC_{50}$ of antimotility assay and $IC_{50}$ of vitality testing (antirespiratory), and MIC/MBC. For the second strain, only MIC/MBC were performed, since all except one preselected hit compound exhibited killing activity against *H. pylori.* The determined activities were similar in the two strains.

***H. pylori* N6**

| Compound | Luminescence | $IC_{50}$ luc | $IC_{50}$ vit | MIC | MBC |
|---|---|---|---|---|---|
| A13 | 22% | 0,2 μg/ml, 0,55 μM | 1,76 μg/ml, 4,8 μM | 2 μg/ml, 5,5 μM | >32 μg/ml, 88,1 μM ? |
| A14 | 19% | 2,28 μg/ml, 5,9 μM | 6,97 μg/ml, 18,2 μM | 8 μg/ml, 20,8 μM | 16 μg/ml, 41,7 μM |
| A15 | 19% | 0,04 μg/ml, 0,14 μM | 0,01 μg/ml, 0,04 μM | 0,125 μg/ml, 0,4 μM | 4 μg/ml, 14,2 μM |

***Table 1 continued***

| ***H. pylori* P12** | | |
|---|---|---|
| Compound | MIC | MBC |
| A1 | 2 μg/ml, 6,3 μM | 8 μg/ml, 25,3 μM |
| A2 | 1 μg/ml, 1,9 μM | > 256 μg/ml, 485,2 μM |
| A3 | 4 μg/ml, 5,8 μM | 16 μg/ml, 23,3 μM |
| A4 | 0,125 μg/ml, 0,4 μM | 8 μg/ml, 24,1 μM |
| A5 | 4 μg/ml, 14,6 μM | 8 μg/ml, 29,2 μM |
| A6 | 8 μg/ml, 36,3 μM | > 256 μg/ml, 1162.4 μM |
| A7 | 1 μg/ml, 1,9 μM | 4 μg/ml, 7,6 μM |
| A8 | 0,5 μg/ml, 1,6 μM | 1 μg/ml, 3,2 μM |
| A9 | 0,125 μg/ml, 0,5 μM | 0,25 μg/ml, 1 μM |
| A10 | 64 μg/ml, 152,2 μM | 256 μg/ml, 609 μM |
| A11 | 16 μg/ml, 52,6 μM | 32 μg/ml, 105.1 μM |
| A12 | 0,125 μg/ml, 0,5 μM | 1 μg/ml, 4 μM |
| A13 | 4 μg/ml, 11 μM | > 256 μg/ml, 704,7 μM |
| A14 | 2 μg/ml, 5,2 μM without DMSO: 8 μg/ml, 20,8 μM | 4 μg/ml, 10,4 μM without DMSO : 16 μg/ml, 41,7 |
| A15 | 0,125 μg/ml, 0,4 μM | > 0,5 μg/ml, 1,8 μM |

Table 2

| **Table 2: Specific activity against *H. pylori.*** comparative antibacterial activity testing (MIC/MBC) of preselected panel of primary actives (A1-A15) against two different intestinal bacterial species, *Campylobacter jejuni* (strain 11168) and *Escherichia coli* (strain RP437). Some weak antibacterial activity of various primary actives was detected against *C. jejuni,* while no antibacterial activity was found against E. *coli.* Since *C. jejuni* is exquisitely sensitive against the solvent DMSO, selected assays were performed in the absence of DMSO.<br>***C. jejuni* 11168** | | |
|---|---|---|
| Compound | MIC | MBC |
| A1 | > 256 μg/ml, 808,9 μM | > 256 μg/ml, 808,9 μM |
| A2 | > 256 μg/ml, 485,2 μM | > 256 μg/ml, 485,2 μM |
| A3 | 4 μg/ml, 5,8 μM | 8 μg/ml, 11,6 μM |
| A4 | 32 μg/ml, 96,4 μM | 128 μg/ml, 385,8 μM |
| A5 | 16 μg/ml, 58,4 μM | 16 μg/ml, 58,4 μM |
| A6 | > 256 μg/ml, > 1162,4 μM | > 256 μg/ml, > 1162,4 μM |
| A7 | > 256 μg/ml, 488,9 μM | > 256 μg/ml, 488,9 μM |
| A8 | 8 μg/ml, 25,8 μM | 16 μg/ml, 51,5 μM |
| A9 | 4 μg/ml, 16,5 μM | 32 μg/ml, 132,1 μM |
| A10 | 256 μg/ml, 609 μM | > 256 μg/ml, 609 μM |
| A11 | 128 μg/ml, 420,6 μM | 128 μg/ml, 420,6 μM |
| A12 | 4 μg/ml, 16 μM | 8 μg/ml, 16 μM |
| A13 | > 256 μg/ml, 704,7 μM | > 256 μg/ml, 704,7 μM |
| A14 | 4 μg/ml, 10,4 μM without DMSO:16 μg/ml, 41,7 μM | 8 / 32 μg/ml, 20,8 / 83,4 μM ? without DMSO: 128 μg/ml, 333,5 μM |
| A15 | > 128 μg/ml, 453,3 μM | > 128 μg/ml, 453,3 μM |

***E. coli* RP437**

| Compound | MIC | MBC |
|---|---|---|
| A1 | > 256 μg/ml, 808,9 μM | > 256 μg/ml, 808,9 μM |
| A2 | > 256 μg/ml, 485,2 μM | > 256 μg/ml, 485,2 μM |
| A3 | 32 μg/ml, 46,5 μM | > 256 μg/ml, μM |
| A4 | > 256 μg/ml, 771,6 μM | > 256 μg/ml, 771,6 μM |
| A5 | 32 μg/ml, 116,7 μM | 64 μg/ml, 233,4 μM |
| A6 | > 256 μg/ml, 1162,4 μM | > 256 μg/ml, 1162,4 μM |
| A7 | > 256 μg/ml, 488,9 μM | > 256 μg/ml, 488,9 μM |
| A8 | > 256 μg/ml, 824,8 μM | > 256 μg/ml, 824,8 μM |
| A9 | > 256 μg/ml, 1056,7 μM | > 256 μg/ml, 1056,7 μM |
| A10 | > 256 μg/ml, 609 μM | > 256 μg/ml, 609 μM |
| A11 | > 256 μg/ml, 841,2 μM | > 256 μg/ml, 841,2 μM |
| A12 | > 256 μg/ml, 1026,8 μM | > 256 μg/ml, 1026,8 μM |

(continued)

| Compound | MIC | MBC |
|---|---|---|
| A13 | > 256 μg/ml, 704,7 μM | > 256 μg/ml, 704,7 μM |
| A14 | > 256 μg/ml, 667 μM | > 256 μg/ml, 667 μM |
| A15 | > 256 μg/ml, 906,6 μM | > 256 μg/ml, 906,6 μM |

**Table 3**

**Table 3:** Results for *H. pylori* screens (luciferase and metabolic) of analogous compounds Active 2 (BL2) and Active 2a (BL2a); $IC_{50}$ of luciferase flagellar reporter assay for Active 2 and Active 2a. > 100% values for metabolic assay indicate no inhibition by the compound on the central bacterial metabolism. This corresponds to MIC values of >256, no growth inhibition by compound (see main text).

| ID | Luciferase assay (% of control) | Metabolic assay (% of control) | IC50 (luciferase assay) |
|---|---|---|---|
| Active 2a | 1,4 | 107,7 | 3,05 μg/ml, 10,3 μM |
| Active 2 | 2,6 | 102,2 | 2,74 μg/ml, 8 μM |

# Table 4

**Table 4:** mouse group order for proof-of-principle treatment experiment in chronic *H. pylori* mouse model for small compound BL2 (Active 2)

| Mouse group | Number of animals (n) | HP-infected (Y/-) | Metronidazole (Y/-) | Active 2 (Y/-) | Not-treated (Nt) |
|---|---|---|---|---|---|
| 1 | 10 | Y | - | - | Nt |
| 2 | 10 | Y | Y | Y | |
| 3 | 10 | Y | Y | - | |
| 4 | 10 | Y | - | Y | |
| 5 | 8 | - | - | - | Nt |
| 6 | 8 | - | - | Y | |
| | | | | | |

# Table 5

Table 5: AMOVA (Bray-Curtis) of microbiota analyses of Active 2-treated mice confirm no significant difference of microbiota

| [illegible] | Among | Within | Total |
|---|---|---|---|
| SS | [illegible] | [illegible] | [illegible] |
| df | [illegible] | [illegible] | [illegible] |
| MS | [illegible] | [illegible] | |
| Fs | [illegible] | | |
| p-value: [illegible] | | | |
| [illegible] | Among | Within | Total |
| SS | [illegible] | [illegible] | [illegible] |
| df | [illegible] | [illegible] | [illegible] |
| MS | [illegible] | [illegible] | |
| Fs | [illegible] | | |
| p-value: [illegible] | | | |
| [illegible] | Among | Within | Total |
| SS | [illegible] | [illegible] | [illegible] |
| df | [illegible] | [illegible] | [illegible] |
| MS | [illegible] | [illegible] | |
| Fs | [illegible] | | |
| p-value: [illegible] | | | |
| [illegible] | Among | Within | Total |
| SS | [illegible] | [illegible] | [illegible] |
| df | [illegible] | [illegible] | [illegible] |
| MS | [illegible] | [illegible] | |
| Fs | [illegible] | | |
| p-value: [illegible] | | | |
| [illegible] | Among | Within | Total |
| SS | [illegible] | [illegible] | [illegible] |
| df | [illegible] | [illegible] | [illegible] |
| MS | [illegible] | [illegible] | |
| Fs | [illegible] | | |
| p-value: [illegible] | | | |
| [illegible] | Among | Within | Total |
| SS | [illegible] | [illegible] | [illegible] |
| df | [illegible] | [illegible] | [illegible] |
| MS | [illegible] | [illegible] | |
| Fs | [illegible] | | |
| p-value: [illegible] | | | |
| [illegible] | Among | Within | Total |
| SS | [illegible] | [illegible] | [illegible] |
| df | [illegible] | [illegible] | [illegible] |
| MS | [illegible] | [illegible] | |
| Fs | [illegible] | | |
| p-value: [illegible] | | | |
| [illegible] | Among | Within | Total |
| SS | [illegible] | [illegible] | [illegible] |
| df | [illegible] | [illegible] | [illegible] |
| MS | [illegible] | [illegible] | |
| Fs | [illegible] | | |
| p-value: [illegible] | | | |
| [illegible] | Among | Within | Total |
| SS | [illegible] | [illegible] | [illegible] |
| df | [illegible] | [illegible] | [illegible] |
| MS | [illegible] | [illegible] | |
| Fs | [illegible] | | |
| p-value: [illegible] | | | |
| [illegible] | Among | Within | Total |
| SS | [illegible] | [illegible] | [illegible] |
| df | [illegible] | [illegible] | [illegible] |
| MS | [illegible] | [illegible] | |
| Fs | [illegible] | | |
| p-value: [illegible] | | | |
| [illegible] | Among | Within | Total |
| SS | [illegible] | [illegible] | [illegible] |
| df | [illegible] | [illegible] | [illegible] |
| MS | [illegible] | [illegible] | |
| Fs | [illegible] | | |
| p-value: [illegible] | | | |
| [illegible] | Among | Within | Total |
| SS | [illegible] | [illegible] | [illegible] |
| df | [illegible] | [illegible] | [illegible] |
| MS | [illegible] | [illegible] | |
| Fs | [illegible] | | |
| p-value: [illegible] | | | |
| [illegible] | Among | Within | Total |
| SS | [illegible] | [illegible] | [illegible] |
| df | [illegible] | [illegible] | [illegible] |
| MS | [illegible] | [illegible] | |
| Fs | [illegible] | | |
| p-value: [illegible] | | | |
| [illegible] | Among | Within | Total |
| SS | [illegible] | [illegible] | [illegible] |
| df | [illegible] | [illegible] | [illegible] |
| MS | [illegible] | [illegible] | |
| Fs | [illegible] | | |
| p-value: [illegible] | | | |
| [illegible] | Among | Within | Total |
| SS | [illegible] | [illegible] | [illegible] |
| df | [illegible] | [illegible] | [illegible] |
| MS | [illegible] | [illegible] | |
| Fs | [illegible] | | |
| p-value: [illegible] | | | |
| [illegible] | Among | Within | Total |
| SS | [illegible] | [illegible] | [illegible] |
| df | [illegible] | [illegible] | [illegible] |
| MS | [illegible] | [illegible] | |
| Fs | [illegible] | | |
| p-value: [illegible] | | | |

## Claims

**1.** A compound of formula (I),

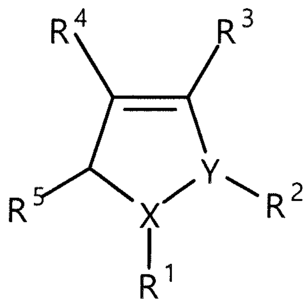

(I)

for use in

(A) treating and/or preventing a bacterial infection, wherein the bacterial infection is caused by flagellated bacteria selected from *Helicobacter spp.* and *Campylobacter spp.*;
(B) treating a condition associated with the bacterial infection as defined in (A), wherein the condition is selected from peptic ulcer, gastritis, duodenitis, gastric mucosaassociated lymphoid tissue (MALT) lymphoma, enteritis, and enterocolitis; or
(C) preventing a condition associated with the bacterial infection as defined in (A), wherein the condition is selected from peptic ulcer, gastritis, duodenitis, gastric (stomach) cancer, gastric MALT lymphoma, enteritis, and enterocolitis; and
wherein in (A) to (C) a therapeutically effective amount of the compound is to be administered to a subject in need thereof, wherein the subject is a mammal, preferably a human, or other animal; and
wherein:

(a) X and Y of formula (I) are independently selected from N, O and S;
(b) $R^1$ is:

(b-1) phenyl, optionally substituted by one or more:

(i) $C_{1-3}$ alkyl or isopropyl; and/or
(ii) X or $CX_3$ (X is selected from Cl, Br, I and F); or

(b-2) biphenyl;

(c) $R^2$ is $CX_3$ or $CH_2CX_3$, where X is selected from H, F, Cl, Br and I;
(d) $R^3$ is $CH_2SX$, where X is H or $C(NH)NH_2$;
(e) $R^4$ is selected from F, Cl, Br and I; and
(f) $R^5$ is O; and

wherein the compound of formula (I) has an anti-motility activity on the bacteria.

**2.** The compound for use according to claim 1, wherein the compound is of formula (II):

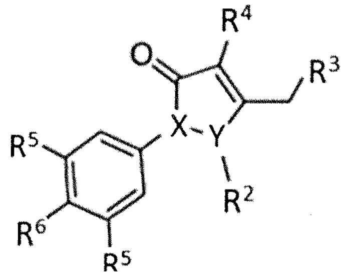

(II),

wherein

(a) X and Y of formula (II) are each independently selected from N, O and S;
(b) $R^2$ is $CH_3$ or $CH_2CH_3$;
(c) $R^3$ is SX, where X is H or

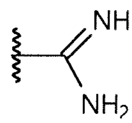

;

and
(d) $R^4$ is selected from F, Cl, Br and I; and
(e) $R^5$ and $R^6$ are each independently selected from X or $CX_3$ (X is selected from H, F, Cl, Br and I), ethyl, isopropyl or phenyl.

**3.** The compound for use according to claim 2, wherein

(a) X and Y of formula (II) are each independently selected from N, O and S;
(b) $R^2$ is $CH_3$ or $CH_2CH_3$;
(c) $R^3$ is SX, where X is H or

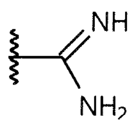

;

and
(d) $R^4$ is selected from F, Cl, Br and I;
(e) $R^5$ is H or $CH_3$; and
(f) $R^6$ is X or $CX_3$ (X is selected from H, F, Cl, Br and I), ethyl, isopropyl or phenyl.

**4.** The compound for use according to any one of claims 1 to 3, wherein the compound is of formula (III):

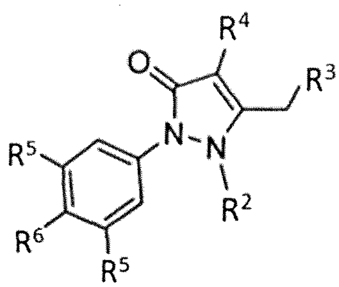

(III),

(a) $R^2$ is $CH_3$ or $CH_2CH_3$;

(b) $R^3$ is SX, where X is H or

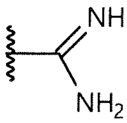 ;

and

(c) $R^4$ is selected from F, Cl, Br and I;

(d) $R^5$ is H or $CH_3$; and

(e) $R^6$ is H, X or $CX_3$ (where X is H, F, Cl, Br or I), ethyl, isopropyl or phenyl.

**5.** The compound for use according to claim 4, wherein

(a) $R^2$ is $CH_2CH_3$ and $R^3$ is SH; or

(b) $R^2$ is $CH_3$ and $R^6$ is H, X (where X is F, Cl, Br or I), $CH_3$, ethyl, isopropyl or phenyl.

**6.** The compound for use according to claim 4 or 5, wherein

(a) $R^2$ is $CH_2CH_3$, $R^3$ is SH, and $R^5$ and $R^6$ are each H; or

(b) $R^2$ is $CH_3$, $R^3$ is SH, and $R^5$ and $R^6$ are each H.

**7.** The compound for use according to claim 1, wherein the compound is selected from:

(a)

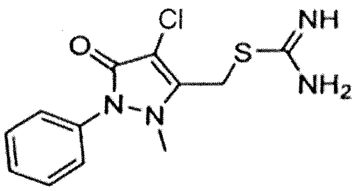

(b)

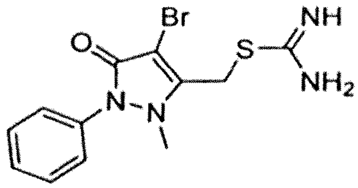

(c)

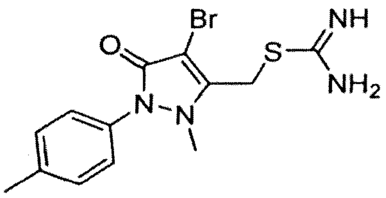

(d)

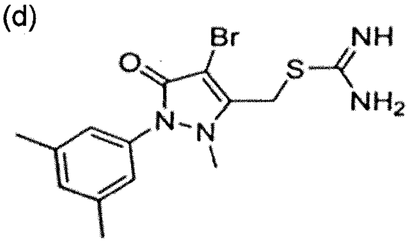

(e)

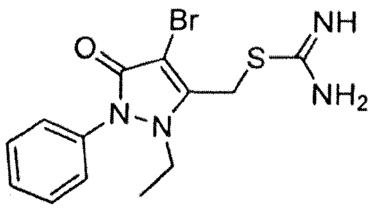

(f)

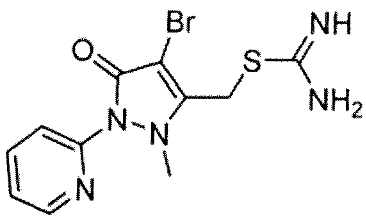

**8.** The compound for use according to claim 1, wherein the compound is selected from:

(a) 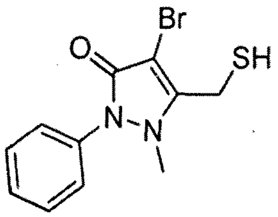

(b) 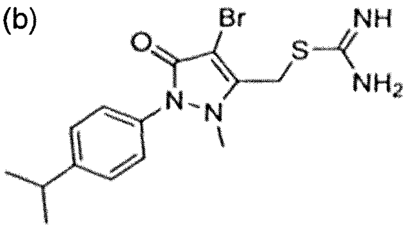

(c) 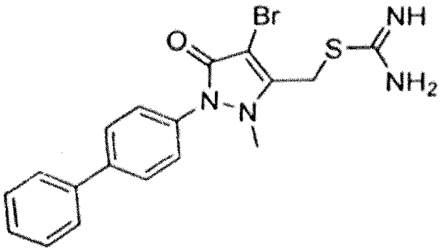

(d) 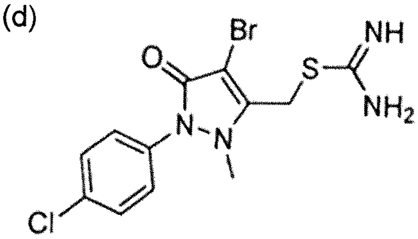

(e) 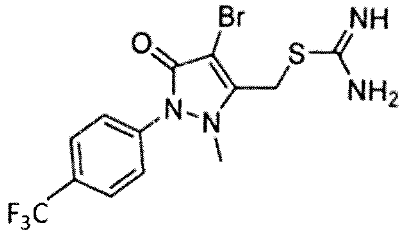

(f) 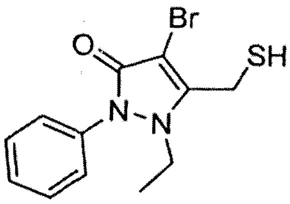

**9.** The compound for use according to any one of claims 1 to 8, wherein the *Helicobacter spp.* and/or the *Campylobacter spp.* are

(a) gastro-intestinal pathogenic bacteria;
(b) gastro-duodenal pathogenic bacteria;
(c) urogenital pathogenic bacteria;
(d) not susceptible to antibiotic treatment;
(e) *Helicobacter pylori;* and/or
(f) *Campylobacter jejuni* or *Campylobacter coli.*

**10.** The compound for use according to any one of claims 1 to 9, wherein the compound is to be administered in combination, or sequentially, with one or more further agents selected from:

(a) antibiotic, preferably selected from amoxicillin, penicillin, clarithromycin, metronidazole, tetracycline, tinidazole, rifampin, rifabutin, ciprofloxacin, gemifloxacin, levofloxacin, moxifloxacin, norfloxacin and ofloxacin or other fluoroquinolone or quinolone; and/or
(b) bismuth salt, preferably bismuth subcitrate potassium; and/or
(c) proton pump inhibitor (PPI), preferably selected from pantoprazole, omeprazole, lansoprazole, esomeprazole, rabeprazole, dexlansoprazole, dexrabeprazole and vonoprazan; and/or
(d) urease inhibitor; and/or
(e) carbonic anhydrase inhibitor, preferably acetazolamide.

**Patentansprüche**

**1.** Verbindung gemäß Formel (I),

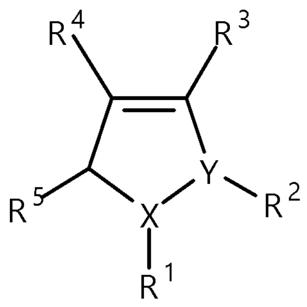

(I)

zur Verwendung bei der

(A) Behandlung und/oder Prävention einer bakteriellen Infektion, wobei die bakterielle Infektion durch flagellierte Bakterien, ausgewählt aus *Helicobacter spp.* und *Campylobacter spp.* verursacht wird;
(B) Behandlung eines Zustands, der mit der in (A) definierten bakteriellen Infektion assoziiert ist, wobei der Zustand ausgewählt ist aus peptischem Ulkus, Gastritis, Duodenitis, Magenschleimhaut-assoziiertem Lymphgewebe-Lymphom (MALT-Lymphom des Magens), Enteritis, und Enterokolitis; oder
(C) Prävention eines Zustands, der mit der bakteriellen Infektion, wie in (A) definiert, assoziiert ist, wobei der Zustand ausgewählt ist aus Magengeschwür, Gastritis, Duodenitis, Magenkrebs, MALT-Lymphom des Magens, Enteritis, und Enterokolitis; und
wobei in (A) bis (C) eine therapeutisch wirksame Menge der Verbindung einem Individuum, das diese benötigt, zu verabreichen ist, wobei das Individuum ein Säuger, vorzugsweise ein Mensch, oder ein anderes Tier ist; und wobei:

(a) X und Y der Formel (I) unabhängig voneinander ausgewählt sind aus N, O und S;
(b) $R^1$ ist:

(b-1) Phenyl, wahlweise substituiert mit einem oder mehreren der Folgenden:

(i) $C_{1-3}$-Alkyl oder Isopropyl; und/oder
(ii) X oder $CX_3$ (X ist ausgewählt aus Cl, Br, I und F); oder

(b-2) Biphenyl;

(c) $R^2$ $CX_3$ oder $CH_2CX_3$ ist, wobei X aus H, F, Cl, Br und I ausgewählt ist;
(d) $R^3$ $CH_2SX$ ist, wobei X H oder $C(NH)NH_2$ ist;
(e) $R^4$ ausgewählt ist aus F, Cl, Br und I; und
(f) $R^5$ O ist; und

wobei die Verbindung gemäß Formel (I) eine Anti-Motilitäts-Aktivität auf die Bakterien hat.

**2.** Verbindung zur Verwendung gemäß Anspruch 1, wobei die Verbindung entsprechend der Formel (II) ist:

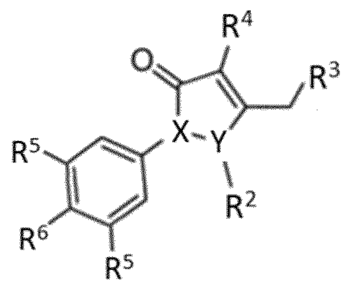

(II),

wobei

(a) X und Y der Formel (II) jeweils unabhängig voneinander ausgewählt sind aus N, O und S;
(b) $R^2$ $CH_3$ oder $CH_2CH_3$ ist;
(c) $R^3$ SX ist, wobei X H oder

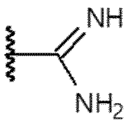

ist; und
(d) $R^4$ ausgewählt ist aus F, Cl, Br und I; und
(e) $R^5$ und $R^6$ jeweils unabhängig voneinander ausgewählt sind aus X oder $CX_3$ (X ist ausgewählt aus H, F, Cl, Br und I), Ethyl, Isopropyl oder Phenyl.

**3.** Verbindung zur Verwendung gemäß Anspruch 2, wobei

(a) X und Y der Formel (II) jeweils unabhängig voneinander ausgewählt sind aus N, O und S;
(b) $R^2$ $CH_3$ oder $CH_2CH_3$ ist;
(c) $R^3$ SX ist, wobei X H oder

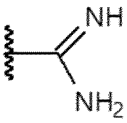

ist; und
(d) $R^4$ ausgewählt ist aus F, Cl, Br und I;
(e) $R^5$ H oder $CH_3$ ist; und
(f) $R^6$ X oder $CX_3$ ist (X ist ausgewählt aus H, F, Cl, Br und I), Ethyl, Isopropyl oder Phenyl.

**4.** Verbindung zur Verwendung gemäß einem der Ansprüche 1 bis 3, wobei die Verbindung entsprechend der Formel (III) ist:

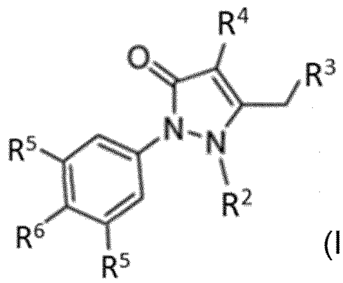

(III),

(a) $R^2$ $CH_3$ oder $CH_2CH_3$ ist;
(b) $R^3$ SX ist, wobei X H oder

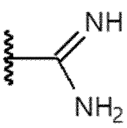

ist; und
(c) $R^4$ ausgewählt ist aus F, Cl, Br und I;
(d) $R^5$ H oder $CH_3$ ist; und
(e) $R^6$ H, X oder $CX_3$ ist (wobei X H, F, Cl, Br oder I ist), Ethyl, Isopropyl oder Phenyl.

**5.** Verbindung zur Verwendung gemäß Anspruch 4, wobei

(a) $R^2$ $CH_2CH_3$ ist und $R^3$ SH ist; oder
(b) $R^2$ $CH_3$ ist und $R^6$ H, X (wobei X F, Cl, Br oder I ist), $CH_3$, Ethyl, Isopropyl oder Phenyl ist.

**6.** Verbindung zur Verwendung gemäß Anspruch 4 oder 5, wobei

(a) $R^2$ $CH_2CH_3$ ist, $R^3$ SH ist, und $R^5$ und $R^6$ jeweils H sind; oder
(b) $R^2$ $CH_3$ ist, $R^3$ SH ist, und $R^5$ und $R^6$ jeweils H sind.

**7.** Verbindung zur Verwendung gemäß Anspruch 1, wobei die Verbindung ausgewählt ist aus:

(a) 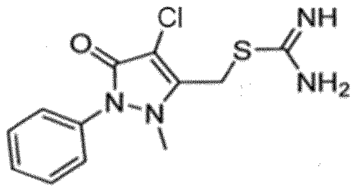

(b) 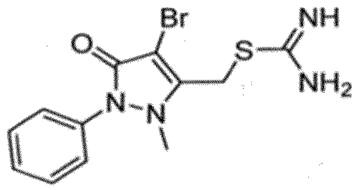

(c) 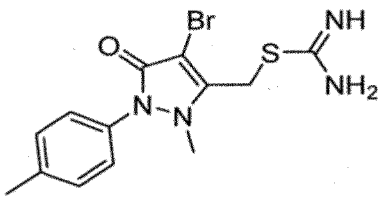

(d) 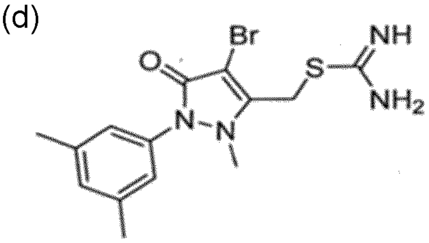

(e) 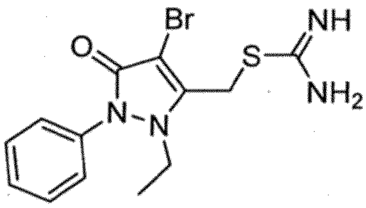

(f) 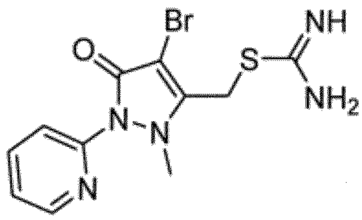

**8.** Verbindung zur Verwendung gemäß Anspruch 1, wobei die Verbindung ausgewählt ist aus:

(a) 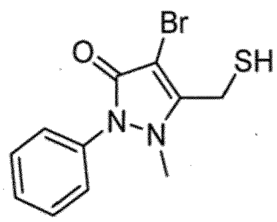

(b) 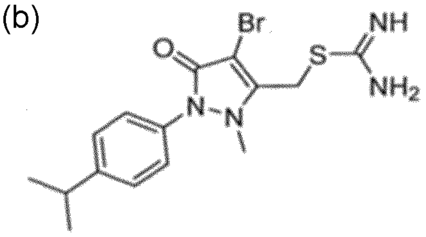

(c) 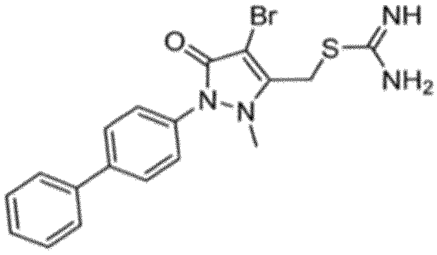

(d) 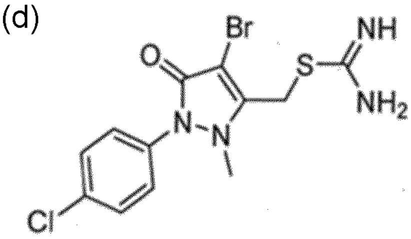

(e) 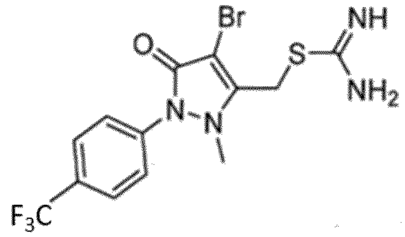 (f) 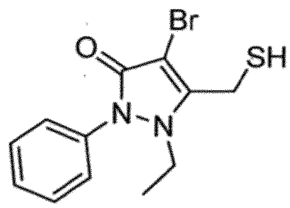

9. Verbindung zur Verwendung gemäß einem der Ansprüche 1 bis 8, wobei die *Helicobacter spp.* und/oder die *Campylobacter spp.* folgende(s) sind:

(a) gastro-intestinale pathogene Bakterien;
(b) gastro-duodenal-pathogene Bakterien;
(c) urogenital-pathogene Bakterien;
(d) nicht empfindlich gegenüber antibiotischer Behandlung;
(e) *Helicobacter pylori*; und/oder
(f) *Campylobacter jejuni* oder *Campylobacter coli.*

10. Verbindung zur Verwendung gemäß einem der Ansprüche 1 bis 9, wobei die Verbindung zu verabreichen ist in Kombination oder nacheinander mit einem oder mehreren weiteren Wirkstoffen ausgewählt aus einem:

(a) Antibiotikum, vorzugsweise ausgewählt aus Amoxicillin, Penicillin, Clarithromycin, Metronidazol, Tetracyclin, Tinidazol, Rifampin, Rifabutin, Ciprofloxacin, Gemifloxacin, Levofloxacin, Moxifloxacin, Norfloxacin und Ofloxacin oder einem anderem Fluorchinolon oder Chinolon; und/oder
(b) Wismutsalz, vorzugsweise Wismutsubcitrat-Kalium; und/oder
(c) Protonenpumpeninhibitor (PPI), vorzugsweise ausgewählt aus Pantoprazol, Omeprazol, Lansoprazol, Esomeprazol, Rabeprazol, Dexlansoprazol, Dexrabeprazol und Vonoprazan; und/oder
(d) Urease-Inhibitor; und/oder
(e) Carboanhydrase-Inhibitor, vorzugsweise Acetazolamid.

## Revendications

1. Composé de formule (1),

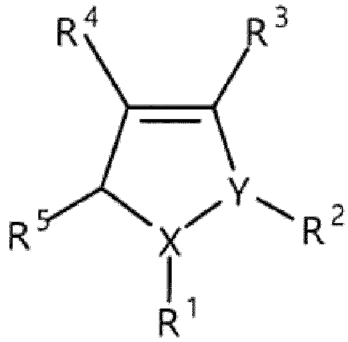

(I)

pour l'utilisation dans

(A) le traitement et/ou la prévention d'une infection bactérienne, où l'infection bactérienne est provoquée par des bactéries flagellées choisies parmi *Helicobacter spp.* et *Campylobacter spp.* ;
(B) le traitement d'un état associé à l'infection bactérienne telle que définie au (A), où l'état est choisi parmi l'ulcère peptique, la gastrite, la duodénite, le lymphome des tissus lymphoïdes associé à la muqueuse gastrique (MALT), l'entérite, et l'entérocolite ; ou
(C) la prévention d'un état associé à l'infection bactérienne telle que définie au (A), où l'état est choisi parmi l'ulcère peptique, la gastrite, la duodénite, le cancer gastrique (estomac), le lymphome gastrique MALT, l'entérite,

et l'entérocolite ; et
dans lequel, au (A) à (C), une quantité thérapeutiquement efficace du composé doit être administrée à un sujet qui en a besoin, où le sujet est un mammifère, de préférence un être humain ou un autre animal ; et
dans laquelle :

(a) X et Y dans la formule (I) sont choisis indépendamment parmi N, O et S ;
(b) $R^1$ est :

(b-1) phényle, éventuellement substitué par un ou plusieurs :

(i) alkyle en $C_{1-3}$ ou isopropyle ; et/ou
(ii) X ou $CX_3$ (X est choisi parmi Cl, Br, 1 et F) ; ou

(b-2) biphényle ;

(c) $R^2$ est $CX_3$ ou $CH_2CX_3$, où X est choisi parmi H, F, Cl, Br et 1 ;
(d) $R^3$ est $CH_2SX$, où X est H ou $C(NH)NH_2$ ;
(e) $R^4$ est choisi parmi F, Cl, Br et I ; et
(f) $R^5$ est O ; et

dans lequel le composé de formule (1) a une activité anti-motilité sur les bactéries.

**2.** Composé pour l'utilisation selon la revendication 1, où le composé est de formule (II) :

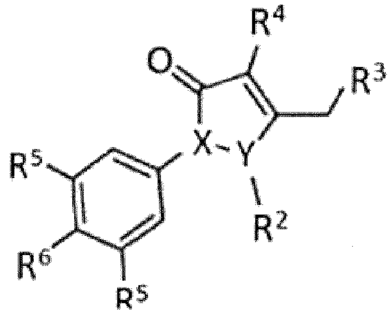

(II),

dans laquelle

(a) X et Y de formule (II) sont choisis chacun indépendamment parmi N, O et S ;
(b) $R^2$ est $CH_3$ ou $CH_2CH_3$ ;
(c) $R^3$ est SX, où X est H ou et

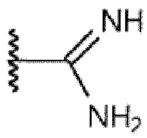

;

(d) $R^4$ est choisi parmi F, Cl, Br
et I ; et
(e) $R^5$ et $R^6$ sont chacun choisis indépendamment parmi X ou $CX_3$ (X est choisi parmi H, F, Cl, Br et 1), éthyle, isopropyle, ou phényle.

**3.** Composé pour l'utilisation selon la revendication 2, dans lequel

(a) X et Y de formule (II) sont choisis chacun indépendamment parmi N, O et S ;
(b) $R^2$ est $CH_3$ ou $CH_2CH_3$ ;

(c) $R^3$ est SX, où X est H ou et

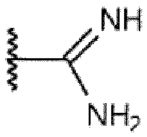

;

(d) $R^4$ est choisi parmi F, Cl, Br et 1 ;
(e) $R^5$ est H ou $CH_3$ ; et
(f) $R^6$ est X ou $CX_3$ (X est choisi parmi H, F, Cl, Br et 1), éthyle, isopropyle, ou phényle.

**4.** Composé pour l'utilisation selon l'une quelconque des revendications 1 à 3, où le composé est de formule (III) :

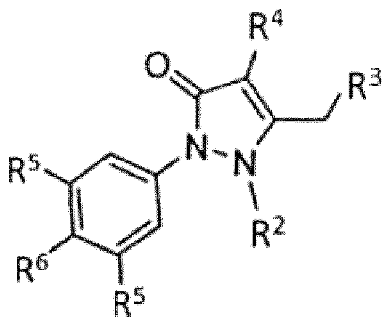

(III),

(a) $R^2$ est $CH_3$ ou $CH_2CH_3$ ;
(b) $R^3$ est SX, où X est H ou et

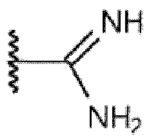

;

(c) $R^4$ est choisi parmi F, Cl, Br
et 1 ;
(d) $R^5$ est H ou $CH_3$ ; et
(e) $R^6$ est H, X ou $CX_3$ (où X est H, F, Cl, Br ou 1), éthyle, isopropyle, ou phényle.

**5.** Composé pour l'utilisation selon la revendication 4, dans lequel

(a) $R^2$ est $CH_2CH_3$ et $R^3$ est SH ; ou
(b) $R^2$ est $CH_3$ et $R^6$ est H, X (où X est F, Cl, Br ou 1), $CH_3$, éthyle, isopropyle, ou phényle.

**6.** Composé pour l'utilisation selon la revendication 4 ou 5, dans lequel

(a) $R^2$ est $CH_2CH_3$, $R^3$ est SH, et $R^5$ et $R^6$ sont chacun H ; ou
(b) $R^2$ est $CH_3$, $R^3$ est SH, et $R^5$ et $R^6$ sont chacun H.

**7.** Composé pour l'utilisation selon la revendication 1, où le composé est choisi parmi :

(a)

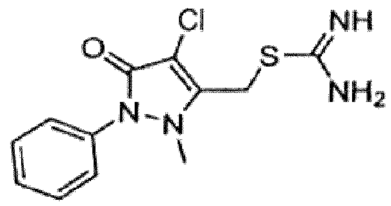

(b)

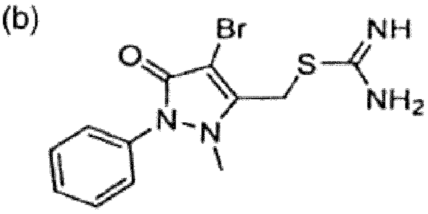

(c)

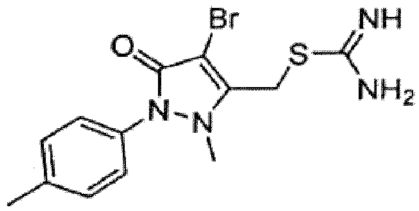

(d)

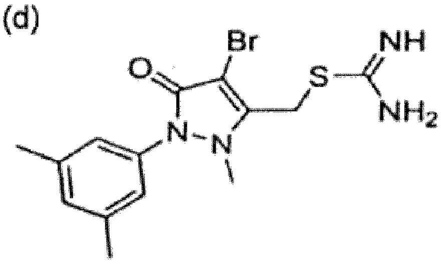

(e)

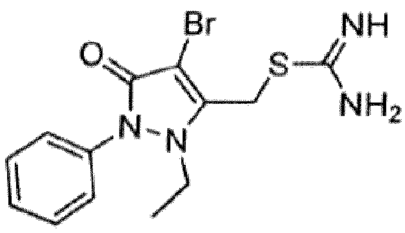

(f)

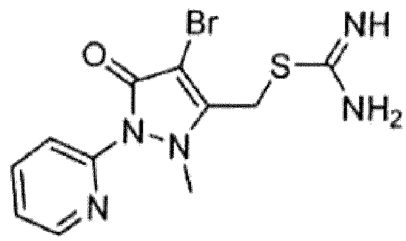

**8.** Composé pour l'utilisation selon la revendication 1, où le composé est choisi parmi :

(a)

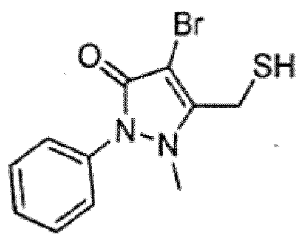

(b)

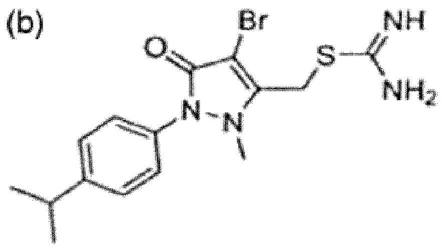

(c)

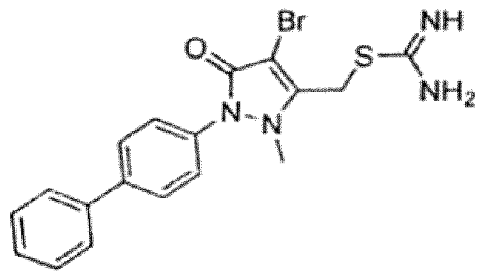

(d)

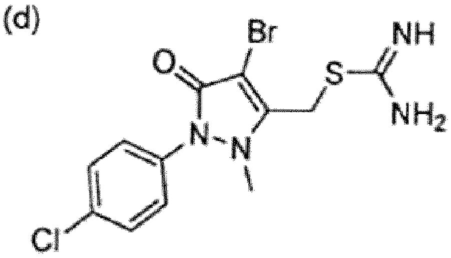

(e)

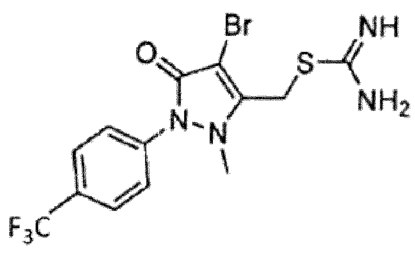

(f)

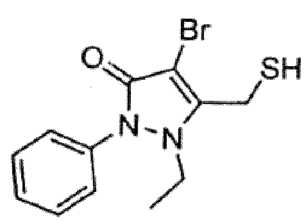

**9.** Composé pour l'utilisation selon l'une quelconque des revendications 1 à 8, dans lequel les *Helicobacter spp.* et/ou *Campylobacter spp.* sont

(a) des bactéries pathogènes gastro-intestinales ;
(b) des bactéries pathogènes gastro-duodénales ;
(c) des bactéries pathogènes urogénitales ;
(d) non sensibles à un traitement antibiotique ;
(e) *Helicobacter pylori* ; et/ou
(f) *Campylobacter jejuni* ou *Campylobacter coli.*

**10.** Composé pour l'utilisation selon l'une quelconque des revendications 1 à 9, où le composé doit être administré en combinaison ou séquentiellement, avec un ou plusieurs autres agents choisis parmi :

(a) des antibiotiques, de préférence choisis parmi l'amoxicilline, la pénicilline, la clarithromycine, le métronidazole, la tétracycline, le tinidazole, la rifampine, la rifabutine, la ciprofloxacine, la gémifloxacine, la lévofloxacine, la moxifloxacine, la norfloxacine, et l'ofloxacine ou une autre fluoroquinolone ou quinolone ; et/ou
(b) un sel de bismuth, de préférence le sous-citrate de bismuth potassique ;
et/ou
(c) un inhibiteur de pompe à protons (IPP), de préférence choisi parmi le pantoprazole, l'oméprazole, le lansoprazole, l'ésoméprazole, le rabéprazole, le dexlansoprazole, le dexrabéprazole et le vonoprazane ; et/ou
(d) un inhibiteur d'uréase ; et/ou
(e) un inhibiteur d'anhydrase carbonique, de préférence l'acétazolamide.

# Figure 1

**A**

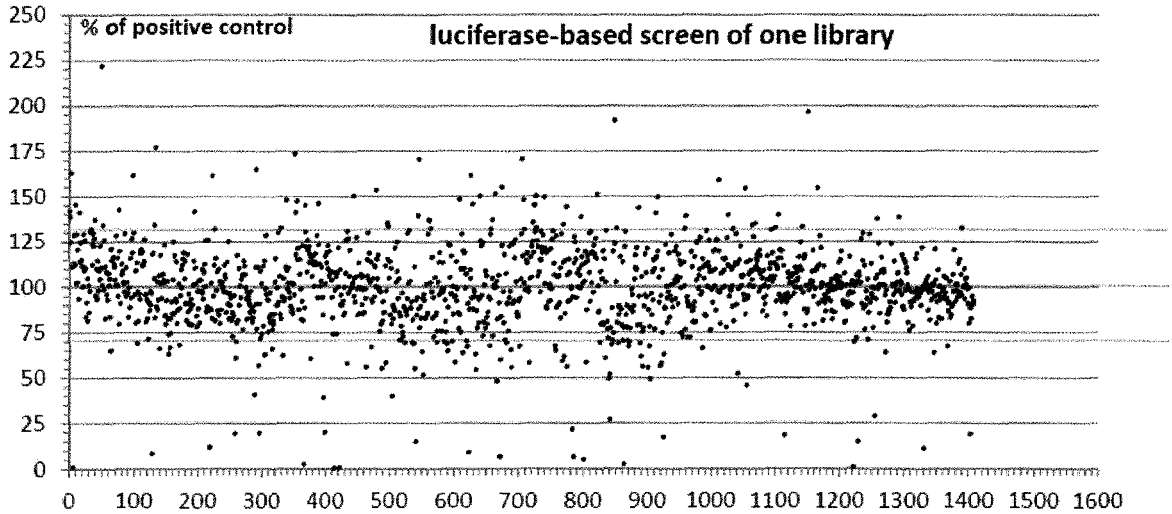


**B**

**library alpha**
257 compounds, 7 hits (luciferase assay)
> 1%

**library beta**
1280 compounds, 113 hits (luciferase assay)
~ 9%

**library gamma**
352 compounds, 28 hits (luciferase assay)
~ 8%

**library delta (random, selected according to Lipinski's rule of 5)**
1120 compounds, 9 hits (luciferase assay)
< 1%

**library epsilon (random unknown)**
1120 compounds, 47 hits (luciferase assay)
~ 4%

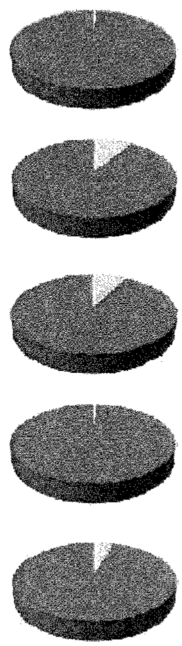

# Figure 2

**A**

In vitro luciferase activity of active compound: $IC_{50}$=0.27 μg/ml

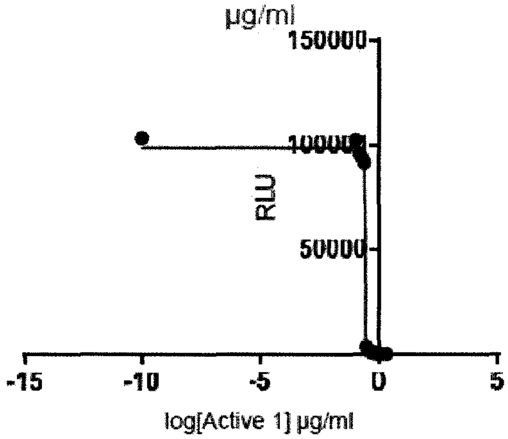


**B**

In vitro metabolic activity of active compound: $IC_{50}$=0.07 μg/ml

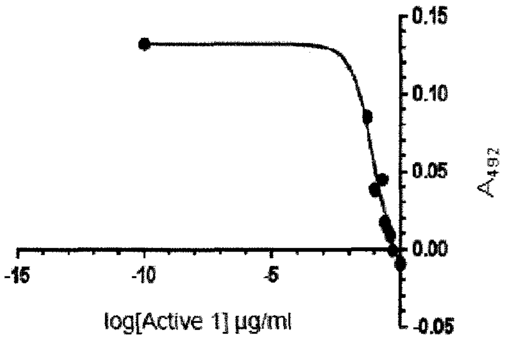


**D**

MHC/MBC of active compound:

MIC = 0.25 μg/ml (1 μM)

MBC = ≥ 1 μg/ml (4.1 μM)

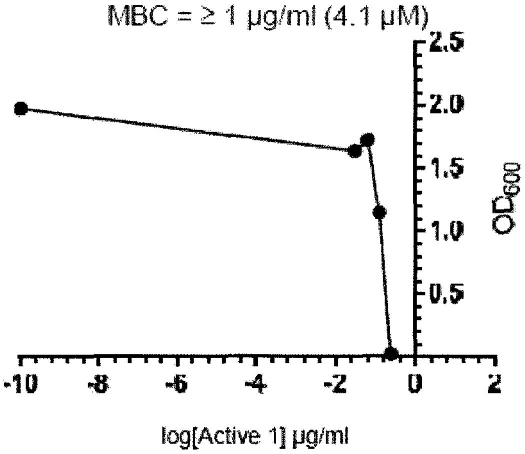

# Figure 3

**A**

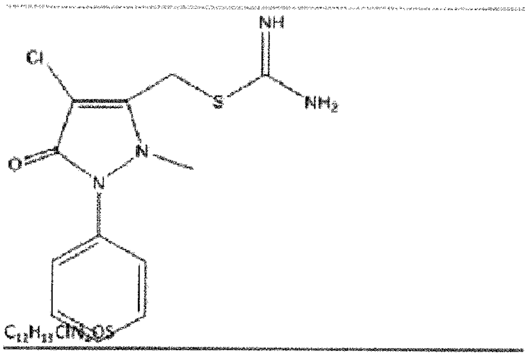

$C_{12}H_{13}ClN_4OS$

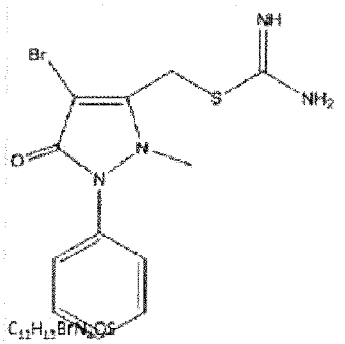

$C_{12}H_{13}BrN_4OS$

**B** In vitro activity (luciferase screen) of active compound

Active2: $IC_{50}$=2.75 µg/ml

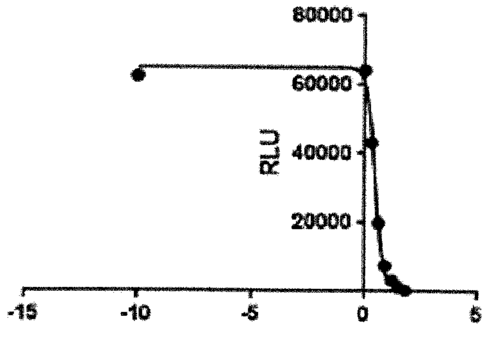

# Figure 4

A

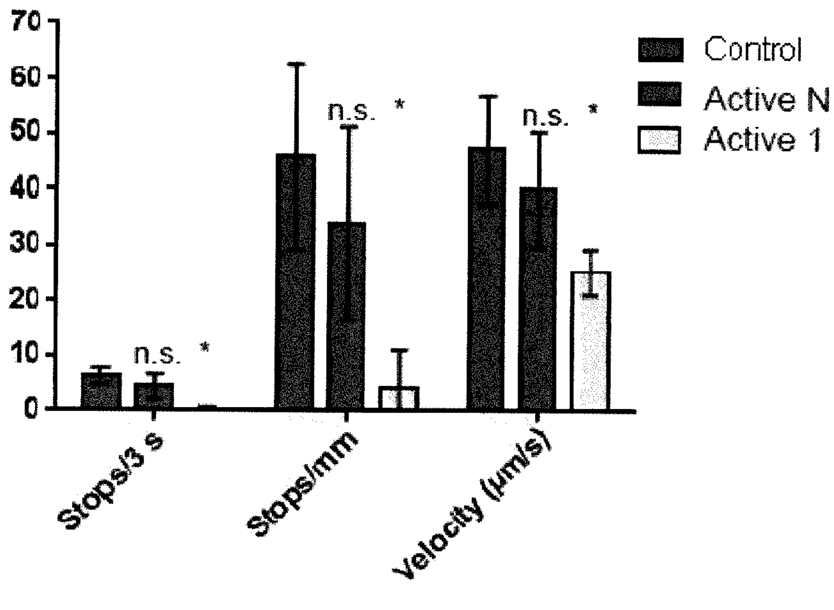
70
60
50
40
30
20
10
0
n.s. *
n.s. *
n.s. *
Control
Active N
Active 1
Stops/3 s
Stops/mm
Velocity (µm/s)

# Figure 5

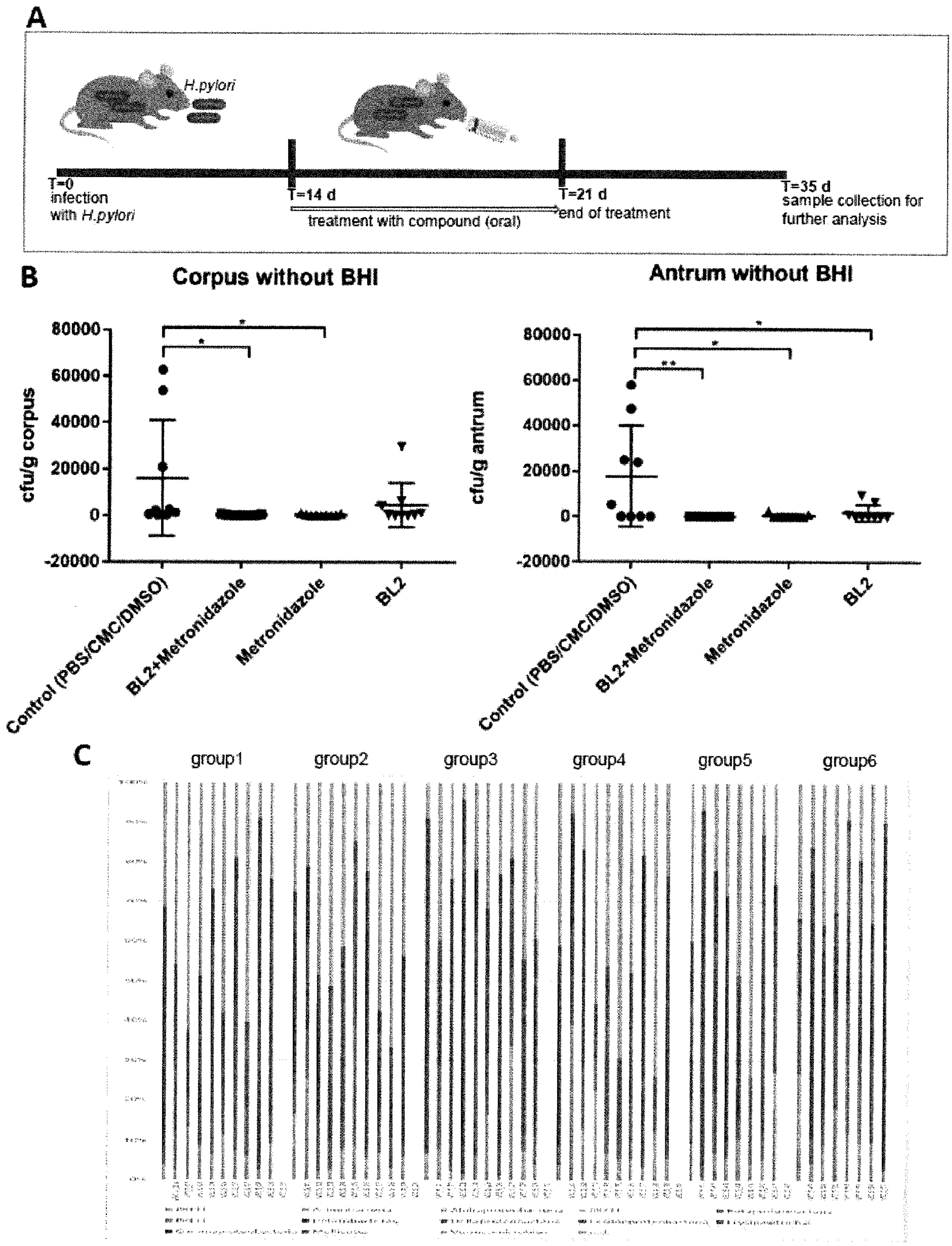
A
H.pylori
T=0
infection
with H.pylori
T=14 d
treatment with compound (oral)
T=21 d
end of treatment
T=35 d
sample collection for
further analysis
B
Corpus without BHI
cfu/g corpus
80000
60000
40000
20000
0
-20000
Control (PBS/CMC/DMSO)
BL2+Metronidazole
Metronidazole
BL2
Antrum without BHI
cfu/g antrum
C
group1
group2
group3
group4
group5
group6

**Figure 6**

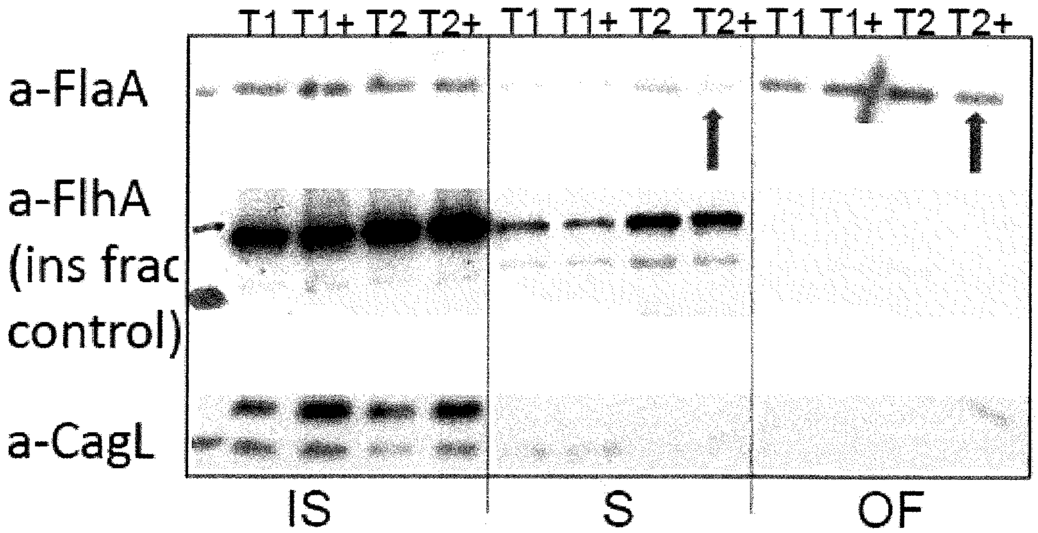
T1 T1+ T2 T2+ T1 T1+ T2 T2+ T1 T1+ T2 T2+
a-FlaA
a-FlhA
(ins frac
control)
a-CagL
IS
S
OF

# Figure 7

A

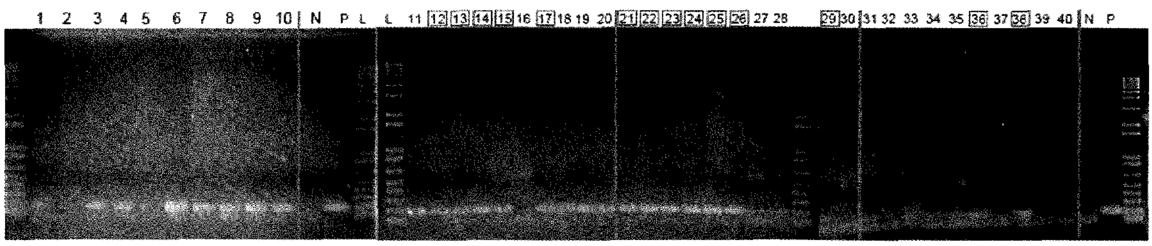


Corpus homogenates 1 to10,NC, PC
Primers: HP87P7_cagL_RT1 and _RT2

Corpus homogenates 11 to 40,NC,PC
Primers: HP87P7_cagL_RT1 and _RT2

B

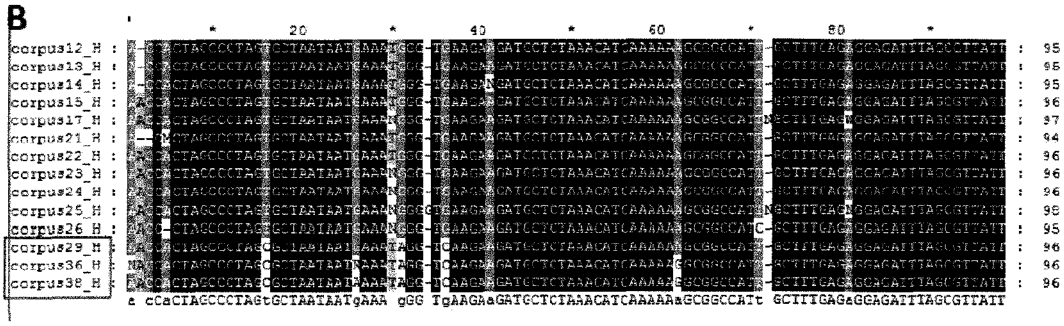


C

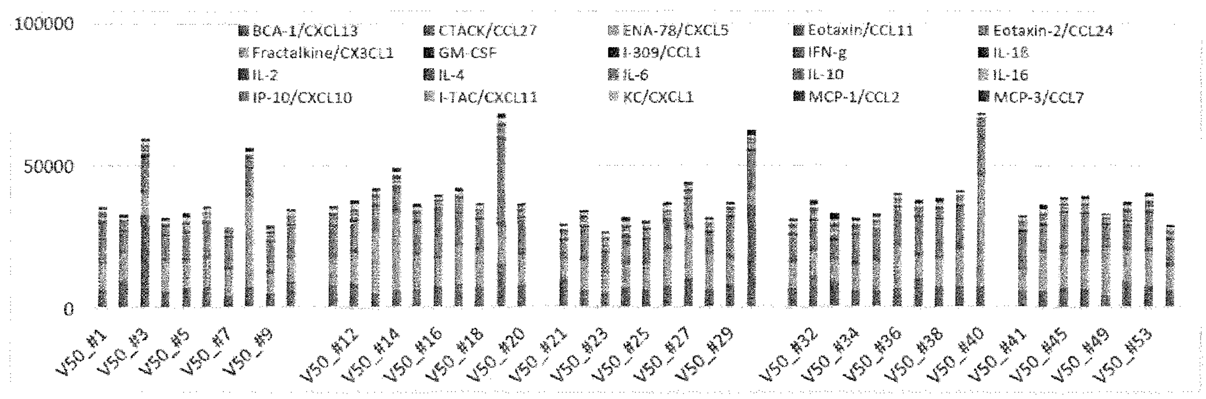

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **CHEVANCE FF ; HUGHES KT.** *Methods Mol Biol.,* 2017, vol. 1593, 47-71
- **STARK ; GRZELAK ; HADFIELD.** *Nature Reviews Genetics,* 2019, vol. 20, 631-656
- **PARKER ; BARNES.** *Meth. Mol. Biol.,* 1999, vol. 106, 247-283
- **HOD.** *Biotechniques,* 1992, vol. 13, 852-854
- **WEIS et al.** *Trends in Genetics,* 1992, vol. 8, 263-264
- **AUSUBEL et al.** Current Protocols of Molecular Biology. John Wiley and Sons, 1997
- **DING ; CANTOR.** *Proc. Natl. Acad. ScL USA,* 2003, vol. 100, 3059-3064
- **LIANG ; PARDEE.** *Science,* 1992, vol. 257, 967-971
- **KAWAMOTO et al.** *Genome Res.,* 1999, vol. 12, 1305-1312
- **OLIPHANT et al.** *Discovery of Markers for Disease (Supplement to Biotechniques),* June 2002
- **FERGUSON et al.** *Anal. Chem.,* 2000, vol. 72, 5618
- **YANG et al.** *Genome Res.,* 2001, vol. 11, 1888-1898
- **FUKUMURA et al.** *Nucl. Acids. Res.,* 2003, vol. 31 (16), e94
- **VELCULESCU et al.** *Science,* 1995, vol. 270, 484-487
- **VELCULESCU et al.** *Cell,* 1997, vol. 88, 243-51
- **BRENNER et al.** *Nature Biotechnology,* 2000, vol. 18, 630-634
- **GORMAN et al.** *Molecular Cell Biology,* 1982, vol. 2, 1044
- **PROST et al.** *Gene,* 1986, vol. 45, 107-111
- **NOLAN et al.** *Proc. Natl. Acad. Sci. USA,* 1988, vol. 85, 2603-2607
- **BERGER et al.** *Gene,* 1988, vol. 66, 1-10
- **CULLEN et al.** *Methods Enzymol.,* 1992, vol. 216, 362-368
- **KARP.** *Biochim. Biophys. Acta,* 1989, vol. 1007, 84-90
- **STEWART et al.** *J. Gen. Microbiol.,* 1992, vol. 138, 1289-1300
- **DE WET et al.** *Mol. Cell. Biol.,* 1987, vol. 7, 725-737
- **CHALFIE et al.** *Science,* 1994, vol. 263, 802-805
- **HEIM et al.** *Nature,* 1995, vol. 373, 663-4
- **DAMMEYER T et al.** *Comput Struct Biotechnol J.,* 2012, vol. 22 (3
- **FRANCIS et al.** *Infect. Immun.,* 2000, vol. 68 (6), 3594-600
- **LACKOWICZ.** Principles of Fluorescence Spectroscopy. Plenum Press, 1983
- **ISLAM, MS et al.** *Micromachines,* 2017, vol. 8 (3), 83
- **LAM.** *Anticancer Drug Des.,* 1997, vol. 12, 145
- **DEWITT et al.** *Proc. Natl. Acad. Sci. U.S.A.,* 1993, vol. 90, 6909
- **ERB et al.** *Proc. Natl. Acad. Sci. U.S.A.,* 1994, vol. 91, 11422
- **ZUCKERMANN et al.** *J Med Chem.,* 1994, vol. 37, 2678
- **CHO et al.** *Science,* 1993, vol. 261, 1303
- **CARELL et al.** *Angew. Chem. Int. Ed Engl.,* 1994, vol. 33, 2059
- **CARELL.** *Angew. Chem. Int. Ed. Engl.,* vol. 33, 2061
- **GALLOP.** *J. Med Chem.,* 1994, vol. 37, 1233
- **HOUGHTEN.** *BioTechniques,* 1992, vol. 13, 412-421
- **LAM KS.** *Nature,* 1991, vol. 354, 82-84
- **FODOR.** *Nature,* 1993, vol. 364, 555-556
- **CULL.** *Proc. Natl. Acad Sci. U.S.A.,* 1992, vol. 89, 1865-1869
- **SCOTT ; SMITH.** *Science,* 1990, vol. 249, 386-390
- **DEVLIN.** *Science,* 1990, vol. 249, 404-406
- **CWIRLA et al.** *Proc. Natl. Acad. Sci.,* 1990, vol. 97, 6378-6382
- **FELICI.** *J. Mol. Biol.,* 1991, vol. 222, 301310
- **TESTERMAN ; WORLD et al.** *J Gastroenterol,* 2014, vol. 20 (36), 12781-12808
- Medical Microbiology. Univ. of Texas Medical Branch at Galveston, 1996
- **ROSNER BM et al.** *Sci Rep.,* 2017, vol. 7 (1), 5139
- **TESTERMAN et al.** *World J Gastroenterol,* 2014, vol. 20 (36), 12781-12808
- **WHITE et al.** *J Inflamm Res.,* 2015, vol. 8, 137-147
- **SUERBAUM ; MICHETTI.** *N Engl J Med.,* 2002, vol. 347 (15), 1175-86
- **O'CONNOR A et al.** *Helicobacter,* 2019, vol. 24 (1), e12640
- **JI-HU ZHANG ; THOMAS D. Y. CHUNG ; KEVIN R.** *Oldenburg J Biomol Screen,* 1999, vol. 4, 67

- **FULMER et al.** *Organometallics,* 2010, vol. 29, 2176-2179
- **OLBERMANN P ; JOSENHANS C ; MOODLEY Y ; UHR M ; STAMER C ; VAUTERIN M ; SUERBAUM S ; ACHTMAN M ; LINZ B.** *PLoS Genet.,* 2010, vol. 6 (8), e1001069
- **AEBISCHER, T. ; WALDUCK, A. ; SCHROEDER, J. ; WEHRENS, A. ; CHIJIOKE, O. ; SCHREIBER, S. ; MEYER, T. F.** A vaccine against Helicobacter pylori: towards understanding the mechanism of protection. *Int J Med Microbiol,* 2008, vol. 298 (1-2), 161-168
- **AGUDO, S ; ALARCON, T ; CIBRELUS, L. ; URRUZUNO, P. ; MARTINEZ, M. J. ; LOPEZ-BREA, M.** High percentage of clarithromycin and metronidazole resistance in Helicobacter pylori clinical isolates obtained from Spanish children]. *Rev Esp Quimioter,* 2009, vol. 22 (2), 88-92
- **ALLEN, R. C. ; POPAT, R ; DIGGLE, S. P ; BROWN, S. P.** Targeting virulence: can we make evolution-proof drugs?. *Nat Rev Microbiol,* 2014, vol. 12 (4), 300-308
- **ANDERMANN, T. M. ; CHEN, Y. T. ; OTTEMANN, K. M.** Two predicted chemoreceptors of Helicobacter pylori promote stomach infection. *Infect Immun,* 2002, vol. 70 (10), 5877-5881
- **ANGELI, A. ; PINTEALA, M. ; MAIER, S. S. ; DEL PRETE, S. ; CAPASSO, C. ; SIMIONESCU, B. C. ; SUPURAN, C. T.** Inhibition of bacterial alpha-, beta- and gamma-class carbonic anhydrases with selenazoles incorporating benzenesulfonamide moieties. *J Enzyme Inhib Med Chem,* 2019, vol. 34 (1), 244-249
- **BEHRENS, W ; SCHWEINITZER, T. ; BAL, J. ; DORSCH, M ; BLEICH, A. ; KOPS, F ; JOSENHANS, C.** Role of energy sensor TlpD of Helicobacter pylori in gerbil colonization and genome analyses after adaptation in the gerbil. *Infect Immun,* 2013, vol. 81 (10), 3534-3551
- **BEHRENS, W ; SCHWEINITZER, T. ; MCMURRY, J. L ; LOEWEN, P. C ; BUETTNER, F. F. ; MENZ, S. ; JOSENHANS, C.** Localisation and protein-protein interactions of the Helicobacter pylori taxis sensor TlpD and their connection to metabolic functions. *Sci Rep,* 2016, vol. 6, 23582
- **BLASER, M. J.** Antibiotic use and its consequences for the normal microbiome. *Science,* 2016, vol. 352 (6285), 544-545
- **CHEVANCE, F. F. ; HUGHES, K. T.** Coordinating assembly of a bacterial macromolecular machine. *Nat Rev Microbiol,* 2008, vol. 6 (6), 455-465
- **DETHLEFSEN, L. ; HUSE, S ; SOGIN, M. L. ; RELMAN, D. A.** The pervasive effects of an antibiotic on the human gut microbiota, as revealed by deep 16S rRNA sequencing. *PLoS Biol,* 2008, vol. 6 (11), e280
- **DUCK, W. M. ; SOBEL, J. ; PRUCKLER, J. M. ; SONG, Q. ; SWERDLOW, D. ; FRIEDMAN, C. ; GOLD, B. D.** Antimicrobial resistance incidence and risk factors among Helicobacter pylori-infected persons, United States. *Emerg Infect Dis,* 2004, vol. 10 (6), 1088-1094
- **EATON, K. A ; MORGAN, D. R ; KRAKOWKA, S.** Motility as a factor in the colonisation of gnotobiotic piglets by Helicobacter pylori. *J Med Microbiol,* 1992, vol. 37 (2), 123-127
- **ERHARDT, M.** Strategies to Block Bacterial Pathogenesis by Interference with Motility and Chemotaxis. *Curr Top Microbiol Immunol,* 2016, vol. 398, 185-205
- **ESTIBARIZ, I. ; OVERMANN, A. ; AILLOUD, F. ; KREBES, J. ; JOSENHANS, C ; SUERBAUM, S.** The core genome m5C methyltransferase JHP1050 (M.Hpy99III) plays an important role in orchestrating gene expression in Helicobacter pylori. *Nucleic Acids Res.,* 2019
- **FERRERO, R. L. ; THIBERGE, J. M. ; HUERRE, M. ; LABIGNE, A.** Recombinant antigens prepared from the urease subunits of Helicobacter spp.: evidence of protection in a mouse model of gastric infection. *Infect Immun,* 1994, vol. 62 (11), 4981-4989
- **GAVRISH, E. ; SHRESTHA, B. ; CHEN, C. ; LISTER, I. ; NORTH, E. J. ; YANG, L. ; LAFLEUR, M. D.** activities of HPi1, a selective antimicrobial against Helicobacter pylori. *Antimicrob Agents Chemother,* 2014, vol. 58 (6), 3255-3260
- **GUTTNER, Y. ; WINDSOR, H. M. ; VIIALA, C. H. ; DUSCI, L. ; MARSHALL, B. J.** Nitazoxanide in treatment of Helicobacter pylori: a clinical and in vitro study. *Antimicrob Agents Chemother,* 2003, vol. 47 (12), 3780-3783
- **HERRERA, V ; PARSONNET, J.** Helicobacter pylori and gastric adenocarcinoma. *Clin Microbiol Infect,* 2009, vol. 15 (11), 971-976
- **HSU, P. I. ; PAN, C. Y. ; KAO, J. Y. ; TSAY, F. W. ; PENG, N. J. ; KAO, S. S. ; TSAI, K. W.** Helicobacter pylori eradication with bismuth quadruple therapy leads to dysbiosis of gut microbiota with an increased relative abundance of Proteobacteria and decreased relative abundances of Bacteroidetes and Actinobacteria. *Helicobacter,* 2018, vol. 23 (4), e12498
- **JOHNSON, J. G. ; YUHAS, C. ; MCQUADE, T. J. ; LARSEN, M. J. ; DIRITA, V. J.** Narrow-spectrum inhibitors of Campylobacter jejuni flagellar expression and growth. *Antimicrob Agents Chemother,* 2015, vol. 59 (7), 3880-3886
- **JOSENHANS, C. ; NIEHUS, E. ; AMERSBACH, S. ; HORSTER, A. ; BETZ, C. ; DRESCHER, B. ; SUERBAUM, S.** Functional characterization of the antagonistic flagellar late regulators FliA and FlgM of Helicobacter pylori and their effects on the H. pylori transcriptome. *Mol Microbiol,* 2002, vol. 43 (2), 307-322

- **JOSENHANS, C. ; SUERBAUM, S.** The role of motility as a virulence factor in bacteria. *Int J Med Microbiol,* 2002, vol. 291 (8), 605-614
- **KAVERMANN, H ; BURNS, B. P. ; ANGERMULLER, K. ; ODENBNEIT, S. ; FISCHER, W. ; MELCHERS, K. ; HAAS, R.** Identification and characterization of Helicobacter pylori genes essential for gastric colonization. *J Exp Med,* 2003, vol. 197 (7), 813-822
- **KENNEDY, A. J. ; BRUCE, A. M ; GINESTE, C ; BALLARD, T. E. ; OLEKHNOVICH, I. N. ; MACDONALD, T. L. ; HOFFMAN, P. S.** Synthesis and Antimicrobial Evaluation of Amixicile-Based Inhibitors of the Pyruvate-Ferredoxin Oxidoreductases of Anaerobic Bacteria and Epsilonproteobacteria. *Antimicrob Agents Chemother,* 2016, vol. 60 (7), 3980-3987
- **KIM, S. E. ; ROH, J. H. ; PARK, M. I. ; PARK, S. J. ; MOON, W. ; KIM, J. H. ; HEO, J. J.** Effect of 7-day Bismuth Quadruple Therapy versus 14-day Moxifloxacin Triple Therapy for Second-line Helicobacter pylori Eradication Therapy. *Korean J Gastroenterol,* 2019, vol. 73 (1), 26-34
- **KO, S. W. ; KIM, Y. J. ; CHUNG, W. C. ; LEE, S. J.** Bismuth supplements as the first-line regimen for Helicobacter pylori eradication therapy: Systemic review and meta-analysis. *Helicobacter,* 2019, e12565
- **LEE, C. W. ; RICKMAN, B. ; ROGERS, A. B. ; GE, Z. ; WANG, T. C ; FOX, J. G.** Helicobacter pylori eradication prevents progression of gastric cancer in hypergastrinemic INS-GAS mice. *Cancer Res,* 2008, vol. 68 (9), 3540-3548
- **LERTSETHTAKARN, P. ; OTTEMANN, K. M. ; HENDRIXSON, D. R.** Motility and chemotaxis in Campylobacter and Helicobacter. *Annu Rev Microbiol,* 2011, vol. 65, 389-410
- **LIU, Q. ; SHI, W. K. ; REN, S. Z. ; NI, W. W ; LI, W. Y. ; CHEN, H. M. ; ZHU, H. L.** Arylamino containing hydroxamic acids as potent urease inhibitors for the treatment of Helicobacter pylori infection. *Eur J Med Chem,* 2018, vol. 156, 126-136
- **LOBO, A. J. ; MCNULTY, C. A. ; UFF, J. S. ; DENT, J. ; EYRE-BROOK, I. A ; WILKINSON, S. P.** Preservation of gastric antral mucus is associated with failure of eradication of Helicobacter pylori by bismuth, metronidazole and tetracycline. *Aliment Pharmacol Ther,* 1994, vol. 8 (2), 181-185
- **LUTHER, J. ; HIGGINS, P. D ; SCHOENFELD, P. S. ; MOAYYEDI, P. ; VAKIL, N. ; CHEY, W. D.** Empiric quadruple vs. triple therapy for primary treatment of Helicobacter pylori infection: Systematic review and meta-analysis of efficacy and tolerability. *Am J Gastroenterol,* 2010, vol. 105 (1), 65-73
- **MALFERTHEINER, P. ; MEGRAUD, F. ; O'MORAIN, C. A. ; GISBERT, J. P ; KUIPERS, E. J. ; AXON, A. T. ; CONSENSUS.** Management of Helicobacter pylori infection-the Maastricht V/Florence Consensus Report. *Gut,* 2017, vol. 66 (1), 6-30
- **MALFERTHEINER, P. ; SELGRAD, M. ; WEX, T ; ROMI, B. ; BORGOGNI, E ; SPENSIERI, F. ; DEL GIUDICE, G.** Efficacy, immunogenicity, and safety of a parenteral vaccine against Helicobacter pylori in healthy volunteers challenged with a Cag-positive strain: a randomised, placebo-controlled phase 1/2 study. *Lancet Gastroenterol Hepatol,* 2018, vol. 3 (10), 698-707
- **MCGEE, D. J. ; LANGFORD, M. L. ; WATSON, E. L. ; CARTER, J. E. ; CHEN, Y. T. ; OTTEMANN, K. M.** Colonization and inflammation deficiencies in Mongolian gerbils infected by Helicobacter pylori chemotaxis mutants. *Infect Immun,* 2005, vol. 73 (3), 1820-1827
- **MENARD, R. ; SCHOENHOFEN, I. C. ; TAO, L. ; AUBRY, A. ; BOUCHARD, P. ; REID, C. W. ; LOGAN, S. M.** Small-molecule inhibitors of the pseudaminic acid biosynthetic pathway: targeting motility as a key bacterial virulence factor. *Antimicrob Agents Chemother,* 2014, vol. 58 (12), 7430-7440
- **NIEHUS, E. ; GRESSMANN, H. ; YE, F. ; SCHLAPBACH, R. ; DEHIO, M. ; DEHIO, C. ; JOSENHANS, C.** Genome-wide analysis of transcriptional hierarchy and feedback regulation in the flagellar system of Helicobacter pylori. *Mol Microbiol,* 2004, vol. 52 (4), 947-961
- **NIEHUS, E ; YE, F ; SUERBAUM, S. ; JOSENHANS, C.** Growth phase-dependent and differential transcriptional control of flagellar genes in Helicobacter pylori. *Microbiology,* 2002, vol. 148 (12), 3827-3837
- **O'MORAIN, C. ; BORODY, T. ; FARLEY, A. ; DE BOER ; W. A., DALLAIRE ; C., SCHUMAN, R.** Efficacy and safety of single-triple capsules of bismuth biskalcitrate, metronidazole and tetracycline, given with omeprazole, for the eradication of Helicobacter pylori: an international multicentre study. *Aliment Pharmacol Ther,* 2003, vol. 17 (3), 415-420
- **O'MORAIN, N. R. ; DORE, M. P ; O'CONNOR, A. J. P. ; GISBERT, J. P. ; O'MORAIN, C. A.** Treatment of Helicobacter pylori infection. *2018. Helicobacter,* 2018, vol. 23 (1), e12519
- **PILOTTO, A. ; FRANCESCHI, M. ; RASSU, M. ; LEANDRO, G ; BOZZOLA, L. ; FURLAN, F ; DI MARIO, F.** Incidence of secondary Helicobacter pylori resistance to antibiotics in treatment failures after 1-week proton pump inhibitor-based triple therapies: a prospective study. *Dig Liver Dis,* 2000, vol. 32 (8), 667-672
- **RASKO, D. A. ; SPERANDIO, V.** Anti-virulence strategies to combat bacteria-mediated disease. *Nat Rev Drug Discov,* 2010, vol. 9 (2), 117-128

- **RUST, M. ; BORCHERT, S. ; NIEHUS, E ; KUEHNE, S. A ; GRIPP, E ; BAJCETA, A. ; JOSENHANS, C.** The Helicobacter pylori anti-sigma factor FlgM is predominantly cytoplasmic and cooperates with the flagellar basal body protein FlhA. *J Bacteriol,* 2009, vol. 191 (15), 4824-4834
- **SALAMA, N. R. ; SHEPHERD, B. ; FALKOW, S.** Global transposon mutagenesis and essential gene analysis of Helicobacter pylori. *J Bacteriol,* 2004, vol. 186 (23), 7926-7935
- **SCHREIBER, S. ; KONRADT, M ; GROLL, C. ; SCHEID, P. ; HANAUER, G ; WERLING, H. O ; SUERBAUM, S.** The spatial orientation of Helicobacter pylori in the gastric mucus. *Proc Natl Acad Sci U S A,* 2004, vol. 101 (14), 5024-5029
- **SCHWEINITZER, T. ; MIZOTE, T. ; ISHIKAWA, N. ; DUDNIK, A ; INATSU, S. ; SCHREIBER, S. ; JOSENHANS, C.** Functional characterization and mutagenesis of the proposed behavioral sensor TlpD of Helicobacter pylori. *J Bacteriol,* 2008, vol. 190 (9), 3244-3255
- **SHARMA, C. M ; HOFFMANN, S. ; DARFEUILLE, F. ; REIGNIER, J. ; FINDEISS, S. ; SITTKA, A ; VOGEL, J.** The primary transcriptome of the major human pathogen Helicobacter pylori. *Nature,* 2010, vol. 464 (7286), 250-255
- **SRIKHANTA, Y. N. ; GORRELL, R. J. ; POWER, P. M. ; TSYGANOV, K. ; BOITANO, M ; CLARK, T. A. ; KWOK, T.** Methylomic and phenotypic analysis of the ModH5 phasevarion of Helicobacter pylori. *Sci Rep,* 2017, vol. 7 (1), 16140
- **SUERBAUM, S ; MICHETTI, P.** Helicobacter pylori infection. *N Engl J Med,* 2002, vol. 347 (15), 1175-1186
- **THEURETZBACHER U ; PIDDOCK L.J.V.** Non-traditional Antibacterial TherapeuticOptions and Challenges. *Cell Host Microbe,* 2019, vol. 26 (1), 61-72
- **TOTSIKA, M.** Disarming pathogens: benefits and challenges of antimicrobials that target bacterial virulence instead of growth and viability. *Future Med Chem,* 2017, vol. 9 (3), 267-269
- **VAN ZANTEN, S. J. ; KOLESNIKOW, T ; LEUNG, V. ; O'ROURKE, J. L ; LEE, A.** Gastric transitional zones, areas where Helicobacter treatment fails: results of a treatment trial using the Sydney strain mouse model. *Antimicrob Agents Chemother,* 2003, vol. 47 (7), 2249-2255
- **XIA, H. X. ; TALLEY, N. J. ; KEANE, C. T ; O'MORAIN, C. A.** Recurrence of Helicobacter pylori infection after successful eradication: nature and possible causes. *Dig Dis Sci,* 1997, vol. 42 (9), 1821-1834
- **YANG, I ; WOLTEMATE, S ; PIAZUELO, M. B. ; BRAVO, L. E. ; YEPEZ, M. C. ; ROMERO-GALLO, J. ; SUERBAUM, S.** Different gastric microbiota compositions in two human populations with high and low gastric cancer risk in Colombia. *Sci Rep,* 2016, vol. 6, 18594
- **YE, F. ; BRAUER, T ; NIEHUS, E. ; DRLICA, K. ; JOSENHANS, C. ; SUERBAUM, S.** Flagellar and global gene regulation in Helicobacter pylori modulated by changes in DNA supercoiling. *Int J Med Microbiol,* 2007, vol. 297 (2), 65-81
- **ZENG, M. ; MAO, X. H. ; LI, J. X ; TONG, W. D. ; WANG, B. ; ZHANG, Y. J. ; ZOU, Q. M.** Efficacy, safety, and immunogenicity of an oral recombinant Helicobacter pylori vaccine in children in China: a randomised, double-blind, placebo-controlled, phase 3 trial. *Lancet,* 2015, vol. 386 (10002), 1457-1464
- **ZHANG, S. ; LEE, D. S. ; MORRISSEY, R. ; APONTE-PIERAS, J. R ; ROGERS, A. B. ; MOSS, S. F.** Early or late antibiotic intervention prevents Helicobacter pylori-induced gastric cancer in a mouse model. *Cancer Lett,* 2015, vol. 359 (2), 345-351